# EUROPEAN PATENT APPLICATION

(11) **EP 4 552 695 A2**
(43) Date of publication of application: **14.05.2025**
(21) Application number: 24205416.1
(22) Date of filing: 08.10.2024
(51) Int. Cl.: A61P 35/00, C07D 215/233, C07D 401/12, A61K 31/4709, A61K 31/47

(54) **TYROSINE KINASES INHIBITORS AND USES THEREOF**

(30) Priority: 17.10.2023 US 202363590975 P
(71) Applicant: Great Novel Therapeutics Biotech & Medicals Corporation, Taipei City 115011, Taiwan (TW)
(72) Inventor: CHEN, JIA-SHIONG, 115011 TAIPEI CITY (TW); YANG, MU-HSUAN, 115011 TAIPEI CITY (TW); CHOU, CHENG-HAN, 115011 TAIPEI CITY (TW); WU, YI-HONG, 115011 TAIPEI CITY (TW); CHU, SZ-HAO, 115011 TAIPEI CITY (TW); CHAO, YE-SU, 115011 TAIPEI CITY (TW); CHEN, CHIA-NAN, 115011 TAIPEI CITY (TW)
(74) Representative: 2K Patentanwälte Blasberg Kewitz & Reichel

(57) **Abstract**

The present disclosure generally relates to compounds of formula (I) which are multiple target inhibitors of tyrosine kinases (TKs) and which can suppress angiogenesis, metastasis, oncogenesis, and/or immune regulation activities by inhibiting TKs and have very potent immunomodulatory activity. The present disclosure also relates to a method of using said tyrosine kinase inhibitors, alone or in combination with HDAC inhibitor, for the treatment of cancers, in particular in cancer immunotherapy, by regulating the tumor microenvironment, including reducing tumor hypoxia, reducing lactic acid accumulation, activating CTL, inhibiting the number and activity of immunosuppressive cells, finally obtaining superior anti-cancer benefits and/or producing lasting immune memory.

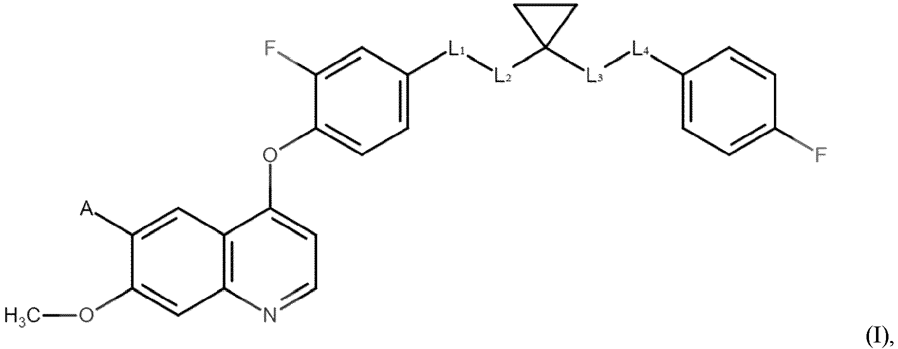

## Description

### Field of the Present Disclosure

The present disclosure generally relates to novel multiple target inhibitor of tyrosine kinases (TKs) which can suppress angiogenesis, metastasis, oncogenesis, and/or immune regulation activities by inhibiting TKs and have very potent immunomodulatory activity. The present disclosure also relates to a method of using the tyrosine kinase inhibitor(s), alone or in combination with an HDAC inhibitor(s), for the treatment of cancers, in particular in cancer immunotherapy, by regulating the tumor microenvironment, including reducing tumor hypoxia, reducing lactic acid accumulation, activating CTL, inhibiting the number and activity of immunosuppressive cells, finally obtaining superior anti-cancer benefits and/or producing lasting immune memory.

### Background of the Present Disclosure

TKs inhibitors have been extensively developed for the treatment of cancers and have been classified as targeted therapies. Cancer immunotherapy emerged, and immune checkpoint inhibitors (ICIs) became clinically successful, opening a new turning point for cancer treatment. However, with the continuous expansion of clinical applications, these emerging therapeutic drugs of ICIs face challenges, and their ORR (objective response rate) is rather low. Thus, a combination drug strategy is needed to improve ORR and allow patients to obtain better treatment benefits.

Although experts have made every effort to find efficient combination regimens of ICIs to enhance therapeutic benefit, the results have shown very limited potential. There is still a strong need to develop combination therapies of anti-cancer drugs.

### Summary of the Present Disclosure

The present disclosure relates to a series of novel structures of multiple target inhibitors of tyrosine kinases (TKs).

In one embodiment, the present disclosure provides a compound of formula (I): 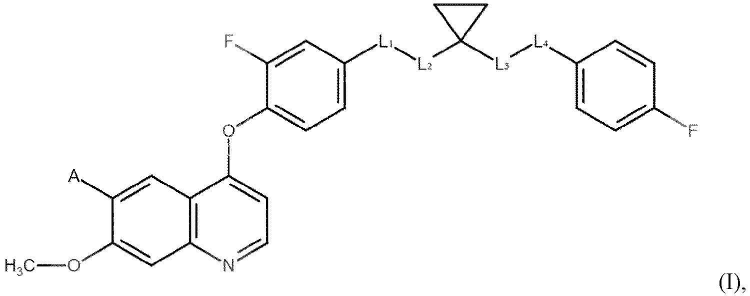 wherein:
A is -OD or 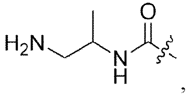 wherein the wavy line represents the linkage position;
D is H, C₁₋₄alkyl or Ar;
Ar is 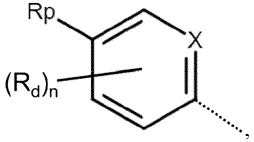 wherein the dashed line represents the linkage position;
X is N, CH or CRa; n is an integer ranging from 0 to 3;
each R_{d}, when present, is independently selected from the group consisting of methyl, NH₂, OH, SH, methoxy, dimethylamino, triflouromethoxy, cyanide, nitro, nitroso, sulfonate, sulfonamide, C₁-C₆ alkyl, Ci-Ce alkyloxy, carboxamide, F, Cl and Br;
Rₚ is selected from the group consisting of H, methyl, NH₂, OH, SH, methoxy, dimethylamino, triflouromethoxy, cyanide, nitro, nitroso, sulfonate, sulfonamide, C₁-C₆ alkyl, C₁-C₆ alkyloxy, carboxamide, F, Cl and Br;
L₁, L₂ is (i) -NH-C(=O)- or (ii) -(C=O)-NH-; and
L₃, L₄ is (i) -NH-C(=O)- or (ii) -(C=O)-NH-;
or a pharmaceutically acceptable salt, hydrate, stereoisomer, solvate or prodrug thereof.

In one embodiment, the present disclosure provides a compound of formula (II): 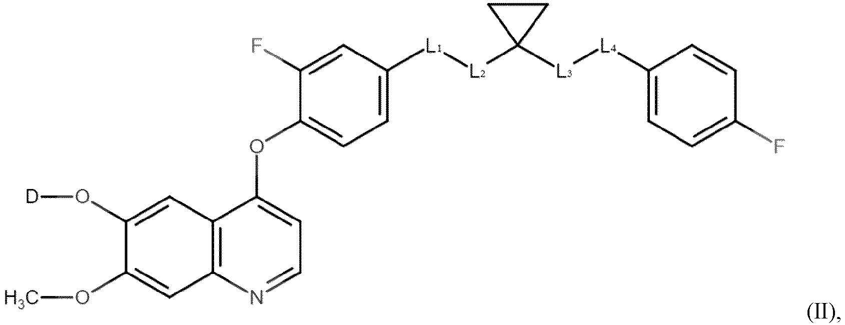 wherein D, L₁, L₂, L₃ and L₄, have the same meaning as described in formula (I); or a pharmaceutically acceptable salt, hydrate, stereoisomer, solvate or prodrug thereof.

In one embodiment, the present disclosure provides a compound of formula (III): 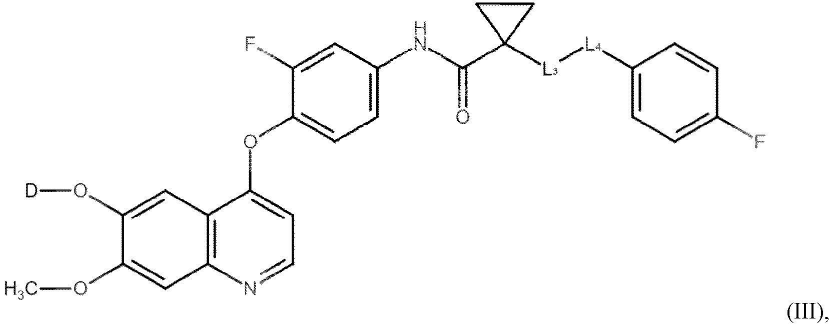 wherein D, L₃ and L₄, have the same meaning as described in formula (I); or a pharmaceutically acceptable salt, hydrate, stereoisomer, solvate or prodrug thereof.

In one embodiment, the present disclosure provides a compound of formula (IV): 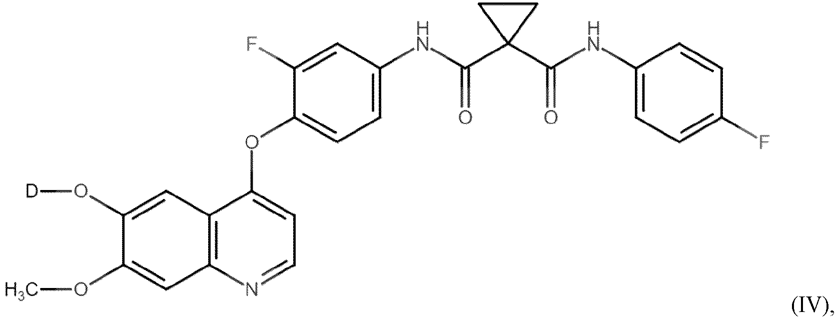 wherein D has the same meaning as described in formula (I); or a pharmaceutically acceptable salt, hydrate, stereoisomer, solvate or prodrug thereof.

In one embodiment, the present disclosure provides a compound selected from the group consisting of: 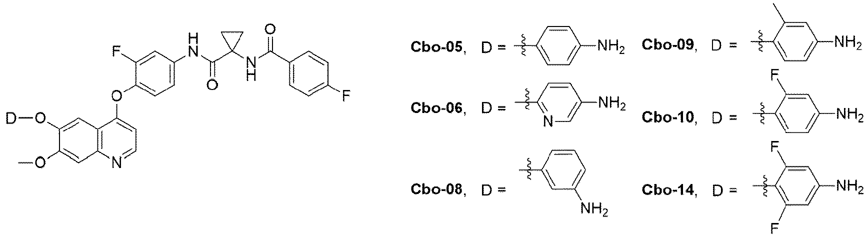 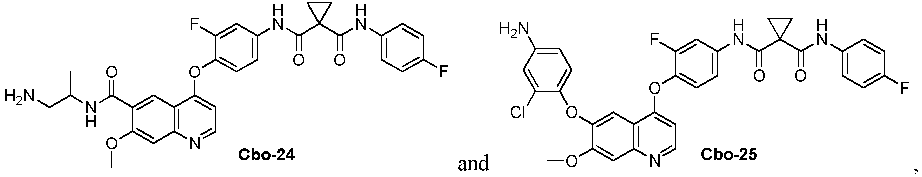 or a pharmaceutically acceptable salt, hydrate, stereoisomer, solvate or prodrug thereof.

In other embodiments, the present disclosure provides a pharmaceutical composition or combination comprising a compound described herein.

In other embodiments, the present disclosure provides a method for suppressing angiogenesis, metastasis, oncogenesis and/or immune regulation activities in a subject in need thereof, comprising administering an effective amount of a compound or a pharmaceutical composition or pharmaceutical combination described herein to the subject.

In other embodiments, the present disclosure provides a method for treating diseases or conditions associated with tyrosine kinases and/or tumors or cancers in a subject in need thereof, comprising administering an effective amount of a compound or a pharmaceutical composition or pharmaceutical combination described herein to the subject.

### Brief Description of the Drawings

**Figure 1** shows chemical structure and detailed NMR data for Cbo-23.
**Figures 2A-2G** show the design of animal testing and results regarding cabozantinib, Cbo-02 and drug combinations for the evaluation of treatment efficacy.
**Figures 3A-3G** show the design of animal testing and results regarding cabozantinib, Cbo-20 and drug combinations for the evaluation of treatment efficacy.
**Figures 4A-4F** show the animal testing results regarding cabozantinib, Cbo-22 and drug combinations for the evaluation of treatment efficacy.
**Figures 5A-5G** show the design of animal testing and results regarding cabozantinib, Cbo-23 and drug combinations for the evaluation of treatment efficacy.
**Figures 6A-6G** show the design of animal testing and results regarding cabozantinib, Cbo-23 and drug combinations for the evaluation of treatment efficacy.
**Figures 7A-7G** show the design of animal testing and results regarding zanzalintinib, Cbo-23-k30 and drug combinations for the evaluation of treatment efficacy.

### Detailed Description of the Invention

### Definitions

Unless otherwise defined, all technical and scientific terms used herein have the same meaning as commonly understood by one of ordinary skill in the art to which this disclosure belongs. The terminology used in the description is for describing particular embodiments only and is not intended to limit the present disclosure.

Where a range of values is provided, it is understood that each intervening value, to the tenth of the unit of the lower limit-unless the context clearly dictates otherwise (such as in the case of a group containing a number of carbon atoms, in which case each carbon atom number falling within the range is provided)-is between the upper and lower limit of that range, and any other stated or intervening value in that stated range is encompassed within the present disclosure. The upper and lower limits of these smaller ranges may independently be included in the smaller ranges and are also encompassed within the present disclosure, subject to any specifically excluded limit in the stated range. Where the stated range includes one or both of the limits, ranges excluding either or both of those included limits are also included in the present disclosure.

The articles "a" and "an" as used herein and in the appended claims are used to refer to one or to more than one (i.e., to at least one) of the grammatical object of the article unless the context clearly indicates otherwise. By way of example, "an element" means one element or more than one element.

The term "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically listed in the "and/or" phrase, whether related or unrelated to those elements specifically identified.

The terms "halo" and "halogen," as used herein, refer to an atom selected from fluorine, chlorine, bromine and iodine.

The term "alkyl" refers to a straight or branched hydrocarbon chain radical consisting solely of carbon and hydrogen atoms, containing no unsaturation, having from one to plural carbon atoms. In some embodiments, an alkyl comprises one to six carbon atoms (e.g., C₁-C₆ alkyl), or one to five carbon atoms (e.g., C₁-C₅ alkyl).In some embodiments, an alkyl comprises one to four carbon atoms (e.g., C₁-C₄ alkyl). In other embodiments, an alkyl comprises one to three carbon atoms (e.g., C₁-C₃ alkyl). In other embodiments, an alkyl comprises one to two carbon atoms (e.g., C₁-C₂ alkyl). In other embodiments, an alkyl comprises one carbon atom (e.g., C₁ alkyl). In other embodiments, an alkyl comprises two to five carbon atoms (e.g., C₂-C₅ alkyl). In other embodiments, an alkyl comprises three to five carbon atoms (e.g., C₃-C₅ alkyl). In other embodiments, the alkyl group is selected from methyl, ethyl, 1-propyl (n-propyl), 1-methylethyl (iso-propyl), 1-butyl (n-butyl), 1-methylpropyl (sec-butyl), 2-methylpropyl (iso-butyl), 1,1-dimethylethyl (tert-butyl), 1-pentyl (n-pentyl). The alkyl is attached to the rest of the molecule by a single bond. Unless specifically stated otherwise in the specification, an alkyl group is optionally substituted by one or more substituents.

The term "alkoxy" refers to a radical bonded through an oxygen atom of the formula - O-alkyl, where alkyl is an alkyl chain as defined above.

The term "cycloalkyl," as used herein, denotes a monovalent group derived from a monocyclic or polycyclic saturated or partially unsaturated carbocyclic ring compound. Examples of C₃-C₈-cycloalkyl include, but are not limited to, cyclopropyl, cyclobutyl, cyclopentyl, cyclohexyl, cyclopentyl and cyclooctyl.

The term "aryl," as used herein, refers to a mono- or poly-cyclic carbocyclic ring system having one or more aromatic rings, fused or non-fused, including, but not limited to, phenyl, naphthyl, tetrahydronaphthyl, indanyl, indenyl and the like.

The term "heteroaryl," as used herein, refers to a mono- or poly-cyclic (e.g., bi-, or tri-cyclic or more) fused or non-fused, radical or ring system having at least one aromatic ring, having from five to ten ring atoms of which one of the ring atoms is selected from S, O and N; zero, one or two ring atoms are additional heteroatoms independently selected from S, O and N; and the remaining ring atoms are carbon. Heteroaryl includes, but is not limited to, pyridinyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, benzimidazolyl, benzooxazolyl, quinoxalinyl, and the like.

The term "heterocycloalkyl," as used herein, refers to a non-aromatic 3-, 4-, 5-, 6- or 7-membered ring or a bi- or tri-cyclic group fused of non-fused system, where (i) at least one ring contains between one and three heteroatoms independently selected from oxygen, sulfur and nitrogen, (ii) each 5-membered ring has 0 to 1 double bonds and each 6-membered ring has 0 to 2 double bonds, (iii) the nitrogen and sulfur heteroatoms may optionally be oxidized, (iv) the nitrogen heteroatom may optionally be quaternized, and (iv) any of the above rings may be fused to a benzene ring. Representative heterocycloalkyl groups include, but are not limited to, [1,3]dioxolane, pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, and tetrahydrofuryl.

The term "pharmaceutically acceptable salt" refers to salts prepared from pharmaceutically acceptable non-toxic bases or acids including inorganic or organic bases and inorganic or organic acids. Salts of basic compounds encompassed within the term "pharmaceutically acceptable salt" refer to non-toxic salts of the compounds of present disclosure which are generally prepared by reacting the free base with a suitable organic or inorganic acid. Representative salts of basic compounds of the present disclosure include, but are not limited to, the following: acetate, ascorbate, adipate, alginate, aspirate, benzenesulfonate, benzoate, bicarbonate, bisulfate, bitartrate, borate, bromide, butyrate, camphorate, camphorsulfonate, camsylate, carbonate, chloride, clavulanate, citrate, cyclopentane propionate, diethylacetic, digluconate, dihydrochloride, dodecylsulfanate, edetate, edisylate, estolate, esylate, ethanesulfonate, formate, fumarate, gluceptate, glucoheptanoate, gluconate, glutamate, glycerophosphate, glycollylarsanilate, hemisulfate, heptanoate, hexanoate, hexylresorcinate, hydrabamate, hydrobromide, hydrochloride, 2-hydroxyethanesulfonate, hydroxynaphthoate, hydroiodide, iodide, isonicotinate, isothionate, lactate, lactobionate, laurate, malate, maleate, mandelate, mesylate, methylnitrate, methylsulfate, methanesulfonate, mucate, 2-naphthalenesulfonate, napsylate, nicotinate, nitrate, oleate, oxalate, pamoate (embonate), palmitate, pantothenate, pectinate, persulfate, phosphate/diphosphate, pimelate, phenylpropanoate, polygalacturonate, propionate, salicylate, stearate, sulfate, subacetate, succinate, tannate, tartrate, teoclate, thiocyanate, tosylate, triethiodide, trifluoroacetate, undeconate, valerate and the like. Furthermore, where the compounds of the present disclosure carry an acidic moiety, suitable pharmaceutically acceptable salts thereof include, but are not limited to, salts derived from inorganic bases including aluminum, ammonium, calcium, copper, ferric, ferrous, lithium, magnesium, manganic, mangamous, potassium, sodium, zinc, and the like. Salts derived from pharmaceutically acceptable organic non-toxic bases include salts of primary, secondary, and tertiary amines, cyclic amines, dicyclohexyl amines and basic ion-exchange resins, such as arginine, betaine, caffeine, choline, N,N-dibenzylethylenediamine, diethylamine, 2-diethylaminoethanol, 2-dimethylaminoethanol, ethanolamine, ethylamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like. Also included are the basic nitrogen-containing groups that may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

The term "subject" includes living organisms such as humans, monkeys, cows, sheep, horses, pigs, cattle, goats, dogs, cats, mice, rats, cultured cells, and transgenic species thereof. In a preferred embodiment, the subject is a human.

The term "administering" includes routes of administration which allow the active ingredients of present disclosure to perform their intended function.

The term "treat" or "treatment" refers to a method of reducing the effects of a disease or condition. Treatment can also refer to a method of reducing the underlying cause of the disease or condition itself rather than just the symptoms. The treatment can be any reduction from native levels and can be, but is not limited to, the complete ablation of the disease, condition, or the symptoms of the disease or condition.

The term "prevent," "prevention" or "preventing" means inhibition or averting of symptoms associated with the target disease.

The term "therapeutically effective amount" refers to that amount of a compound, material, or composition comprising a compound of the present disclosure which is effective for producing a desired therapeutic effect, at a reasonable benefit/risk ratio applicable to any medical treatment.

### Tyrosine kinase (TK) inhibitor compounds

Tyrosine kinase inhibitors are believed to be a successful class of anti-cancer drugs in term of clinical application. Potential TK targets for inhibition include c-MER, AXL, ROS 1, BTK, TYROS (RSE), NTRK2 (TRKB), LCK, LYNB, FLT4 (VEGFR3), EPHA2, HCK, FER, FLT3, FGR, FRK (PTK5), RET, KDR (VEGFR2), SRC, c-MET, PDGFRA, CSFIR (FMS), MST1R (RON), LYNA, INSRR (IRR), and TEK (Tie-2). Numerous TK inhibitors have been approved by the US FDA, including those for treatment for oncology indications and non-oncology indications. Some TK inhibitors are also believed to have immune-regulatory activity in tumor microenvironment, but it is difficult to confirm whether their inhibition of those TKs is closely related to immune regulation. Many TKs inhibitors (such as Regorafenib, Lenvatinib, and Cabozantinib) that have been marketed have been combined with ICIs (such as anti-PD-1/anti-PD-L1 Ab) in clinical trials, with the hope of improving the anti-tumor treatment benefits of ICI through the immunomodulatory ability of TK inhibitors. However, the vast majority of combination regimens have not met expectations or demonstrated the primary endpoint efficacy of clinical trials. This also shows that in order to significantly improve the anti-cancer efficacy of cancer immunotherapy, it is necessary to overcome the rigidity of ICIs, and to create different new cancer immunotherapy mechanisms of new drugs, to be used alone or in combination.

More and more studies have found that the ORR that determines cancer immunotherapy is controlled by the tumor microenvironment. However, the tumor microenvironment is dynamic and complex, and its composition is uncertain as the status of tumor growth changes. Therefore, it is indeed very difficult to effectively regulate the tumor microenvironment. The applicant has been focusing on this field of research and has made important discoveries. Without being bound to theory, the applicant surprisingly notes the following tasks would be important to achieve the goal of regulating tumor microenvironment. The first important task in regulating the tumor microenvironment is to normalize tumor blood vessels. Only normalization of tumor blood vessels has the potential to remodel the tumor microenvironment. This will effectively reverse the attraction of a large number of immunosuppressive cells (such as Treg, TAM, and MDSCs) to the tumor microenvironment, and facilitate the attraction of CTL and NK to the tumor microenvironment to attack the tumor. The second important task is to alleviate the problem of hypoxia in the tumor microenvironment, and reduce the expression of HIF-1, which will help CTL continue to maintain the ability to attack the tumor. The third important task is to effectively overcome anaerobic respiration that regulates the tumor microenvironment, reduce the synthesis and accumulation of lactic acid, and diminish the continuous maintenance of acidic environment in the tumor microenvironment, which is conducive to the activity and survival of CTL for the attack of tumors. The fourth important task is to attract more CTLs that already have the ability to identify the tumor, home into the tumor microenvironment so as to effectively attack the tumor and achieve the benefit of tumor removal. Among them, increasing antigen presentation is very important to improving the cancer cell recognition ability of CTL. The last important task is to induce the immune memory that the host can generate, so that tumors with the same surface antigen will be eradicated and not recur, so that the host can obtain long-term therapeutic benefits.

The present disclosure thus relates to a certain class of multi-kinase inhibitors that can be used in combination with a certain class of HDAC inhibitors, wherein the combination can meet the needs of the above-mentioned mechanisms, obtain excellent ORR, and generate long-term immune memory of cancer immunotherapy. Surprisingly, the compounds of formula (I) described herein (or formulae (II) to (IV), for example, Cbo-23 and its derivatives) are demonstrated to have the dual properties of multi-kinase inhibitor and tumor immune activation through repeated verification in animal tests, and when they are combined with certain HDAC inhibitors, excellent cancer immunotherapy activity is exhibited and long-lasting immune memory is generated. Particularly, the compounds of formulae (I), (II), (III) or (IV), for example Cbo-23 and its derivatives, may be further developed into novel drugs for the treatment of tumors, and are one component of a new generation of cancer immunotherapy with immune-regulating activity in the tumor microenvironment.

### Multiple target inhibitors of tyrosine kinases (TKs)

As described herein, the compounds of formulae (I), (II), (III) and/or (IV) or a pharmaceutically acceptable salt, hydrate, stereoisomer, solvate or prodrug thereof can act as multiple target inhibitors of tyrosine kinases.

In any preceding embodiments, Ar is 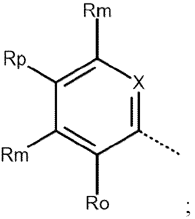 X is N, CH or CRₒ; Rₚ is selected from the group consisting of H, NH₂ and nitro; each Rₘ is independently selected from the group consisting of H, methyl, NH₂, nitro, nitroso, sulfonate, sulfonamide, C₁-C₆ alkyl, C₁-C₆ alkyloxy, carboxamide, F, Br and Cl; and each Rₒ is independently selected from the group consisting of H, methyl, NH₂, nitro, nitroso, sulfonate, sulfonamide, C₁-C₆ alkyl, C₁-C₆ alkyloxy, carboxamide, F, Br and Cl. In one embodiment, Ar is 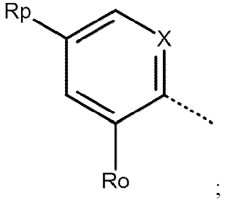 X is N, CH or CRₒ; Rₚ is selected from the group consisting of H, NH₂ and nitro; and each Rₒ is independently selected from the group consisting of H, methyl, NH₂, nitro, nitroso, sulfonate, sulfonamide, C₁-C₆ alkyl, C₁-C₆alkyloxy, carboxamide, F, Br and Cl.

In any preceding embodiments, Ar is selected from the group consisting of

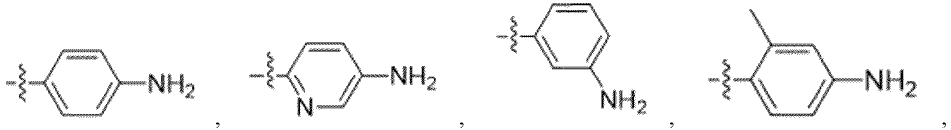

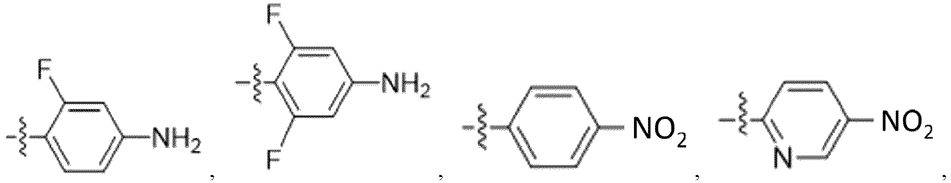

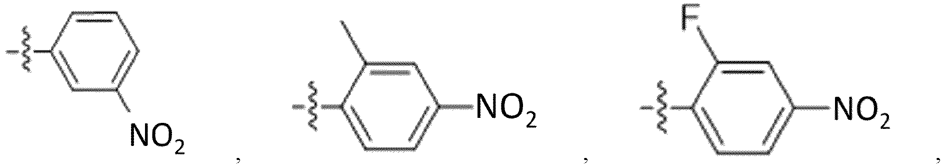

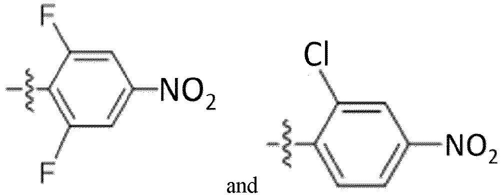

In one embodiment, in any one of the compounds of formulae (I), (II), (III) and/or (IV), L₁, L₂ is -NH-C(=O)- and L₃, L₄ is -NH-C(=O)-. In one embodiment, in any one of the compounds of formulae (I), (II), (III) and/or (IV), L₁, L₂ is -NH-C(=O)- and L₃, L₄ is -(C=O)-NH-. In one embodiment, in any one of the compounds of formulae (I), (II), (III) and/or (IV), L₁, L₂ is -(C=O)-NH- and L₃, L₄ together represents -NH-C(=O)-. In one embodiment, in any one of the compounds of formulae (I), (II), (III) and/or (IV), L₁, L₂ is -(C=O)-NH- and L₃, L₄ is -(C=O)-NH-.

In any preceding embodiments, Rₚ is selected from the group consisting of H, NH₂ and nitro (NO₂).

In any preceding embodiments, each R_{d}, when present, is independently selected from the group consisting of methyl, NH₂, nitro (NO₂), C₁-C₃ alkyl, F, Cl and Br. In any preceding embodiment, Rₚ is H, n is 1, and R_{d} is located at a meta-position with respect to the dashed line. In any preceding embodiment, n is 1 or 2 and each R_{d} is located at an ortho-position with respect to the dashed line.

The compounds of formula (I) described herein (or formulae (II) to (IV), for example, Cbo-23 and its derivatives) are potent multi-kinase inhibitors and the mechanism of action of Cbo-23 and its derivatives is as kinase-specific inhibitor. The compounds of formula (I) described herein (or formulae (II) to (IV), for example, Cbo-23 and its derivatives) are also multi-kinase inhibitors with very potent immunomodulatory activity. For example, the results of animal studies show that Cbo-23 and its derivatives, alone or in combination with HDAC inhibitors, exhibited very good anti-cancer activity, and the combination particularly had superior anti-cancer treatment benefits. The superior anti-cancer benefits of this combination are related to the anti-cancer activity of cancer immunotherapy. Without being bound to theory, the mechanisms of cancer immunotherapy involve regulating the tumor microenvironment, including reducing tumor hypoxia, reducing lactic acid accumulation, activating CTL, inhibiting the number and activity of immunosuppressive cells, etc., which, through complex interaction, will result in superior anti-cancer benefits and produce lasting immune memory. The compounds disclosed herein, including but not being limited to Cbo-23 and its derivatives, could be important components of cancer immunotherapy drug combinations and have potential in the treatment of cancer.

The present disclosure encompasses all stereoisomeric forms of the compounds of formulae (I) to (IV) or a pharmaceutically acceptable salt, hydrate, stereoisomer, solvate or prodrug thereof. Centers of asymmetry that are present in the compounds of formulae (I) to (IV) can all independently of one another have (R) configuration or (S) configuration. When bonds to the chiral carbon are depicted as straight lines in the structural formulae of the present disclosure, it is understood that both (R) and (S) are configurations of the chiral carbon, and hence both enantiomers and mixtures thereof are embraced within the formulae. When a particular configuration is depicted, that enantiomer (either (R) or (S), at that center)) is intended. Similarly, when a compound name is recited without a chiral designation for a chiral carbon, it is understood that both (R) and (S) are configurations of the chiral carbon, and hence individual enantiomers and mixtures thereof are embraced by the name.

The present disclosure includes all possible enantiomers, regioisomers, and diastereomers and mixtures of two or more stereoisomers, for example, mixtures of enantiomers and/or diastereomers, in all ratios. Thus, enantiomers are a subject of the present disclosure in enantiomerically pure form, both as levorotatory and as dextrorotatory antipodes, in the form of racemates and in the form of mixtures of the two enantiomers in all ratios. In the case of a cis/trans isomerism, the present disclosure includes both cis form and trans form as well as mixtures of these forms in all ratios. The preparation of individual stereoisomers can be carried out, if desired, by separation of a mixture by customary methods, for example by chromatography or crystallization, by the use of stereochemically uniform starting materials for the synthesis or by stereoselective synthesis. Optionally, a derivatization can be carried out before a separation of stereoisomers. The separation of a mixture of stereoisomers can be carried out at an intermediate step during the synthesis of a compound of formulae (I) to (IV) or a pharmaceutically acceptable salt, hydrate, stereoisomer, solvate or prodrug thereof, or it can be done on a final racemic product. Absolute stereochemistry may be determined by X-ray crystallography of crystalline products or crystalline intermediates which are derivatized, if necessary, with a reagent containing a stereogenic center of known configuration. Where compounds of the present disclosure are capable of tautomerization, all individual tautomers as well as mixtures thereof are included in the scope of the present disclosure. The present disclosure includes all such isomers, as well as salts, solvates (including hydrates) and solvated salts of such racemates, enantiomers, diastereomers and tautomers and mixtures thereof.

As used herein, the symbols and conventions used in these processes, schemes and examples, regardless of whether a particular abbreviation is specifically defined, are consistent with those used in the contemporary scientific literature, for example, the Journal of the American Chemical Society or the Journal of Biological Chemistry. Specifically, but without limitation, the following abbreviations may be used in the examples and throughout the specification: g (grams); mg (milligrams); mL (milliliters); µL (microliters); mM (millimolar); M (micromolar); Hz (Hertz); MHz (megahertz); mmol (millimoles); hr or hrs (hours); min (minutes); MS (mass spectrometry); ESI (electrospray ionization); TLC (thin layer chromatography); and HPLC (high pressure liquid chromatography). For all of the following examples, standard work-up and purification methods known to those skilled in the art can be utilized. Unless otherwise indicated, all temperatures are expressed in °C (degrees Centigrade). All reactions are conducted at room temperature unless otherwise noted. Synthetic methodologies illustrated herein are intended to exemplify the applicable chemistry through the use of specific examples and are not indicative of the scope of the disclosure.

The compounds of formulae (I) to (IV) of the present disclosure are prepared according to general chemical synthetic procedures. An exemplified synthetic route is shown below: 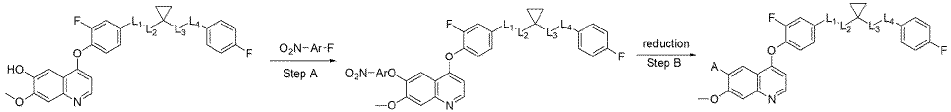
Step A involves a base and nucleophilic aromatic substitution reaction.
Step B is a reduction leading to formula (I).

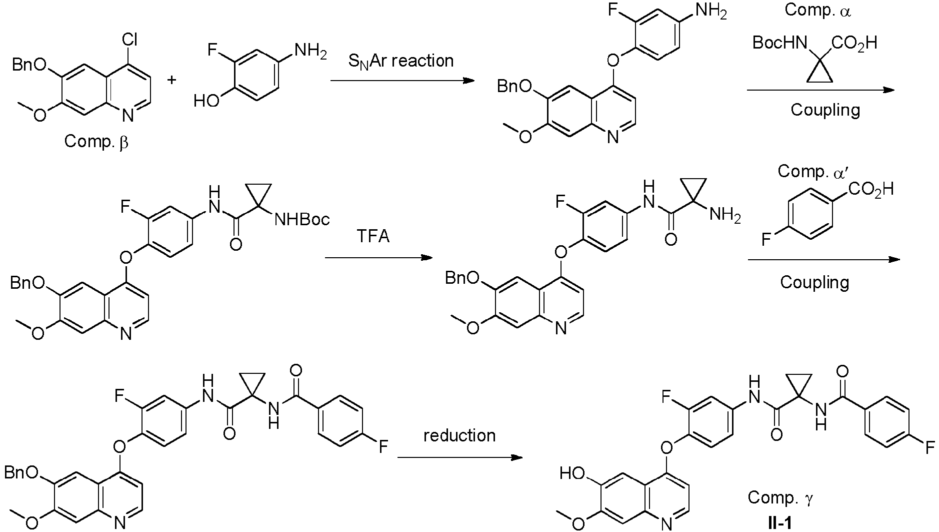

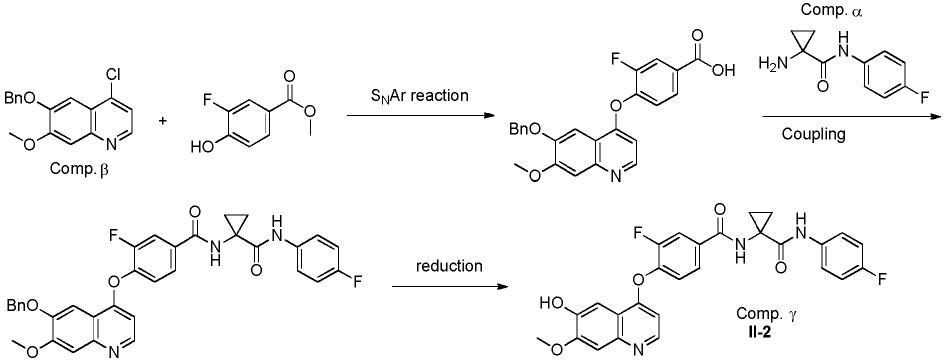

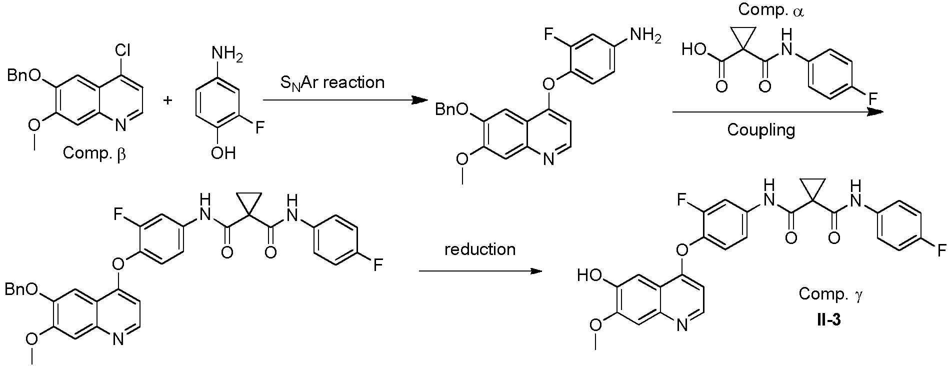

Other proper modifications of the process, e.g., using suitable protection/deprotection agents on groups susceptible to certain reaction conditions during the synthesis, isolating and purifying intermediates for subsequent reactions, selecting proper solvents, etc., can also be introduced by a skilled person in the art based on need. For example, the -OH group of a starting reagent or an intermediate in the route depicted above may be protected before reaction (e.g., Compound (β), having a protected group "-OBn," can be derived from an unprotected reagent and then used in subsequent reaction with Compound (α)), and the resulting (intermediate) product having protecting group(s) can be deprotected to give subsequent or final product(s) (e.g., Compound (γ)), etc.

### Pharmaceutical Compositions/Pharmaceutical Combinations

In another aspect, the present disclosure provides a pharmaceutical composition or pharmaceutical combination comprising a compound of any of formulae (I) to (IV), or a pharmaceutically acceptable salt, hydrate, stereoisomer, solvate or prodrug thereof, together with a pharmaceutically acceptable carrier.

The pharmaceutical composition or pharmaceutical combination can comprise one or more second or additional agents. In one embodiment, the second or additional agent is an HDAC inhibitor or an immune checkpoint inhibitor. In a further embodiment, the pharmaceutical composition or pharmaceutical combination comprises a compound described herein and an HDAC inhibitor. In a further embodiment, the pharmaceutical composition or pharmaceutical combination comprises a compound described herein and an immune checkpoint inhibitor. In a further embodiment, the pharmaceutical composition or pharmaceutical combination comprises a compound described herein, an HDAC inhibitor and an immune checkpoint inhibitor.

In one embodiment, the HDAC inhibitor comprises, but is not limited to, chidamide, GNTbm-38 (*E*)-*N*-(2-aminophenyl)-4-(4-(pyridin-3-yl)but-3-enamido)benzamide, entinostat, mocetinostat, vorinostat, romidepsin, belinostat, panobinostat, zabadinostat and OKI-179 (Bocodepsin).

In one embodiment, the immune checkpoint inhibitor can be used in the pharmaceutical combination described herein to stimulate an immune system against cancer cells and to treat a cancer. The immune checkpoint inhibitor is an anti-cytotoxic T-lymphocyte antigen-4 (CTLA-4) antibody or agent, anti-programmed cell death protein 1 (PD-1) antibody or agent, an anti-programmed death-ligand 1 (PD-L1) antibody or agent, an anti-T-cell immunoglobulin and mucin domain-3 (TIM-3) antibody or agent, anti-B- and T-lymphocyte attenuator (BTLA) antibody or agent, anti- V-domain Ig containing suppressor of T-cell activation (VISTA) antibody or agent, an anti-lymphocyte activation gene-3 (LAG-3) antibody or agent, KIR (killer-cell immunoglobulin-like receptor) inhibitor or antibody, A2AR (adenosine A2A receptor) inhibitor or antibody, CD276 inhibitor or antibody, or VTCN1 inhibitor or antibody. More preferably, the immune checkpoint inhibitor is pembrolizumab, lambrolizumab, pidilizumab, nivolumab, durvalumab, avelumab, or atezolizumab. Examples of PD-1 or PD-L1 inhibitors include, without limitation, humanized antibodies blocking human PD-1 such as lambrolizumab (anti-PD-1 Ab, trade name Keytruda) or pidilizumab (anti-PD-1 Ab), Bavencio (anti-PD-L1 Ab, avelumab), Imfinzi (anti-PD-L1 Ab, durvalumab), and Tecentriq (anti-PD-L1 Ab, atezolizumab) as well as fully human antibodies such as nivolumab (anti-PD-1 Ab, trade name Opdivo) and cemiplimab-rwlc (anti-PD-1 Ab, trade name Libtayo). Other PD-1 inhibitors may include presentations of soluble PD-1 ligand including, without limitation, PD-L2 Fc fusion protein, also known as B7-DC-Ig, or AMP-244 and other PD-1 inhibitors presently under investigation and/or development for use in therapy. In addition, immune checkpoint inhibitors may include, without limitation, humanized or fully human antibodies blocking PD-L1 such as durvalumab and MIH1 (anti-CD274 (PD-L1, B7-H1) monoclonal antibody) and other PD-L1 inhibitors presently under investigation.

The additional anti-cancer agent or therapy is any anti-cancer agent or therapy described herein or known in the art. In one embodiment, the additional anti-cancer agent or therapy is chemotherapy or, in particular, platinum-based doublet chemotherapy. In one embodiment, the additional anti-cancer agent is an anti-VEGF or an anti-VEGFR antibody or compound. In other embodiments, the anti-cancer agent is a platinum agent (e.g., cisplatin, carboplatin), a mitotic inhibitor (e.g., paclitaxel, albumin-bound paclitaxel, docetaxel, taxotere, docecad), a fluorinated Vinca alkaloid (e.g., vinflunine, javlor), vinorelbine, vinblastine, etoposide, or pemetrexed gemcitabin. In one embodiment, the additional anti-cancer agent is 5-flurouracil (5-FU). In certain embodiments, the additional anti-cancer agent is any other anti-cancer agent known in the art.

To prepare the pharmaceutical compositions or pharmaceutical combinations of the present disclosure, one or more compounds of the present disclosure as the active ingredient may be thoroughly admixed with a pharmaceutical carrier according to conventional pharmaceutical compounding techniques, which carrier may take a wide variety of forms depending of the form of preparation desired for administration, e.g., oral or parenteral, such as intramuscular. In preparing the compositions in oral dosage form, any of the usual pharmaceutical media may be employed. Thus, for liquid oral preparations, such as, for example, suspensions, elixirs and solutions, suitable carriers and additives include water, glycols, oils, alcohols, flavoring agents, preservatives, coloring agents and the like; for solid oral preparations such as, for example, powders, capsules, caplets, gel caps and tablets, suitable carriers and additives include starches, sugars, diluents, granulating agents, lubricants, binders, disintegrating agents and the like. Because of their ease of administration, tablets and capsules represent the most advantageous oral dosage unit form, in which case solid pharmaceutical carriers are obviously employed. If desired, tablets may be sugar-coated or enteric-coated by standard techniques. For parenterals, the carrier will usually comprise sterile water, though other ingredients may be included, for example, for purposes such as aiding solubility or for preservation. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above.

The liquid forms in which the novel compositions of the present disclosure may be incorporated for administration orally or by injection include: aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions include: synthetic and natural gums such as tragacanth, acacia, alginate, dextran, sodium carboxymethylcellulose, methylcellulose, polyvinyl-pyrrolidone or gelatin.

Tablets and capsules for oral administration are normally presented in unit dose form and contain conventional excipients such as binders, fillers (including cellulose, mannitol, lactose), diluents, tableting agents, lubricants (including magnesium stearate), detergents, disintegrants (e.g., polyvinylpyrrolidone and starch derivatives such as sodium glycolate starch), coloring agents, flavoring agents, and wetting agents (for example sodium lauryl sulfate).

The oral solid compositions can be prepared by conventional methods of blending, filling or tableting. The blending operation can be repeated to distribute the active principle throughout compositions containing large quantities of fillers. Such operations are conventional.

For parenteral administration, fluid unit dosages can be prepared containing the compound and a sterile vehicle. The compound can be either suspended or dissolved, depending on the vehicle and concentration. The parenteral solutions are normally prepared by dissolving the compound in a vehicle, sterilizing by filtration, filling suitable vials and sealing. Advantageously, adjuvants such as local anaesthetics, preservatives and buffering agents can also be dissolved in the vehicle. To increase stability, the composition can be frozen after having filled the vials and removed the water under vacuum. Parenteral suspensions are prepared in substantially the same manner, except that the compound can be suspended in the vehicle instead of being dissolved, and sterilized by exposure to ethylene oxide before suspension in the sterile vehicle. Advantageously, a surfactant or wetting agent can be included in the composition to facilitate uniform distribution of the compound of the application.

A pharmaceutical preparation for administration by inhalation can be delivered from an insufflator or a nebulizer pressurized pack.

### Therapeutic Applications

In another aspect, the present disclosure provides a method for suppressing angiogenesis in a subject in need thereof, comprising administering an effective amount of a compound or a pharmaceutical composition or pharmaceutical combination described herein to the subject.

In another aspect, the present disclosure provides a method for suppressing metastasis in a subject in need thereof, which comprises administering an effective amount of a compound or a pharmaceutical composition or pharmaceutical combination described herein to the subject.

In another aspect, the present disclosure provides a method for suppressing oncogenesis in a subject in need thereof, comprising administering an effective amount of a compound or a pharmaceutical composition or pharmaceutical combination described herein to the subject.

In another aspect, the present disclosure provides a method for suppressing immune regulation activities in a subject in need thereof, comprising administering an effective amount of a compound or a pharmaceutical composition or pharmaceutical combination described herein to the subject.

In another aspect, the present disclosure provides a compound or a pharmaceutical composition or pharmaceutical combination described herein for use in a method for suppressing angiogenesis, metastasis, oncogenesis and/or immune regulation activities in a subject in need thereof, the method comprising administering an effective amount of a compound or a pharmaceutical composition or pharmaceutical combination described herein to the subject. In another aspect, the present disclosure provides use of a compound or a pharmaceutical composition or pharmaceutical combination described herein for suppressing angiogenesis, metastasis, oncogenesis and/or immune regulation activities in a subject in need thereof. In another aspect, the present disclosure provides use of a compound or a pharmaceutical composition or pharmaceutical combination described herein in the manufacture of a medicament for suppressing angiogenesis, metastasis, oncogenesis and/or immune regulation activities in a subject in need thereof.

In another aspect, the present disclosure provides a method for treating diseases or conditions associated with tyrosine kinases in a subject in need thereof, comprising administering an effective amount of a compound or a pharmaceutical composition or pharmaceutical combination described herein to the subject.

In another aspect, the present disclosure provides a method for treating tumors or cancers in a subject in need thereof, comprising administering an effective amount of a compound or a pharmaceutical composition or pharmaceutical combination described herein to the subject.

In another aspect, the present disclosure provides a compound, pharmaceutical composition or pharmaceutical combination described herein for use in a method for treating diseases or conditions associated with tyrosine kinases, or treating tumors or cancers in a subject in need thereof, the method comprising administering an effective amount of a compound, pharmaceutical composition or pharmaceutical combination described herein to the subject. In another aspect, the present disclosure provides use of a compound, pharmaceutical composition or pharmaceutical combination described herein for treating diseases or conditions associated with tyrosine kinases, or treating tumors or cancers in a subject in need thereof. In another aspect, the present disclosure provides use of a compound, pharmaceutical composition or pharmaceutical combination described herein in the manufacture of a medicament for treating diseases or conditions associated with tyrosine kinases, or treating tumors or cancers in a subject in need thereof.

In another aspect, the present disclosure provides a cancer immunotherapy for treating tumors or cancers in a subject in need thereof, comprising administering an effective amount of a compound, pharmaceutical composition or pharmaceutical combination described herein to the subject, for example, by regulating the tumor microenvironment, including reducing tumor hypoxia, reducing lactic acid accumulation, activating CTL, inhibiting the number and activity of immunosuppressive cells, to ultimately achieve superior anti-cancer benefits and/or produce lasting immune memory.

In another aspect, the present disclosure provides a compound, pharmaceutical composition or pharmaceutical combination described herein for use in a cancer immunotherapy for treating tumors or cancers in a subject in need thereof, the method comprising administering an effective amount of a compound, pharmaceutical composition or pharmaceutical combination described herein to the subject. In another aspect, the present disclosure provides use of a compound or a pharmaceutical composition or pharmaceutical combination described herein in a cancer immunotherapy for treating tumors or cancers in a subject in need thereof. In another aspect, the present disclosure provides use of a compound, pharmaceutical composition or pharmaceutical combination described herein in the manufacture of a medicament in a cancer immunotherapy for treating tumors or cancers in a subject in need thereof.

In another aspect, the present disclosure provides a method for treating tumors or cancers in a subject in need thereof with enhanced objective response rate (ORR) as compared to treatment of tumors or cancers based on a pharmaceutical composition or pharmaceutical combination comprising conventional TK inhibitors, comprising administering an effective amount of a compound or a pharmaceutical composition or pharmaceutical combination described herein to the subject.

In another aspect, the present disclosure provides a compound, pharmaceutical composition or pharmaceutical combination described herein for use in a method for treating tumors or cancers in a subject in need thereof with enhanced objective response rate (ORR) as compared to treatment of tumors or cancers based on a pharmaceutical composition or pharmaceutical combination comprising conventional TK inhibitors, the method comprising administering an effective amount of a compound, pharmaceutical composition or pharmaceutical combination described herein to the subject. In another aspect, the present disclosure provides use of a compound, pharmaceutical composition or pharmaceutical combination described herein for treating tumors or cancers in a subject in need thereof with enhanced objective response rate (ORR) as compared to treatment of tumors or cancers based on a pharmaceutical composition or pharmaceutical combination comprising conventional TK inhibitors. In another aspect, the present disclosure provides use of a compound, pharmaceutical composition or pharmaceutical combination described herein in the manufacture of a medicament for treating tumors or cancers in a subject in need thereof with enhanced objective response rate (ORR) as compared to treatment of tumors or cancers based on a pharmaceutical composition or pharmaceutical combination comprising conventional TK inhibitors.

Examples of cancer which can be treated in accordance with the present disclosure include, but are not limited to, invasive breast carcinoma, adenocarcinoma, lung cancer (non-small cell, squamous cell carcinoma, adenocarcinoma, and large cell lung cancer), liver cancer, colorectal cancer, brain, head and neck cancer (e.g., neuro/glioblastoma), breast cancer, ovarian cancer, transitional cell carcinoma of the bladder, prostate cancer, oral squamous cell carcinoma, bone sarcoma, adrenocortical cancer, gastrointestinal tumors including colorectal cancer, biliary tract cancer such as gallbladder carcinoma (GBC), bladder cancer, esophageal cancer, gastric cancer, cervical cancer, salivary gland cancer, diarrhea benign neoplasm, ductal carcinoma in situ, paronychia, cholangiocarcinoma, kidney cancer, pancreatic cancer, medulloblastoma, glioblastoma, luminal, HER2-positive and triple negative mammary tumors, hematologic malignancies and leukemia (acute myelogenous leukemia (AML), B-precursor cell acute lymphoblastic leukemia (ALL), a fraction of T-cell ALL, and chronic myelogenous leukemia (CML)).

The compounds, or pharmaceutically acceptable salts thereof, are administered orally, nasally, transdermally, pulmonary, inhalationally, buccally, sublingually, intraperitoneally, subcutaneously, intramuscularly, intravenously, rectally, intrapleurally, intrathecally or parenterally. In one embodiment, the compound is administered orally. One skilled in the art will recognize the advantages of certain routes of administration.

The dosage regimen utilizing the compounds is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter, or arrest the progress of the condition.

The present disclosure having now been described by way of written description, those of skill in the art will recognize that the present disclosure can be practiced in a variety of embodiments and that the foregoing description and the examples below are for illustrative purposes only and do not limit the claims that follow.

### Examples

Materials and methods of preparing the exemplified compounds of the present disclosure are described below.

All commercial chemicals and solvents were reagent grade and used without further purification unless otherwise stated. All reactions were monitored for completion by thin layer chromatography using Merck 60 F₂₅₄ silica gel glass backed plates (20 × 20 cm). The resulting chromatograms were visualized under UV irradiation (254 nm). ¹H NMR spectra were recorded on Bruker AVANCE III HD 600 MHz Spectrometer and the chemical shifts were recorded in parts per million (ppm, *δ*). Multiplicities are recorded as s (singlet), br s (broad singlet), d (doublet), t (triplet), q (quartet), quin (quintet), sex (sextet), and m (multiplet). Coupling constants (*J*) are expressed in hertz. Electrospray mass spectra (ESMS) were recorded as *m*/*z* values using Waters mass spectrometer. Purity of the final compound was determined with Waters ACQUITY Arc system using C₁₈ column (Waters XSelect HSS T3 5 µm, 4.6 mm x 250 mm) operating at 40 °C. Elution was carried out using water containing 0.1% trifluoroacetic acid as mobile phase A and methanol as mobile phase B. Elution condition: at 0 min, phase A 90% + phase B 10%; at 6 min, phase A 70% + phase B 30%; at 12 min, phase A 50% + phase B 50%; at 18 min, phase A 10% + phase B 90%; at 23 min, phase A 90% + phase B 10%. The flow-rate of the mobile phase was 1 mL/min, the injection volume of the sample was 20 µL, and the run time was 30 minutes. Peaks were detected at 210-400 nm. Purity of final compound was found to be >95%.

### Preparation Examples

### Example 1 Cbo-02

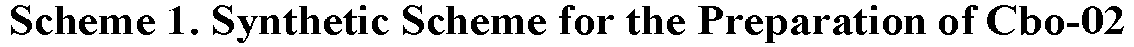

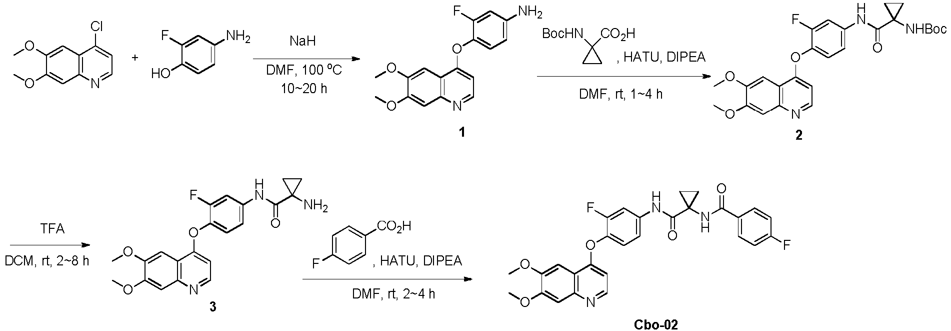

### 4-((6,7-diMethoxyquinolin-4-yl)oxy)-3-fluoroaniline (1)

To a solution of 4-chloro-6,7-dimethoxyquinoline (1 eq), 4-amino-2-fluorophenol (1.2~2 eq), and NaH (1.2∼2.2 eq) in DMF was stirred at 100 °C for 10-20 h. The mixture was diluted with EA, washed with water, brine, and dried over Na₂SO₄. The mixture was filtered and concentrated to dryness. The crude product was purified by column chromatography to give compound 1 (yield 38%) as a beige solid.

### tert-butyl (1-((4-((6,7-diMethoxyquinolin-4-yl)oxy)-3-fluorophenyl)carbamoyl)c-ylopropyl)carbamate (2)

To a solution of compound 1 (1 eq), 1-((*tert*-butoxycarbonyl)ami-no)cyclopropane-1-carboxylic acid (1~1.5 eq) and HATU (1~1.5 eq) in DMF was added DIPEA (2∼4 eq). The mixture was stirred at rt. for 1-4 h. The mixture was diluted with EA, washed with water, brine, and dried over Na₂SO₄. The mixture was filtered and concentrated to dryness. The crude product was purified by column chromatography to give compound 2 (yield 97%) as a yellow solid.

### 1-Amino-N (4-((6,7-dimethoxyquinolin-4-yl)oxy)-3-fluorophenyl)cyclopropane-1-carboxamide (3)

To a solution of compound **2** (1 eq) in DCM was added TFA (1 eq). The mixture was stirred at rt. for 2~8 h. The mixture was diluted with DCM, neutralized with sat. NaHCO_{3(aq)} to pH = 7. The organic layer was washed with water, brine, and dried over Na₂SO₄. The mixture was filtered and concentrated to dryness to give compound **3** (yield 86%) as a yellow solid.

### N-(1-((4-((6,7-diMethoxyquinolin-4-yl)oxy)-3-fluorophenyl)carbamoyl)cyclopropyl)-4-fluorobenzamide (Cbo-02)

To a solution of compound **3** (1 eq), 4-fluorobenzoic acid (1~1.5 eq) and HATU (1∼1.5 eq) in DMF, DIPEA (1∼3 eq) was added. The mixture was stirred at rt. for 2-4 h. The mixture was diluted with EA, washed with water, brine, and dried over Na₂SO₄. The mixture was filtered and concentrated to dryness. The crude product was purified by column chromatography to give **Cbo-02** (yield 78%) as a white solid.

¹H NMR (600 MHz, DMSO-d6) *δ* 9.92 (s, 1H), 9.09 (s, 1H), 8.46 (d, *J =* 5.2 Hz, 1H), 8.04 (dd, *J=* 8.6, 5.6 Hz, 2H), 7.87 (dd, *J=* 13.3, 2.3 Hz, 1H), 7.56 (d, *J=* 7.5 Hz, 1H), 7.52 (s, 1H), 7.41 (t, *J=* 8.0 Hz, 2H), 7.33 (t, *J=* 8.8 Hz, 2H), 6.44 (d, *J=* 5.2 Hz, 1H), 3.95 (s, 3H), 3.94 (s, 3H), 1.51 (q, *J* = 3.9 Hz, 2H), 1.15 (q, *J=* 4.0 Hz, 2H). LCMS (ESI) *m*/*z* 520.4 [M+H]⁺. HPLC purity: 98.88%.

### Example 2 Cbo-03

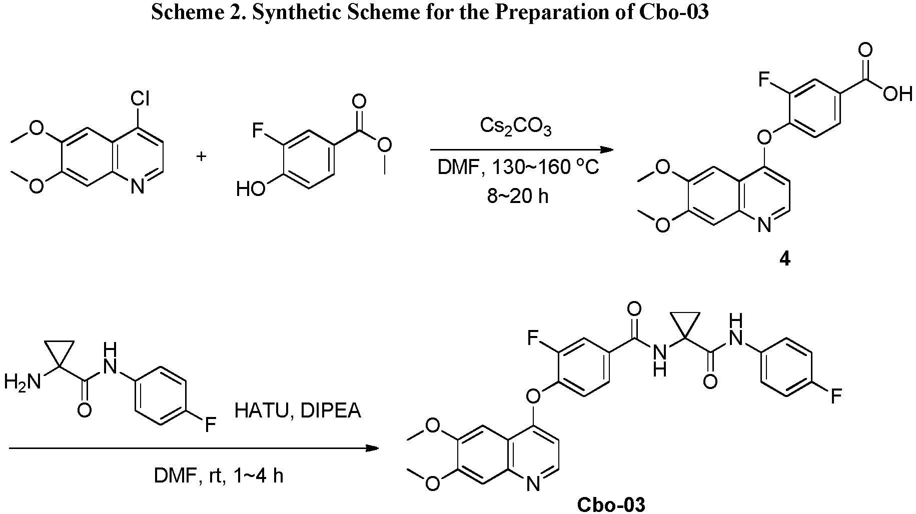

### 4-((6,7-diMethoxyquinolin-4-yl)oxy)-3-fluorobenzoic acid (4)

A solution of 4-chloro-6,7-dimethoxyquinoline (1 eq), methyl 3-fluoro-4-hydroxybenzoate (1∼1.5 eq), and Cs₂CO₃ (2~3 eq) in DMF was stirred at 130~160 °C for 8-20 h. The mixture was diluted with EA, and neutralized with 2 N HCl to a pH of 6. The mixture was washed with water, brine, and dried over Na₂SO₄. The mixture was filtered and concentrated to dryness. The crude product was purified by column chromatography to give compound **4** (yield 13%) as a beige solid.

### 4-((6,7-diMethoxyquinolin-4-yl)oxy)-3-fluoro-N-(1-((4-fluorophenyl)carbamoy-l)cyclopropyl)benzamide (Cbo-03)

To a solution of compound **4** (1 eq), 1-amino N-(4-fluorophenyl)cyclopropane-1-carboxamide (1~1.5 eq) and HATU (1~1.5 eq) in DMF, DIPEA (2~3 eq) was added. The mixture was stirred at rt. for 1-4 h. The mixture was diluted with EA, washed with water, brine, and dried over Na₂SO₄. The mixture was filtered and concentrated to dryness. The crude product was purified by column chromatography to give **Cbo-03** (yield 44%) as a white solid..

¹H NMR (600 MHz, DMSO-d6) *δ* 9.71 (s, 1H), 9.19 (s, 1H), 8.52 (d, *J =* 5.2 Hz, 1H), 8.08 (dd, *J* = 11.6, 1.8 Hz, 1H), 7.93 (dd, *J* = 8.4,1.5 Hz, 1H), 7.63-7.55 (m, 2H), 7.52 (s, 1H), 7.44 (s, 1H), 7.14 (t, *J=* 8.9 Hz, 2H), 6.49 (d, *J=* 5.1 Hz, 1H), 3.96 (s, 3H), 3.95 (s, 3H), 1.49 (q, *J=* 3.9 Hz, 2H), 1.13 (q, *J=* 3.9 Hz, 2H). LCMS (ESI) *m*/*z* 520.4 [M+H]⁺. HPLC purity: 98.98%.

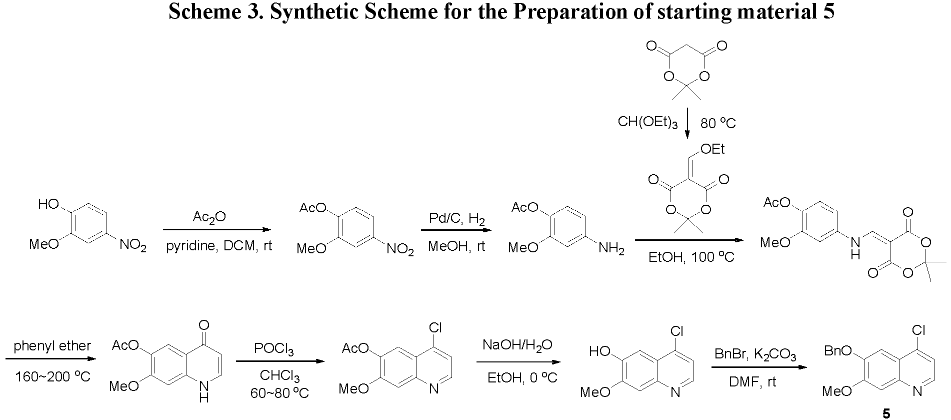

### 2-Methoxy-4-nitrophenyl acetate

To a solution of 2-methoxy-4-nitrophenol (1 eq) in DCM was added pyridine (1∼1.5 eq) and Ac₂O (1~1.5 eq). The mixture was stirred at rt. for 2-5 h. The mixture was washed with water, brine, and dried over Na₂SO₄. The mixture was filtered and concentrated to dryness to give 2-methoxy-4-nitrophenyl acetate (12.3 g, 98%) as a yellow solid.

### 4-Amino-2-methoxyphenyl acetate

To a solution of 2-methoxy-4-nitrophenyl acetate (1 eq) in MeOH, Pd/C (3-5% w/w) was added, and the mixture was stirred at rt. under hydrogen atmosphere for 8~20 h. The mixture was filtered through a Celite pad and the filtrate was concentrated to dryness. The crude product was purified by column chromatography to give 4-amino-2-methoxyphenyl acetate (yield 62%) as a yellow oil.

### 5-(Ethoxymethylene)-2,2-dimethyl-1,3-dioxane-4,6-dione

A solution of 2,2-dimethyl-1,3-dioxane-4,6-dione in triethylorthoformate was stirred at 80 °C for 2-5 h. The mixture was concentrated to dryness to give 5-(ethoxymethylene)-2,2-dimethyl-1,3-dioxane-4,6-dione (yield 91%) as a white solid.

### 4-(((2,2-dimethyl-4,6-dioxo-1,3-dioxan-5-ylidene)methyl)amino)-2-methoxyphenyl acetate

A solution of 4-amino-2-methoxyphenyl acetate (1 eq) and 5-(ethoxymethylene)-2,2-dimethyl-1,3-dioxane-4,6-dione (1 eq) in EtOH was stirred at 100 °C for 1~4 h. The resulting solid was collected by filtration to give 4-(((2,2-dimethyl-4,6-dioxo-1,3-dioxan-5-ylidene)methyl)amino)-2-methoxyphenyl acetate (yield 88%) as a yellow solid.

### 7-Methoxy-4-oxo-1,4-dihydroquinolin-6-yl acetate

A solution of 4-(((2,2-dimethyl-4,6-dioxo-1,3-dioxan-5-ylidene)methyl)amino)-2-methoxyphenyl acetate in diphenylether was stirred at 160-200 °C for 1-4 h. The resulting solid was collected by filtration, and washed with hexanes to give 7-methoxy-4-oxo-1,4-dihydroquinolin-6-yl acetate (yield 81%) as a brown solid.

### 4-Chloro-7-methoxyquinolin-6-yl acetate

To a solution of 7-methoxy-4-oxo-1,4-dihydroquinolin-6-yl acetate (1 eq) in CHCl₃, POCl₃ (2∼6 eq) was added, and the mixture was stirred at 60~80 °C for 1-2 h. The mixture was concentrated to dryness. The residue was diluted with EA and neutralized with sat. NaHCO_{3(aq.)} to pH 7. The organic layer was washed with water, brine, and dried over Na₂SO₄. The mixture was filtered and concentrated to dryness. The crude product was purified by column chromatography to give 4-chloro-7-methoxyquinolin-6-yl acetate (yield 60%) as a yellow solid.

### 4-Chloro-7-methoxyquinolin-6-ol

To a solution of 4-chloro-7-methoxyquinolin-6-yl acetate (1 eq) in EtOH, NaOH (1∼1.2 eq) was added, and the mixture stirred at 0 °C for 6~16 h. The mixture was acidified with 1N HCl to pH 7. The resulting solid was collected by filtration to give 4-chloro-7-methoxyquinolin-6-ol (yield 88%) as a white solid.

### 6-(Benzyloxy)-4-chloro-7-methoxyquinoline (5)

To a solution of 4-chloro-7-methoxyquinolin-6-ol (1 eq) in DMF, K₂CO₃ (2~3 eq) and benzyl bromide (1~1.5 eq) were added. The mixture was stirred at rt. for 6-16 h. Water was added to the mixture and the resulting solid was collected by filtration to give compound **5** (yield 81%) as a white solid.

### Example 3 Cbo-07

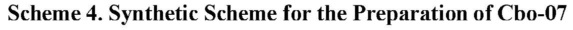

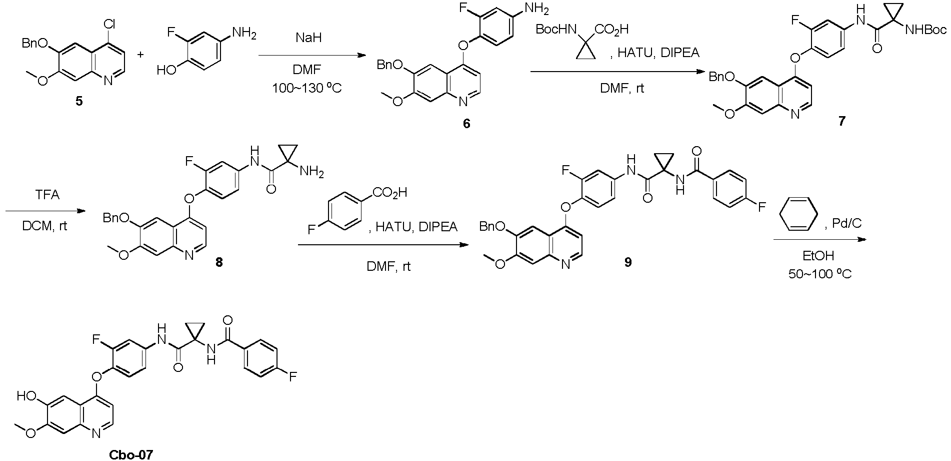

### 4-((6-(Benzyloxy)-7-methoxyquinolin-4-yl)oxy)-3-fluoroaniline (6)

A solution of compound **5** (1 eq), 4-amino-2-fluorophenol (2~4 eq), and NaH (2~4 eq) in DMF was stirred at 100-130 °C for 8-20 h. The mixture was diluted with EA, washed with water, brine, and dried over Na₂SO₄. The mixture was filtered and concentrated to dryness. The crude product was purified by column chromatography to give compound **6** (yield 66%) as a beige solid.

### tert-butyl (1-((4-((6-(Benzyloxy)-7-methoxyquinolin-4-yl)oxy)-3-fluorophenyl)carbamoyl)cyclopropyl)carbamate (7)

To a solution of compound **6,** 1-((*tert*-butoxycarbonyl)amino)cyclopropane-1-carboxylic acid (1 eq) and HATU (1∼1.5 eq) in DMF, DIPEA (1∼3 eq) was added. The mixture was stirred at rt. for 2-8 h. The mixture was diluted with EA, washed with water, brine, and dried over Na₂SO₄. The mixture was filtered and concentrated to dryness. The crude product was purified by column chromatography to give compound **7** (yield 89%) as a yellow solid.

### 1-Amino-N-(4-((6-(benzyloxy)-7-methoxyquinolin-4-yl)oxy)-3-fluorophenyl)cyclopropane-1-carboxamide (8)

To a solution of compound 7 (1 eq) in DCM, TFA (3~10 eq) was added. The mixture was stirred at rt. for 2-8 h. The mixture was diluted with DCM and neutralized with sat. NaHCO_{3(aq.)} to a pH of 7. The organic layer was washed with water, brine, and dried over Na₂SO₄. The mixture was filtered and concentrated to dryness to give compound **8** (yield 99%) as a yellow solid.

### N-(1-((4-((6-(Benzyloxy)-7-methoxyquinolin-4-yl)oxy)-3-fluorophenyl)carbamoyl)cyclopropyl)-4-fluorobenzamide (9)

To a solution of compound **8** (1 eq), 4-fluorobenzoic acid (1∼1.5 eq) and HATU (1∼1.5 eq) in DMF, DIPEA (2~3 eq) was added. The mixture was stirred at rt. for 2-4 h. The mixture was diluted with EA, washed with water, brine, and dried over Na₂SO₄. The mixture was filtered and concentrated to dryness. The crude product was purified by column chromatography to give compound 9 (yield 83%) as a white solid.

### 4-Fluoro-N-(1-((3-fluoro-4-((6-hydroxy-7-methoxyquinolin-4-yl)oxy)phenyl)carbamoyl)cyclopropyl)benzamide (Cbo-07)

A solution of compound **9** (1 eq), 1,4-cyclohexadiene (5∼10 eq) and Pd/C (20% w/w) in EtOH was stirred at 50-100 °C for 1-4 h. The mixture was filtered through a celite pad and the filtrate was concentrated to dryness. The crude product was purified by column chromatography to give **Cbo-07** (yield 95%) as a white solid.

¹H NMR (600 MHz, DMSO-*d*6) *δ* 10.06 (s, 1H), 9.91 (s, 1H), 9.10 (s, 1H), 8.39 (d, *J* = 5.2 Hz, 1H), 8.03 (dd, *J =* 8.5, 5.7 Hz, 2H), 7.85 (dd, *J =* 13.3, 2.1 Hz, 1H), 7.54 (d, *J =* 9.4 Hz, 1H), 7.47 (s, 1H), 7.39-7.30 (m, 4H), 6.39 *(d, J =* 5.2 Hz, 1H), 3.94 (s, 3H), 1.50 (q, *J* = 3.8 Hz, 2H), 1.14 (d, *J =* 3.9 Hz, 2H). LCMS (ESI) *m*/*z* 506.2 [M+H]⁺. HPLC purity: 98.02%.

### Example 4 Cbo-05, 06, 08-10, and 14 and 10a-10f

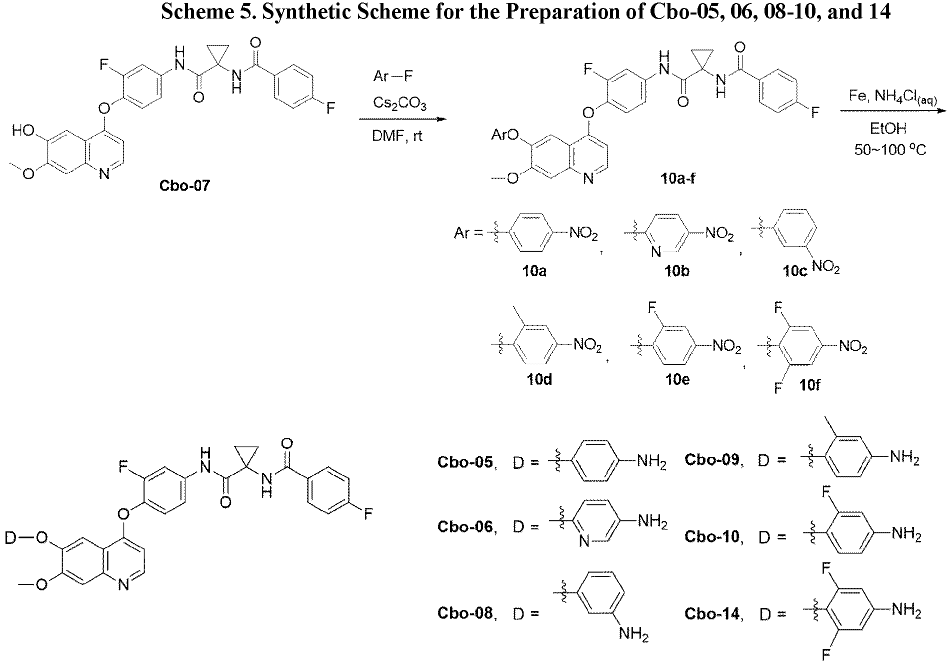

### General procedure for the synthesis of 10a-f

To a solution of Cbo-07 (1eq) and Cs₂CO₃ (2~3eq) in DMF,-fluoronitrobenzene or *m-*fluoronitrobenzene (1∼1.5 eq) was added. The mixture was stirred at rt. for 1-4 h. The mixture was diluted with EA, washed with water, brine, and dried over Na₂SO₄. The mixture was filtered and concentrated to dryness. The crude product was purified by column chromatography to give the desired products.

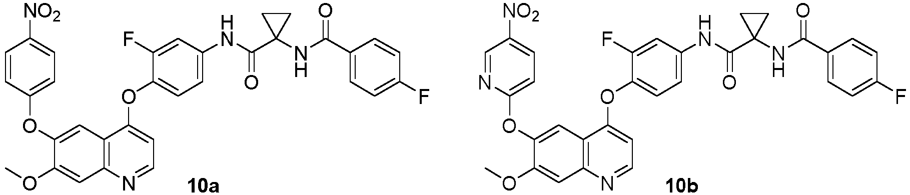

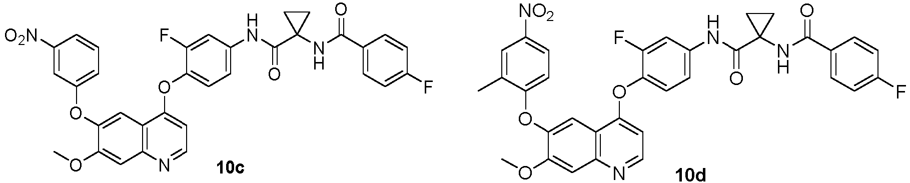

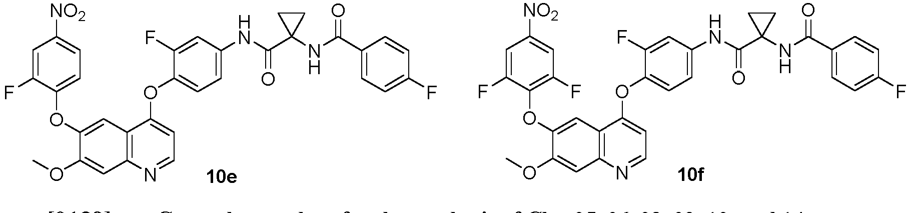

### General procedure for the synthesis of Cbo-05, 06, 08, 09, 10, and 14

To a solution of nitro adduct (1eq), Fe (3~6eq) in EtOH / sat. NH₄Cl_{(aq.)} was added. The mixture was stirred at 50-100 °C for 1-4 h. The mixture was filtered through a Celite pad. The filtrate was diluted with EA, washed with water, brine, and dried over Na₂SO₄. The mixture was filtered and concentrated to dryness. The crude product was purified by column chromatography to give the desired products.

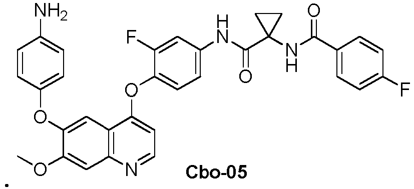

Yield: 65 mg, 43%. ¹H NMR (600 MHz, DMSO-d6) *δ* 9.86 (s, 1H), 9.03 (s, 1H), 8.47 (d, *J* = 5.2 Hz, 1H), 8.03 (d, *J* = 5.8 Hz, 1H), 7.82 (d, *J =* 13.1 Hz, 1H), 7.51 (d, *J =* 8.6 Hz, 1H), 7.50 (s, 1H), 7.35-7.28 (m, 3H), 7.24 (s, 1H), 6.86 (d, *J =* 8.5 Hz, 2H), 6.62 *(d, J =* 8.5 Hz, 2H), 6.40 (d, *J =* 5.2 Hz, 1H), 5.03 (s, 2H), 4.00 (s, 3H), 1.50 (q, *J =* 3.7 Hz, 2H), 1.14 (q, *J =* 3.7 Hz, 2H). LCMS (ESI) *m*/*z* 597.3 [M+H]⁺. HPLC purity: 95.69%.

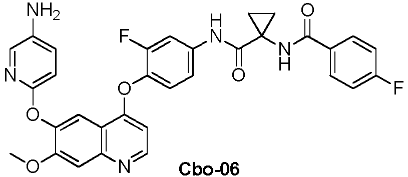

Yield: 138 mg, 73%. ¹H NMR (600 MHz, DMSO-d6) *δ* 9.88 (s, 1H), 9.04 (s, 1H), 8.54 (d, *J =* 5.2 Hz, 1H), 8.04 (d, *J =* 5.8 Hz, 1H), 8.02 (d, *J =* 5.7 Hz, 1H), 7.84 (dd, *J =* 13.1, 1.7 Hz, 1H), 7.63 (s, 1H), 7.53 (d, *J =* 12.4 Hz, 1H), 7.52 (s, 1H) 7.47 (d, *J =* 2.7 Hz, 1H), 7.37 (t, *J* = 9.1 Hz, 1H), 7.33 (t, *J* = 8.8 Hz, 2H), 7.10 (dd, *J =* 8.6, 2.8 Hz, 1H), 6.85 (d, *J =* 8.6 Hz, 1H), 6.45 (d, *J =* 5.2 Hz, 1H), 5.05 (s, 2H), 3.90 (s, 3H), 1.50 (q, *J =* 3.8 Hz, 2H), 1.14 (q, *J =* 3.8 Hz, 2H). LCMS (ESI) *m*/*z* 598.2 [M+H]⁺. HPLC purity: 99.16%.

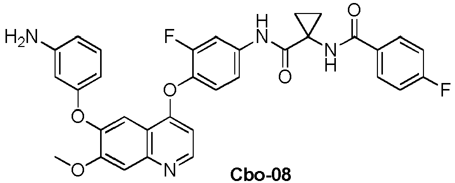

Yield: 86 mg, 53%. ¹H NMR (600 MHz, DMSO-d6) *δ* 9.91 (s, 1H), 9.09 (s, 1H), 8.53 (d, *J =* 5.2 Hz, 1H), 8.03 (dd, *J* = 8.7, 5.6 Hz, 2H), 7.84 (dd, *J* = 13.3, 2.3 Hz, 1H), 7.56 (d, *J =* 3.4 Hz, 2H), 7.53 (d, *J=* 8.9 Hz, 1H), 7.38-7.29 (m, 3H), 7.01 (t, *J=* 7.9 Hz, 1H), 6.44 (d, *J=* 5.2 Hz, 1H), 6.33 (d, *J=* 7.2 Hz, 1H), 6.21-6.17 (m, 2H), 5.23 (s, 2H), 3.97 (s, 3H), 1.50 (q, *J=* 3.9 Hz, 2H), 1.14 (q, *J=* 4.0 Hz, 2H). LCMS (ESI) *m*/*z* 597.3 [M+H]⁺. HPLC purity: 97.69%.

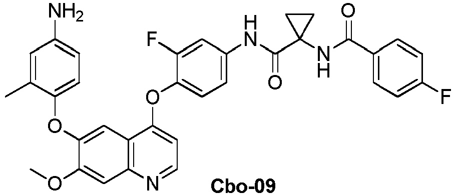

Yield: 90 mg, 47%. ¹H NMR (600 MHz, DMSO-d6) *δ* 9.87 (s, 1H), 9.06 (s, 1H), 8.46 (d, *J =* 5.2 Hz, 1H), 8.03 (dd, *J* = 8.9, 5.6 Hz, 2H), 7.81 (dd, *J* = 13.3, 2.4 Hz, 1H), 7.53-7.49 (m, 2H), 7.35-7.28 (m, 3H), 7.05 (s, 1H), 6.78 (d, *J=* 8.5 Hz, 1H), 6.52 (d, *J=* 2.6 Hz, 1H), 6.46 (dd, *J=* 8.5, 2.7 Hz, 1H), 6.39 (d, *J=* 5.1 Hz, 1H), 4.97 (s, 2H), 4.02 (s, 3H), 1.97 (s, 3H), 1.49 (q, *J=* 3.9 Hz, 2H), 1.13 (q, *J* = 4.0 Hz, 2H). LCMS (ESI) *m*/*z* 611.3 [M+H]⁺. HPLC purity: 97.80%.

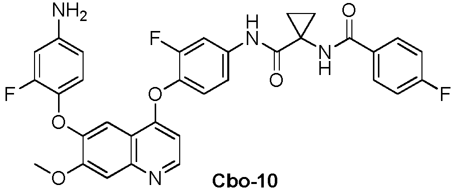

Yield: 135 mg, 64%. ¹H NMR (600 MHz, DMSO-d6) *δ* 9.90 (s, 1H), 9.09 (s, 1H), 8.49 (d, *J=* 5.2 Hz, 1H), 8.06-7.99 (m, 2H), 7.82 (dd, *J=* 13.3, 2.4 Hz, 1H), 7.52 (s, 1H), 7.51 (d, *J=* 1.4 Hz, 1H), 7.32 (t, *J*= 8.8 Hz, 3H), 7.17 (s, 1H), 7.01 (t, *J =* 9.1 Hz, 1H), 6.52 (dd, *J=* 13.2, 2.6 Hz, 1H), 6.44-6.40 (m, 2H), 5.39 (s, 2H), 4.02 (s, 3H), 1.49 (q, *J=* 4.0 Hz, 2H), 1.13 (q, *J=* 4.0 Hz, 2H). LCMS (ESI) *m*/*z* 615.3 [M+H]⁺. HPLC purity: 98.10%.

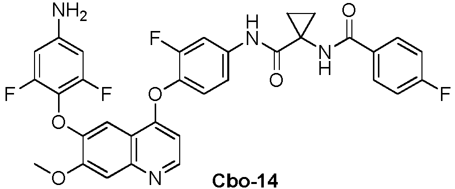

Yield: 226 mg, 91%. ¹H NMR (600 MHz, DMSO-d6) *δ* 9.90 (s, 1H), 9.10 (s, 1H), 8.51 (d, *J=* 5.2 Hz, 1H), 8.05-8.00 (m, 2H), 7.83 (dd, *J=* 13.3, 2.4 Hz, 1H), 7.54 (s, 1H), 7.52 (dd, *J=* 9.0, 1.4 Hz, 1H), 7.33 (q, *J=* 9.0 Hz, 3H), 7.18 (s, 1H), 6.44 (d, *J=* 5.0 Hz, 1H), 6.39 (d, *J=* 10.6 Hz, 2H), 5.76 (s, 2H), 4.04 (s, 3H), 1.50 (q, *J =* 4.0 Hz, 2H), 1.14 (q, *J =* 4.0 Hz, 2H). LCMS (ESI) *m*/*z* 633.3 [M+H]⁺. HPLC purity: 99.34%.

### Example 5 Cbo-20

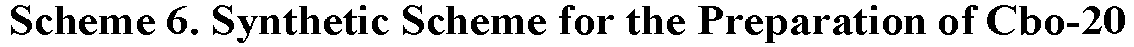

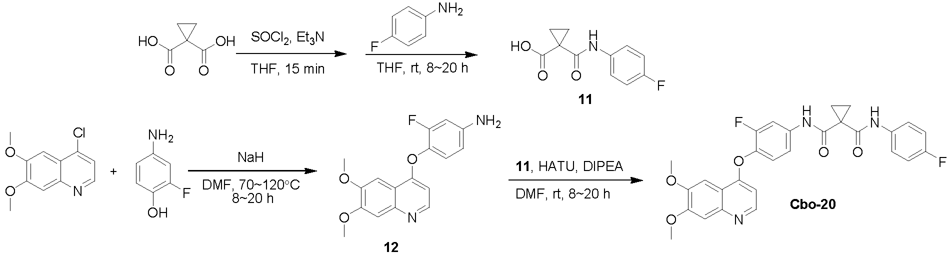

### 1-((4-Fluorophenyl)carbamoyl)cyclopropane-1-carboxylic acid (11)

To a solution of cyclopropane-1,1-dicarboxylic acid (1 eq) in THF, triethylamine (1~1.5 eq) was added. The solution was stirred for 15 min at 0°C before SOCl₂ (1~1.5 eq) was added via syringe. After another 15 min of stirring, 4-fluoroaniline (1~1.5 eq) in THF was added at 0°C, and the solution was stirred at rt. for 8-20 h. The reaction mixture was quenched with NaOH_{(aq)} and diluted with EA. The phases were separated, and the organic phase was washed with NaOH_{(aq)}. The combined basic extracts were then acidified to a pH of 2 with HCl, and the title compound was achieved by suction filtration as a white solid (yield 13%).

### 4-((6,7-Dimethoxyquinolin-4-yl)oxy)-3-fluoroaniline (12)

A solution of 4-chloro-6,7-dimethoxyquinoline (1 eq), 4-amino-2-fluorophenol (1.5∼4 eq), and NaH (1.5∼4 eq) in DMF was stirred at 70~120 °C for 8-20 h. The mixture was diluted with EA, washed with water, brine, and dried over Na₂SO₄. The mixture was filtered and concentrated to dryness. The crude product was purified by column chromatography to give compound **12** (yield 41%) as a beige solid.

### N (4-((6,7-dimethoxyquinolin-4-yl)oxy)-3-fluorophenyl)-N (4-fluorophenyl)cyclopropane-1,1-dicarboxamide (Cbo-20)

To a solution of compound **11** (1 eq), compound **12** (0.9~1.5 eq) and HATU (0.9∼1.5 eq) in DMF, DIPEA (2~3 eq) was added. The mixture was stirred at rt. for 8-20 h. The mixture was diluted with EA, washed with water, brine, and dried over Na2SO4. The mixture was filtered and concentrated to dryness. The crude product was purified by column chromatography to give compound **Cbo-20** (yield 51%) as a white solid.

¹H NMR (600 MHz, DMSO-d6) *δ*10.42 (s, 1H), 10.01 (s, 1H), 8.53 (d, *J=* 5.2 Hz, 1H), 7.92 (d, *J=* 13.0 Hz, 1H), 7.65 (dd, *J=* 8.3, 5.1 Hz, 2H), 7.56 (s, 1H), 7.54 (d, *J* = 8.9 Hz, 1H), 7.44 (t,*J* = 8.9 Hz, 2H), 7.15 *(t, J=* 8.8 Hz, 2H), 6.50 *(d, J=* 4.5 Hz, 1H), 3.96 (s, 6H), 1.48 *(d, J=* 6.4 Hz, 4H). LCMS (ESI) *m*/*z* 520.73 [M+H]+. HPLC purity: 99.65%.

### Example 6 Cbo-22

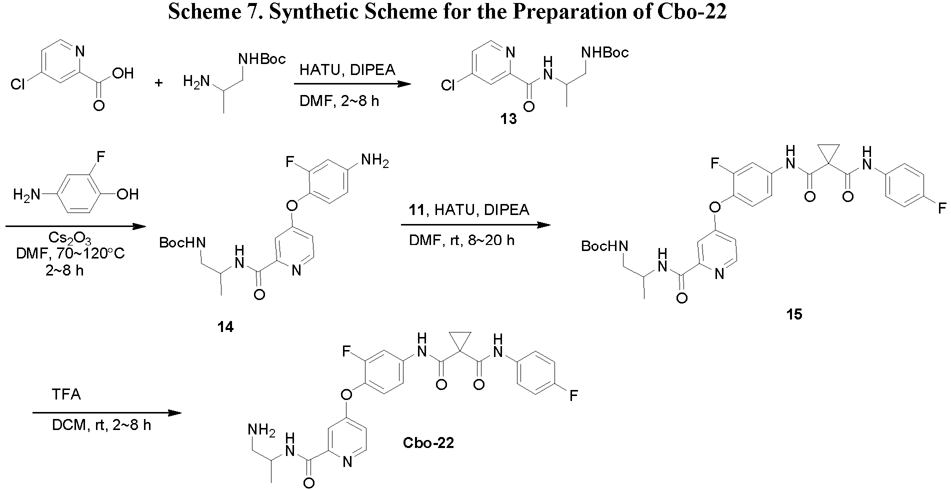

### tert-Butyl (2-(4-chloropicolinamido)propyl)carbamate (13)

To a solution of 4-chloropicolinic acid (1~1.5 eq), tert-butyl (2-aminopropyl)carbamate (1 eq) and HATU (1∼1.5 eq) in DMF, DIPEA (2~5 eq) was added. The mixture was stirred at rt. for 2-8 h. The mixture was diluted with EA, washed with water, brine, and dried over Na₂SO₄. The mixture was filtered and concentrated to dryness. The crude product was purified by column chromatography to give compound **13** (yield 75%) as a white solid.

### tert-Butyl (2-(4-(4-amino-2-fluorophenoxy)picolinamido)propyl)carbamate (14)

A solution of tert-butyl (2-(4-chloropicolinamido)propyl)carbamate (1 eq), 4-amino-2-fluorophenol (1∼2 eq), and Cs₂CO₃ (2~3 eq) in DMF was stirred at 70~120 °C for 2-8 h. The mixture was diluted with EA, washed with water, brine, and dried over Na₂SO₄. The mixture was filtered and concentrated to dryness. The crude product was purified by column chromatography to give compound **14** (yield 65%) as a beige solid.

### tert-Butyl (2-(4-(2-fluoro-4-(1-((4-fluorophenyl)carbamoyl)cyclopropane-1-carboxamido)phenoxy)picolinamido)propyl)carbamate (15)

To a solution of compound **11** (1~1.5 eq), compound **3** (1 eq) and HATU (1~1.5 eq) in DMF, DIPEA (1.5∼5 eq) was added. The mixture was stirred at rt. for 8-20 h. The mixture was diluted with EA, washed with water, brine, and dried over Na₂SO₄. The mixture was filtered and concentrated to dryness. The crude product was purified by column chromatography to give compound **15** (yield 76%) as a white solid.

### N-(4-((2-((1-aminopropan-2-yl)carbamoyl)pyridin-4-yl)oxy)-3-fluorophenyl)-N-(4-fluorophenyl)cyclopropane-1,1-dicarboxamide (Cbo-22)

To a solution of compound 4 (1 eq) in DCM, TFA (5∼10 eq) was added. The mixture was stirred at rt. for 2~8 h. The mixture was diluted with EA and neutralized with sat. NaHCO3 (aq) to pH = 8. The mixture was washed with water, brine, and dried over Na2SO4. The mixture was filtered and concentrated to dryness. The crude product was purified by column chromatography to give **Cbo-22** (yield 54%).

¹H NMR (600 MHz, DMSO-*d*6) *δ* 10.38 (brs, 1H), 10.03 (brs, 1H), 8.53 (t, *J =* 6.5 Hz, 2H), 7.90 (d, *J =* 13.1 Hz, 1H), 7.64 (dd, *J* = 8.7, 5.1 Hz, 2H), 7.50 (d, *J =* 8.7 Hz, 1H), 7.38 (t, *J =* 9.0 Hz, 1H), 7.35 (d, *J =* 2.3 Hz, 1H), 7.23 (dd, *J =* 5.5, 2.5 Hz, 1H), 7.15 (t, *J =* 8.8 Hz, 2H), 3.90-3.83 (m, 1H), 2.67 (dd, *J* = 12.6, 5.9 Hz, 1H), 2.60 (dd, *J* = 12.6, 5.5 Hz, 1H), 1.60(brs, 2H) 1.46 (s, 4H), 1.11 *(d, J=* 6.6 Hz, 3H). LCMS (ESI) *m*/*z* 510.69 [M+H]+. HPLC purity: 99.82%.

### Example 7 Cbo-23

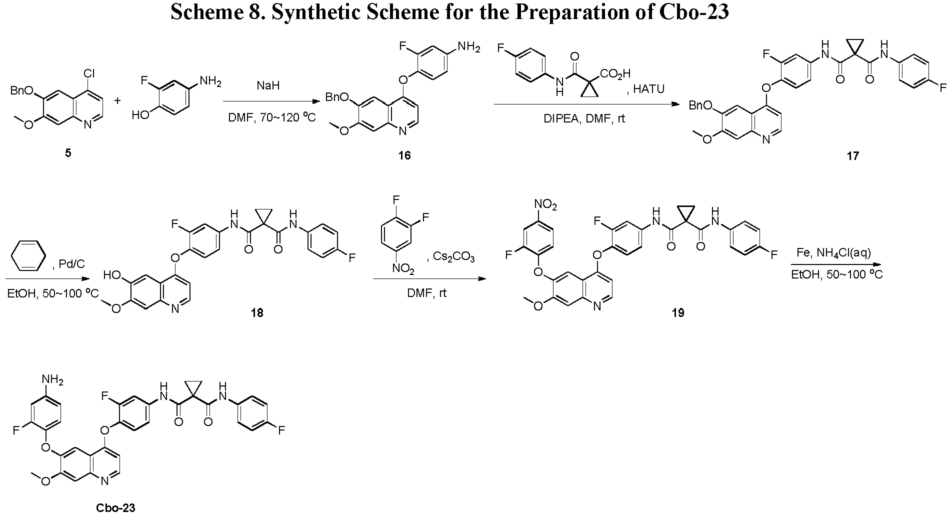

### 4-((6-(benzyloxy)-7-methoxyquinolin-4-yl)oxy)-3-fluoroaniline (16)

To a solution of 6-(benzyloxy)-4-chloro-7-methoxyquinoline (1 eq), 4-amino-2-fluorophenol (2~5 eq) in DMF, NaH (2~5 eq) was added, and the mixture was stirred at 70~120 °C for 16-30 h. The mixture was diluted with EA, washed with water, brine, and dried over Na₂SO₄. The mixture was filtered and concentrated to dryness. The crude product was purified by column chromatography to give compound 16 (yield 27%) as a beige solid.

### N-(4-((6-(benzyloxy)-7-methoxyquinolin-4-yl)oxy)-3-fluorophenyl)-N-(4-fluorophenyl)cyclopropane-1,1-dicarboxamide (17)

To a solution of compound **16** (1 eq), 1-((4-fluorophenyl)carbamoyl)- cyclopropane-1-carboxylic acid (1∼1.5 eq) and HATU (1~1.5 eq) in DMF, DIPEA (2~3 eq) was added. The mixture was stirred at rt. for 10 h. The mixture was diluted with EA, washed with water, brine, and dried over Na₂SO₄. The mixture was filtered and concentrated to dryness. The crude product was purified by column chromatography to give compound **17** (yield 82%) as a white solid.

### N-(3-fluoro-4-((6-hydroxy-7-methoxyquinolin-4-yl)oxy)phenyl)-N-(4-fluorophenyl)cyclopropane-1,1-dicarboxamide (18)

To a solution of compound **17** (1 eq), 1,4-cyclohexadiene (5∼10 eq) in EtOH (30 mL), Pd/C (10-40% w/w) was added, and the mixture was stirred at 50-100 °C for 1-6 h. The mixture was filtered through a Celite pad and the filtrate was concentrated to dryness. The crude product was purified by column chromatography to give **18** (yield 99%) as a white solid.

### N-(3-fluoro-4-((6-(2-fluoro-4-nitrophenoxy)-7-methoxyquinolin-4-yl)oxy)phenyl)-N-(4-fluorophenyl)cyclopropane-1,1-dicarboxamide (19)

To a solution of compound **18** (1 eq) and Cs₂CO₃ (2~3 eq) in DMF, 1,2-difluoro-4-nitrobenzene (1∼1.5 eq) was added. The mixture was stirred at rt. for 1-4 h. The mixture was diluted with EA, washed with water, brine, and dried over Na₂SO₄. The mixture was filtered and concentrated to dryness. The crude product was purified by column chromatography to give compound **19** (yield 75%) as a pink solid.

### N-(4-((6-(4-amino-2-fluorophenoxy)-7-methoxyquinolin-4-yl)oxy)-3-fluoropheny-l)-N-(4-fluorophenyl)cyclopropane-1,1-dicarboxamide (Cbo-23)

To a solution of compound **19** (1 eq), Fe (3∼6 eq) in EtOH, sat. NH₄Cl_{(aq.)} was added. The mixture was stirred at 50-100 °C for 1-6 h. The mixture was filtered through a Celite pad. The filtrate was diluted with EA, washed with water, brine, and dried over Na₂SO₄. The mixture was filtered and concentrated to dryness. The crude product was purified by column chromatography to give **Cbo-23** (yield 83%) as a pink solid.

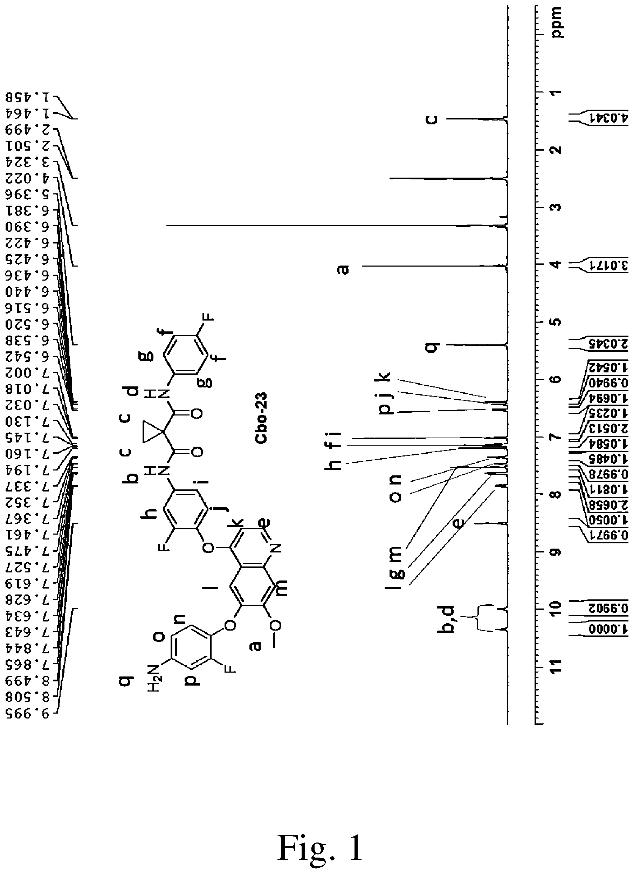

¹H NMR (600 MHz, DMSO-*d*6) *δ* 10.35 (s, 1H), 10.00 (s, 1H), 8.50 (d, *J =* 5.2 Hz, 1H), 7.85 (d, *J =* 13.0 Hz, 1H), 7.63 (dd, *J =* 8.8, 5.1 Hz, 2H), 7.53 (s, 1H), 7.47 (d, *J* = 8.6 Hz, 1H), 7.35 (t, *J =* 9.0 Hz, 1H), 7.19 (s, 1H), 7.15 (t, *J =* 8.9 Hz, 2H), 7.02 (t, *J =* 9.0 Hz, 1H), 6.53 (dd, *J =* 13.2, 2.3 Hz, 1H), 6.43 (dd, *J* = 8.7, 2.1 Hz, 1H), 6.39 (d, *J* = 5.2 Hz, 1H), 5.40 (s, 2H), 4.02 (s, 3H), 1.49-1.44 (m, 4H). LCMS (ESI) *m*/*z* 615.74 [M+H]⁺. HPLC purity: 98.41 %. Detailed NMR data for Cbo-23 can be found in **Figure 1** and **Table 1.**

### Example 8 Cbo-24

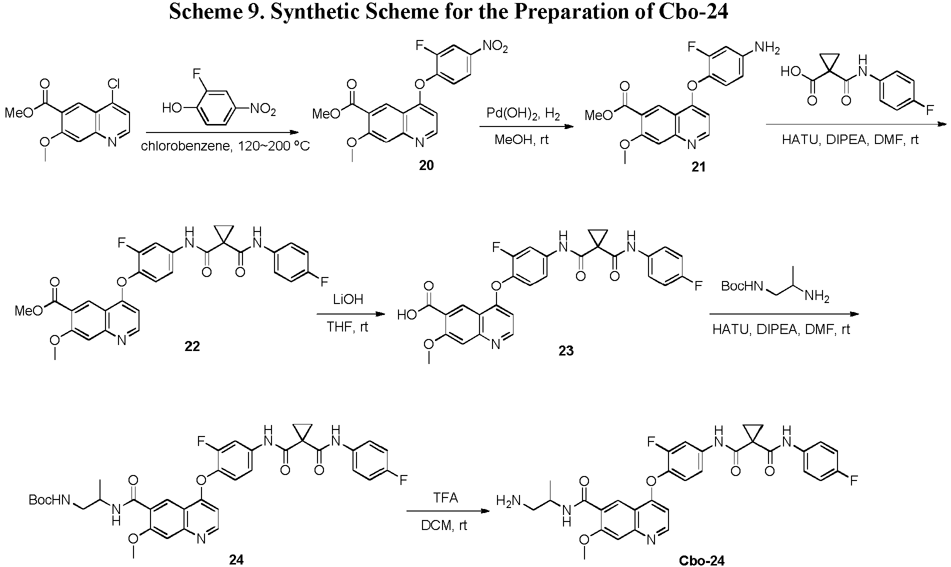

### Methyl 4-(2-fluoro-4-nitrophenoxy)-7-methoxyquinoline-6-carboxylate (20)

A solution of methyl 4-chloro-7-methoxyquinoline-6-carboxylate (1 eq), 2-fluoro-4-nitrophenol (1.5∼3 eq) in chlorobenzene was stirred at 120~200 °C for 8~16 h. The mixture was diluted with EA, washed with 10% NaOH_{(aq)}, water, brine, and dried over Na₂SO₄. The mixture was filtered and concentrated to dryness. The crude product was purified by column chromatography to give compound 20 (yield 95%) as a yellow solid.

### Methyl 4-(4-amino-2-fluorophenoxy)-7-methoxyquinoline-6-carboxylate (21)

To a solution of compound 20 (1 eq) in MeOH, Pd(OH)₂ (40% w/w) was added. The mixture was stirred at rt. under H₂ for 8~20 h. The mixture was filtered through a Celite pad and the filtrate was concentrated to dryness to give compound **21** (yield 68%) as a yellow solid.

### Methyl 4-(2-fluoro-4-(1-((4-fluorophenyl)carbamoyl)cyclopropane-1-carboxamido)phenoxy)-7-methoxyquinoline-6-carboxylate (22)

To a solution of compound **21** (1 eq), 1-((4-fluorophenyl)carbamoyl)-cyclopropane-1-carboxylic acid (1∼1.5 eq) and HATU (1~1.5 eq) in DMF, DIPEA (2~3 eq) was added. The mixture was stirred at rt. for 8-20 h. The mixture was diluted with EA, washed with water, brine, and dried over Na₂SO₄. The mixture was filtered and concentrated to dryness. The crude product was purified by column chromatography to give compound **22** (yield 99%) as a white solid.

### 4-(2-Fluoro-4-(1-((4-fluorophenyl)carbamoyl)cyclopropane-1-carboxamido)phenoxy)-7-methoxyquinoline-6-carboxylic acid (23)

To a solution of **22** (1 eq) in THF, LiOH (2~5 eq) was added, and the mixture was stirred at rt. for 1~4 h. The mixture was concentrated to remove THF. The aqueous solution was acidified to a pH of 3 with 1N HCl_{(aq.)}. The resulting solid was filtered to give compound **23** (yield 84%) as a white solid.

### tert-Butyl (2-(4-(2-fluoro-4-(1-((4-fluorophenyl)carbamoyl)cyclopropane-1-carboxamido)phenoxy)-7-methoxyquinoline-6-carboxamido)propyl)carbamate (24)

To a solution of compound **23** (1 eq), tert-butyl (2-aminopropyl)carbamate (1~1.5 eq) and HATU (1∼1.5 eq) in DMF, DIPEA (2~3 eq) was added. The mixture was stirred at rt. for 1-4 h. The mixture was diluted with EA, washed with water, brine, and dried over Na₂SO₄. The mixture was filtered and concentrated to dryness. The crude product was purified by column chromatography to give compound **24** (yield 98%) as a yellow solid.

### O-phenyl-N-(4-fluorophenyl)cyclopropane-1,1-dicarboxamide (Cbo-24)

To a solution of compound **24** (1 eq) in DCM, TFA (5∼10 eq) was added. The mixture was stirred at rt. for 8-20 h. The mixture was diluted with EA and neutralized with sat. NaHCO_{3(aq)} to pH = 8. The resulting solid was filtered to give **Cbo-24** (yield 68%) as a yellow solid.

¹H NMR (600 MHz, DMSO-d6) *δ* 10.42 (s, 1H), 9.99 (s, 1H), 8.67 (d, *J =* 5.2 Hz, 1H), 8.60 (s, 1H), 8.32 (d, *J =* 8.1 Hz, 1H), 7.92 (d, *J =* 12.9 Hz, 1H), 7.64 (dd, *J =* 8.9, 5.1 Hz, 2H), 7.55-7.52 (m, 2H), 7.44 (t, *J =* 9.0 Hz, 1H), 7.16 (t, *J =* 8.9 Hz, 2H), 6.88 (brs, 2H), 6.49 (d, *J =* 5.2 Hz, 1H), 4.22 (dt, *J =* 13.9, 6.8 Hz, 1H), 4.02 (s, 3H), 2.90 (d, *J =* 6.5 Hz, 2H), 1.50-1.44 (m, 4H), 1.22 (d, *J =* 6.7 Hz, 3H). LCMS (ESI) *m*/*z* 590.33 [M+H]⁺. HPLC purity: 99.67%.

### Example 9 Cbo-25

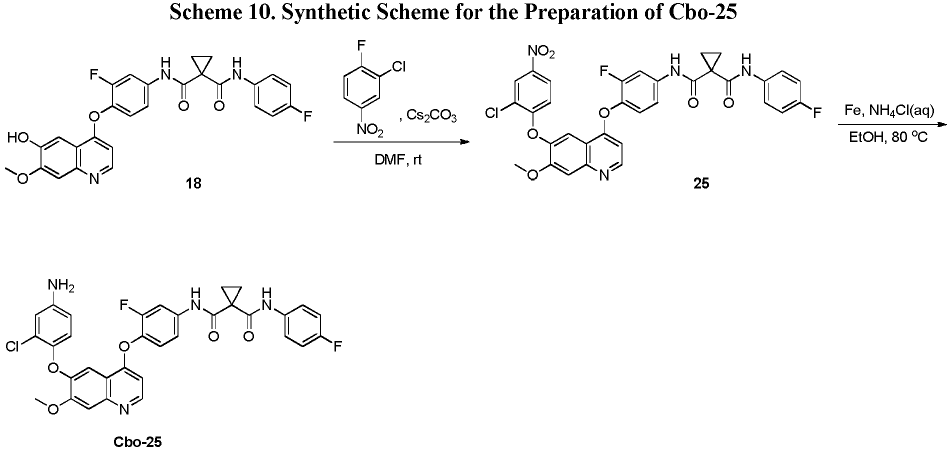

### N-(4-((6-(2-chloro-4-nitrophenoxy)-7-methoxyquinolin-4-yl)oxy)-3-fluorophenyl)-N-(4-fluorophenyl)cyclopropane-1,1-dicarboxamide (25)

To a solution of compound **18** (1eq) and Cs₂CO₃ (2~5eq) in DMF, 2-chloro-1-fluoro-4-nitrobenzene (1~2eq) was added. The mixture was stirred at rt for 1~4 h. Water and EA were added to the mixture. The resulting solid was filtered to give compound **25** (yield 82%) as a white solid.

### N-(4-((6-(4-amino-2-chlorophenoxy)-7-methoxyquinolin-4-yl)oxy)-3-fluorophenyl)-N-(4-fluorophenyl)cyclopropane-1,1-dicarboxamide (Cbo-25)

To a solution of compound **25** (1eq) and Fe (2~5eq) in EtOH, sat. NH₄Cl_{(aq.)} was added. The mixture was stirred at 70-100 °C for 1-4 h. The mixture was filtered through a Celite pad. The filtrate was diluted with EA, washed with water and then with brine, and removed H₂O with Na₂SO₄. The mixture was filtered and concentrated to dryness. The crude product was purified by column chromatography to give **Cbo-25** (yield 88%) as a beige solid.

¹H NMR (600 MHz, DMSO-d6) *δ* 10.35 (s, 1H), 9.99 (s, 1H), 8.50 (d, *J=* 5.2 Hz, 1H), 7.85 (dd, *J* = 13.4, 1.8 Hz, 1H), 7.65-7.61 (m, 2H), 7.53 (s, 1H), 7.47 (d, *J* = 8.9 Hz, 1H), 7.35 (t, *J* = 9.0 Hz, 1H), 7.17-7.12 (m, 2H), 7.10 (s, 1H), 7.01 (d, *J* = 8.6 Hz, 1H), 6.76 (d, *J* = 2.6 Hz, 1H), 6.59 (dd, *J* = 8.6, 2.6 Hz, 1H), 6.38 (d, *J=* 5.2 Hz, 1H), 5.38 (s, 2H), 4.03 (s, 3H), 1.48-1.43 (m, 4H). LCMS (ESI) *m*/*z* 631.62 [M+H]⁺. HPLC purity: 99.26%.

### Efficacy Test Examples

### Example 10 In Vitro Cytotoxicity Assay

Six different cell lines were used, including human breast cancer cell lines MDA-MB-231 (6×10³) and MDA-MB-453 (2.4×10⁴), human colorectal cancer cell line SW48 (2.4×10⁴), human breast epithelial cell line M10 (6×10³), human lung cancer cell line A549 (1.4×10³), and murine colorectal carcinoma tumors CT26 (6×10³), which were seeded in a 96-well plate. Cell lines were obtained from Bioresource Collection and Research Center, BCRC, Taiwan. All cell lines were treated with the compounds including the compound series and Cabozantinib and Zanzalintinib (MCE^{®}, Cat#HY-13016 and Cat#HY-138696) as positive control, with doses ranging from 25 , 12.5 , 6.25 , 3.125 and 1.56 µM, and then incubated at 37°C under 5% CO₂ for 72 h. After 72 h, MTT assay (Cayman^{™}) was used to determine the cellular viability. MDA-MB-231, MDA-MB-453 and SW48 cell lines were maintained in DMEM/F12 supplemented with 10% FBS, 0.2% antibiotic (MycoZap^{™}, Pluse-CL). M10 cell line was maintained in MEM Alpha (Gibco^{™}) supplemented with 10% FBS, 0.2% antibiotic (MycoZap^{™}, Pluse-CL). CT26 cell line was maintained in RPMI 1640 (CORNING^{™}) supplemented with 10% FBS, 0.2% antibiotic (MycoZap^{™}, Pluse-CL). A549 cell line was maintained in DMEM (CORNING^{™}) supplemented with 10% FBS, 0.2% antibiotic (MycoZap^{™}, Pluse-CL).

### Example 11 Screening methodology for 60 human cancer cell lines

The human tumor cell lines of the cancer screening panel are grown in RPMI 1640 medium containing 5% fetal bovine serum and 2 mM L-glutamine. For a typical screening experiment, cells are inoculated into 96 well microtiter plates in 100 µL at plating densities ranging from 5,000 to 40,000 cells/well depending on the doubling time of individual cell lines. After cell inoculation, the microtiter plates are incubated at 37° C, 5 % CO₂, 95 % air and 100 % relative humidity for 24 h prior to addition of experimental drugs. After 24 h, two plates of each cell line are fixed in situ with TCA, to represent a measurement of the cell population for each cell line at the time of drug addition (Tz). Experimental drugs are solubilized in dimethyl sulfoxide at 400-fold the desired final maximum test concentration and stored frozen prior to use. At the time of drug addition, an aliquot of frozen concentrate is thawed and diluted to twice the desired final maximum test concentration with complete medium containing 50 µg/ml gentamicin. An additional four, 10-fold or ½ log serial dilutions are made to provide a total of five drug concentrations plus control. Aliquots of 100 µl of these different drug dilutions are added to the appropriate microtiter wells already containing 100 µl of medium, resulting in the required final drug concentrations. Following drug addition, the plates are incubated for an additional 48 h at 37°C, 5 % CO₂, 95 % air, and 100 % relative humidity. For adherent cells, the assay is terminated by the addition of cold TCA. Cells are fixed in situ by the gentle addition of 50 µl of cold 50 % (w/v) TCA (final concentration, 10 % TCA) and incubated for 60 minutes at 4°C. The supernatant is discarded, and the plates are washed five times with tap water and air dried. Sulforhodamine B (SRB) solution (100 µl) at 0.4 % (w/v) in 1 % acetic acid is added to each well, and plates are incubated for 10 minutes at room temperature. After staining, unbound dye is removed by washing five times with 1 % acetic acid and the plates are air dried. Bound stain is subsequently solubilized with 10 mM trizma base, and the absorbance is read on an automated plate reader at a wavelength of 515 nm. For suspension cells, the methodology is the same except that the assay is terminated by fixing settled cells at the bottom of the wells by gently adding 50 µl of 80 % TCA (final concentration, 16 % TCA).

### Example 12 Tyrosine kinase inhibition assay

Tyrosine kinase inhibition assays were performed at ThermoFisher Life Technologies (Madison, WI, USA) using standard protocols (SelectScreen Biochemical Profiling Service, Thermo Fisher Scientific). ATP concentrations used were at the apparent Km. Test compounds were screened in 1% dimethylsulffoxide (DMSO) (final concentration) in the well. For 10-point titrations, 3-fold serial dilutions were conducted from the starting concentration of 10 µM. All Peptide/Kinase Mixtures were diluted to a 2X working concentration in the appropriate Kinase Buffer. All ATP Solutions were diluted to a 4X working concentration in Kinase Buffer (50 mM HEPES pH 7.5, 0.01% BRIJ-35, 10 mM MgCl₂, 1 mM EGTA). The apparent ATP Km was previously determined using a Z'-LYTE assay. The Development Reagent was diluted in Development Buffer.

### Example 13 Cell lines and cell culture for animal study

LLC1 and CT26 were obtained from Bioresource Collection and Research Center (BCRC, Taiwan). LLC1 was grown at 37 °C under humidified air supplemented with CO₂ in DMEM (#10-013-CM, Corning^{®}) containing 10% heat inactivated fetal calf serum, and 1X concentration of MycoZap Antibiotics. CT26 was grown at 37 °C under humidified air supplemented with CO₂ in RPMI1640 (cat. #10-040-CM, Corning^{®}) containing 10% heat inactivated fetal calf serum, and 1X concentration of MycoZap Antibiotics. After the cancer cell culture was completed, it was implanted (1×10⁶ cells/mouse) into the back of the mouse for growth.

### Example 14 Experimental tumor model and therapies

Animal study was approved and overseen by the Taipei Medical University Institutional Animal Care and Use Committee (TMU IACUC, NO: LAC-2022-0197 and LAC-2022-0306). Six- to eight-week-old male Balb/c mice (National Laboratory Animal Center, Taiwan) were used for all animal experiments. Tumors were established by s.c. injection of 1×10⁶ CT26 cells with Matrigel (354248, Corning^{®}) into the left flank of mice, and growth was determined by measuring two perpendicular diameters. Tumors were allowed to grow for 10-12 days (tumor size about 250-300 mm³) before randomization and treatment. The tumor volume of the mice was measured once every three or four days (twice/week). Animals were euthanized when tumors reached more than 3000 mm³ in diameter. Cabozantinib (HY-13016, MedChemExpress USA), Regorafenib (HY-10331, MedChemExpress USA), Chidamide-K30, GNTbm-38-K30, GNTbm-38-HCL, Cbo-02, Cbo-20, Cbo-22, Cbo-23 and Cbo-24 were administered orally once daily for 12 or 16 days. Cabozantinib, Regorafenib, Cbo-02, Cbo-20, Cbo-22, Cbo-23 and Cbo-24 were dissolved in DMSO and diluted in water before administration (final concentration of DMSO, 5% v/v). Cbo-23-K30, Chidamide-K30 and GNTbm-38-K30 were directly dissolved in water before administration. Relapse/recurrence was defined as when tumor growth reappeared and tumor size reached at least 5 fold in mice with CR or PR response after secondary tumor assessment. In this study, we defined Complete Response (CR, <0.5 time tumor growth in the tumor bearing mice at tumor assessment after the end of treatment); Partial Response (PR, tumor size ≥0.5 time tumor growth, but < 1 time tumor growth in the tumor bearing mice at tumor assessment after the end of treatment); Stable Disease (SD, ≥ 1 time tumor growth, but < 5 times tumor growth in the tumor bearing mice at tumor assessment after the end of treatment); and Progressive Disease (PD, ≥ 5 times tumor growth in the tumor bearing mice at tumor assessment after the end of treatment) for the evaluation of treatment efficacy.

### Example 15 Tumor rechallenge tests

Mice evaluated as CR/PR can enter the rechallenge study. All mice with PR/CR response went through rechallenges with CT26 cells on the contralateral side. The first rechallenge was performed after second assessment with a tumor dose of 1×10⁶ cells per mouse. After 7 days of growth, CT26 and LLC1 tumor sizes were used as the baseline. Tumor growth was monitored for a duration of 11 days, and on the 11th day, a tumor size assessment was conducted relative to the baseline. If both of the following criteria are met, the response will be considered as relapse: first, the tumor size was over 1.5 folds when compared to that of baseline; second, the tumor volume was over 150 mm³.

### Results

Tables 1 to 8 mentioned herein are provided below:

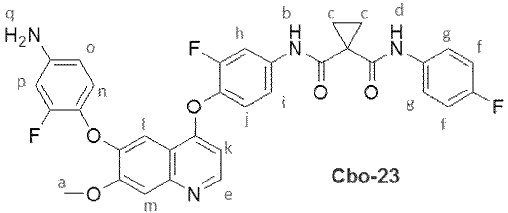

**Table 1. ¹H-NMR Spectroscopic Data (400 MHz, d-DMSO) for Compound Cbo-23.**

| **position** | | **δ_{H} (*J* in Hz)** |
|---|---|---|
| b | NH | 10.35 (s, 1H) |
| d | NH | 10.00 (s, 1H) |
| e | CH | 8.50 (d, *J* = 5.2 Hz, 1H) |
| l | CH | 7.85 (d, *J* = 13.0 Hz, 1H) |
| g | CH | 7.63 (dd, *J =* 8.8, 5.1 Hz, 2H) |
| m | CH | 7.53 (s, 1H) |
| o | CH | 7.47 (d, *J* = 8.6 Hz, 1H) |
| n | CH | 7.35 (t, *J =* 9.0 Hz, 1H) |
| h | CH | 7.19 (s, 1H) |
| f | CH | 7.15 (t, *J =* 8.9 Hz, 2H) |
| i | CH | 7.02 (t, *J* = 9.0 Hz, 1H) |
| p | CH | 6.53 (dd, *J* = 13.2, 2.3 Hz, 1H) |
| j | CH | 6.43 (dd, *J* = 8.7, 2.1 Hz, 1H) |
| k | CH | 6.39 (d, *J* = 5.2 Hz, 1H) |
| q | NH2 | 5.40 (s, 2H) |
| a | CH3 | 4.02 (s, 3H) |
| c | CH2 | 1.49-1.44 (m, 4H) |

**Table 2. Growth inhibition of various cancer cell lines treated with various compounds for 72 hours.**

| **Compounds** | **MDA-MB-453 cells IC₅₀ (*µ*M)** | **MDA-MB-231 cells IC₅₀ (*µ*M)** | **SW48 cells IC₅₀ (*µ*M)** |
|---|---|---|---|
| Cabozantinib (as positive control) | 20.48 ± 4.48 | 8.72 ± 1.08 | 4.33 ± 0.33 |
| Zanzalintinib (as positive control) | 22.55 ± 1.05 | 6.17 ± 0.15 | 6.86 ± 0.26 |
| Cbo-02 | 5.31 ± 0.57 | 5.64 ± 0.36 | 0.82 ± 0.011 |
| Cbo-03 | 17.9 ± 1.34 | 12.7 ± 1.37 | >25 |
| Cbo-05 | 8.92 ± 1.49 | 7.84 ± 1.34 | 1.86 ± 0.059 |
| Cbo-06 | >25 | >25 | 8.76 ± 0.52 |
| Cbo-07 | >25 | 5.07 ± 1.04 | 0.74 ± 0.13 |
| Cbo-08 | >25 | >25 | 3.51 ± 0.48 |
| Cbo-09 | 9.28 ± 0.66 | 12.61 ± 0.98 | 1.02 ± 0.008 |
| Cbo-10 | 4.67 ± 0.28 | 2.45 ± 0.14 | 0.79 ± 0.001 |
| Cbo-14 | 2.68 ± 0.75 | 14.44 ± 5.3 | 0.79 ± 0.001 |
| Cbo-20 | 14.1 ± 2.06 | 4.81 ± 1.16 | 2.51 ± 0.18 |
| Cbo-22 | >25 | 16.13 ± 0.19 | 21.9 ± 0.96 |
| Cbo-23 | 1.71 ± 0.14 | 1.95 ± 1.95 | 0.79 ± 0.005 |
| Cbo-24 | 17.02 ± 0.96 | 15.7 ± 0.33 | 8.75 ± 0.55 |

| **Compounds** | **A549 cells IC₅₀ (*µ*M)** | **M10 cells IC₅₀ (*µ*M)** | **CT-26 cells IC₅₀ (*µ*M)** |
|---|---|---|---|
| Cabozantinib (as positive control) | 4.05 ± 0.46 | 9.57 ± 1.62 | 19.63 ±4.3 |
| Zanzalintinib (as positive control) | 7.58 ± 0.18 | 3.28 ± 1.84 | 22.05 ± 0.29 |
| Cbo-02 | 1.51 ± 0.057 | 3.92 ± 0.25 | 8.2 ± 0.6 |
| Cbo-03 | 12.8 ± 6.7 | 16.17 ± 1.44 | - |
| Cbo-05 | 2.51 ± 0.21 | 1.17 ± 0.053 | 8.9 ± 0.61 |
| Cbo-06 | 6.89 ± 1.61 | 5.59 ± 0.46 | - |
| Cbo-07 | 1.49 ± 0.21 | 4.44 ± 1.51 | - |
| Cbo-08 | 2.6 ± 1.82 | 1.83 ± 0.72 | - |
| Cbo-09 | 6.89 ± 1.61 | 0.99 ± 0.044 | - |
| Cbo-10 | 1.18 ± 0.036 | 0.911 ± 0.025 | 5.01 ±0.16 |
| Cbo-14 | 1.23 ± 0.034 | 0.92 ± 0.008 | 4.9 ± 0.11 |
| Cbo-20 | 3.34 ± 0.69 | 5.47 ± 1.73 | 8.89 ± 0.24 |
| Cbo-22 | 11.19 ± 1.18 | 15.14 ± 0.44 | >25 |
| Cbo-23 | 1.69 ± 0.13 | 1.01 ± 0.02 | 5.18 ± 1.73 |
| Cbo-24 | 16.2 ± 0.95 | >25 | >25 |

**Table 3. Growth inhibition of NCI-60 human cancer cell lines treated with Cbo-02.**

| **Cancer** | **Cell Lines** | **GI₅₀ (*µ*M)** |
|---|---|---|
| **Leukemia** | CCRF-CEM | 2.24 |
| | HL-60(TB) | 2.2 |
| | K-562 | 3.05 |
| | MOLT-4 | 5.04 |
| | RPMI-8226 | 4.57 |
| | SR | 3.33 |
| | | |
| **Non-Small Cell Lung Cancer** | A549/ATCC | 3.27 |
| | EKVX | 1.03 |
| | HOP-62 | 1.82 |
| | HOP-92 | 1.26 |
| | NCI-H226 | 1.58 |
| | NCI-H23 | 2.06 |
| | NCI-H322M | 3.94 |
| | NCI-H460 | 2.75 |
| | NCI-H522 | 2.07 |
| | | |
| **Colon Cancer** | COLO 205 | 1.75 |
| | HCC-2998 | 2.5 |
| | HCT-116 | 1.52 |
| | HCT-15 | 2.98 |
| | HT29 | 1.82 |
| | KM12 | 2 |
| | SW-620 | 1.63 |
| | | |
| **Renal Cancer** | 786-0 | 1.98 |
| | A498 | 3.28 |
| | ACHN | 2.14 |
| | CAKI-1 | 1.54 |
| | RXF393 | 2.05 |
| | SN12C | 1.21 |
| | TK-10 | 1.83 |
| | UO-31 | 2.59 |
| | | |
| **Prostate Cancer** | PC-3 | 3.94 |
| | DU-145 | 3.36 |
| | | |
| **CNS Cancer** | SF-268 | 4.17 |
| | SF-295 | 2.31 |
| | SF-539 | 1.69 |
| | SNB-19 | 3.52 |
| | U251 | 2.88 |
| | | |
| **Melanoma** | LOX IMVI | 1.99 |
| | MALME-3M | 1.74 |
| | M14 | 1.91 |
| | MDA-MB-435 | 1.89 |
| | SK-MEL-2 | 6.15 |
| | SK-MEL-28 | 1.61 |
| | SK-MEL-5 | 4.01 |
| | UACC-257 | 2.83 |
| | UACC-62 | 1.84 |
| | | |
| **Breast Cancer** | MCF7 | 3.26 |
| | MDA-MB-231/ATCC | 1.74 |
| | HS 578T | 2.54 |
| | BT-549 | 1.43 |
| | T-47D | 2.12 |
| | MDA-MB-468 | 1.72 |
| | | |
| **Ovarian Cancer** | IGROV1 | 4.48 |
| | OVCAR-3 | 2.4 |
| | OVCAR-4 | 2.38 |
| | OVCAR-5 | 1.91 |
| | OVCAR-8 | 3.03 |
| | NCI/ADR-RES | 4.9 |
| | SK-OV-3 | 2.27 |
| **Average GI₅₀ (µM):** 2.56 | | |

**Table 4. Growth inhibition of NCI-60 human cancer cell lines treated with Cbo-10.**

| **Cancer** | **Cell Lines** | **GI₅₀ (µM)** |
|---|---|---|
| **Leukemia** | CCRF-CEM | 0.436 |
| | HL-60(TB) | 0.521 |
| | K-562 | 1.74 |
| | MOLT-4 | 0.777 |
| | RPMI-8226 | 2.99 |
| | SR | 0.345 |
| | | |
| **Non-Small Cell Lung Cancer** | A549/ATCC | 0.483 |
| | EKVX | 0.488 |
| | HOP-62 | 3.48 |
| | HOP-92 | 0.141 |
| | NCI-H226 | 5.29 |
| | NCI-H23 | 1.04 |
| | NCI-H322M | 0.518 |
| | NCI-H460 | 1.3 |
| | NCI-H522 | 0.45 |
| | | |
| **Colon Cancer** | COLO 205 | 0.931 |
| | HCC-2998 | 6.04 |
| | HCT-116 | 2.63 |
| | HCT-15 | 2.41 |
| | HT29 | 0.413 |
| | KM 12 | 0.13 |
| | SW-620 | 0.661 |
| | | |
| **Renal Cancer** | 786-0 | >100 |
| | A498 | >100 |
| | ACHN | 0.683 |
| | CAKI-1 | 0.276 |
| | RXF 393 | >100 |
| | SN12C | 0.111 |
| | TK-10 | 0.455 |
| | UO-31 | 1.81 |
| | | |
| **Prostate Cancer** | PC-3 | >100 |
| | DU-145 | 1.7 |
| | | |
| **CNS Cancer** | SF-268 | >100 |
| | SF-295 | 0.304 |
| | SF-539 | >100 |
| | SNB-19 | >100 |
| | SNB-75 | >100 |
| | U251 | >100 |
| | | |
| **Melanoma** | LOX IMVI | 1.78 |
| | MALME-3M | 0.313 |
| | M14 | 1.49 |
| | MDA-MB-435 | 0.737 |
| | SK-MEL-2 | >100 |
| | SK-MEL-28 | 1.81 |
| | SK-MEL-5 | 6.97 |
| | UACC-257 | 1.72 |
| | UACC-62 | 0.915 |
| | | |
| **Breast Cancer** | MCF7 | 0.771 |
| | MDA-MB-231/ATCC | 4.43 |
| | HS 578T | 5.32 |
| | BT-549 | 2.41 |
| | T-47D | 1.65 |
| | MDA-MB-468 | 1.73 |
| | | |
| **Ovarian Cancer** | IGROV1 | 0.714 |
| | OVCAR-3 | >100 |
| | OVCAR-4 | >100 |
| | OVCAR-5 | 0.644 |
| | OVCAR-8 | >100 |
| | NCI/ADR-RES | >100 |
| | SK-OV-3 | 0.302 |
| **Average GI₅₀ (µM):** 1.57 | | |

**Table 5. Growth inhibition of NCI-60 human cancer cell lines treated with Cbo-23.**

| **Cancer** | **Cell Lines** | **GI₅₀ (µM)** |
|---|---|---|
| **Leukemia** | CCRF-CEM | 0.553 |
| | HL-60(TB) | 0.596 |
| | K-562 | 0.13 |
| | MOLT-4 | 1.23 |
| | SR | 0.0938 |
| | | |
| **Non-Small Cell Lung Cancer** | A549/ATCC | 0.325 |
| | EKVX | 0.389 |
| | HOP-62 | 1.2 |
| | HOP-92 | <0.01 |
| | NCI-H226 | 0.276 |
| | NCI-H23 | 0.339 |
| | NCI-H322M | 0.444 |
| | NCI-H460 | 0.457 |
| | NCI-H522 | 0.33 |
| | | |
| **Colon Cancer** | COLO 205 | 0.283 |
| | HCC-2998 | 2.28 |
| | HCT-116 | 0.592 |
| | HCT-15 | 0.315 |
| | HT29 | 2.15 |
| | KM12 | <0.01 |
| | SW-620 | 0.449 |
| | | |
| **Renal Cancer** | 786-0 | 0.86 |
| | A498 | 0.459 |
| | ACHN | 0.292 |
| | CAKI-1 | 0.192 |
| | RXF 393 | 0.103 |
| | SN12C | 0.199 |
| | TK-10 | 0.224 |
| | UO-31 | 0.316 |
| | | |
| **Prostate Cancer** | PC-3 | 1.6 |
| | DU-145 | 0.429 |
| | | |
| **CNS Cancer** | SF-268 | 1.35 |
| | SF-295 | 0.0669 |
| | SF-539 | 0.179 |
| | SNB-19 | 3.13 |
| | SNB-75 | 0.178 |
| | U251 | 2.38 |
| | | |
| **Melanoma** | LOX IMVI | 0.306 |
| | MALME-3M | 0.298 |
| | M14 | 0.459 |
| | MDA-MB-435 | 0.363 |
| | SK-MEL-2 | 1.36 |
| | SK-MEL-28 | 0.463 |
| | SK-MEL-5 | 1.38 |
| | UACC-257 | 0.367 |
| | UACC-62 | 0.112 |
| | | |
| **Breast Cancer** | MCF7 | 0.294 |
| | MDA-MB-231/ATCC | 0.981 |
| | HS 578T | 0.148 |
| | BT-549 | 2.27 |
| | T-47D | 0.617 |
| | MDA-MB-468 | 1.12 |
| | | |
| **Ovarian Cancer** | IGROV1 | 0.264 |
| | OVCAR-3 | 0.829 |
| | OVCAR-4 | 2.58 |
| | OVCAR-5 | 0.389 |
| | OVCAR-8 | 2.06 |
| | NCI/ADR-RES | 2.04 |
| | SK-OV-3 | 0.464 |
| **Average GI₅₀ (µM): 0.73** | | |

Minus mean cell lethality percentage.

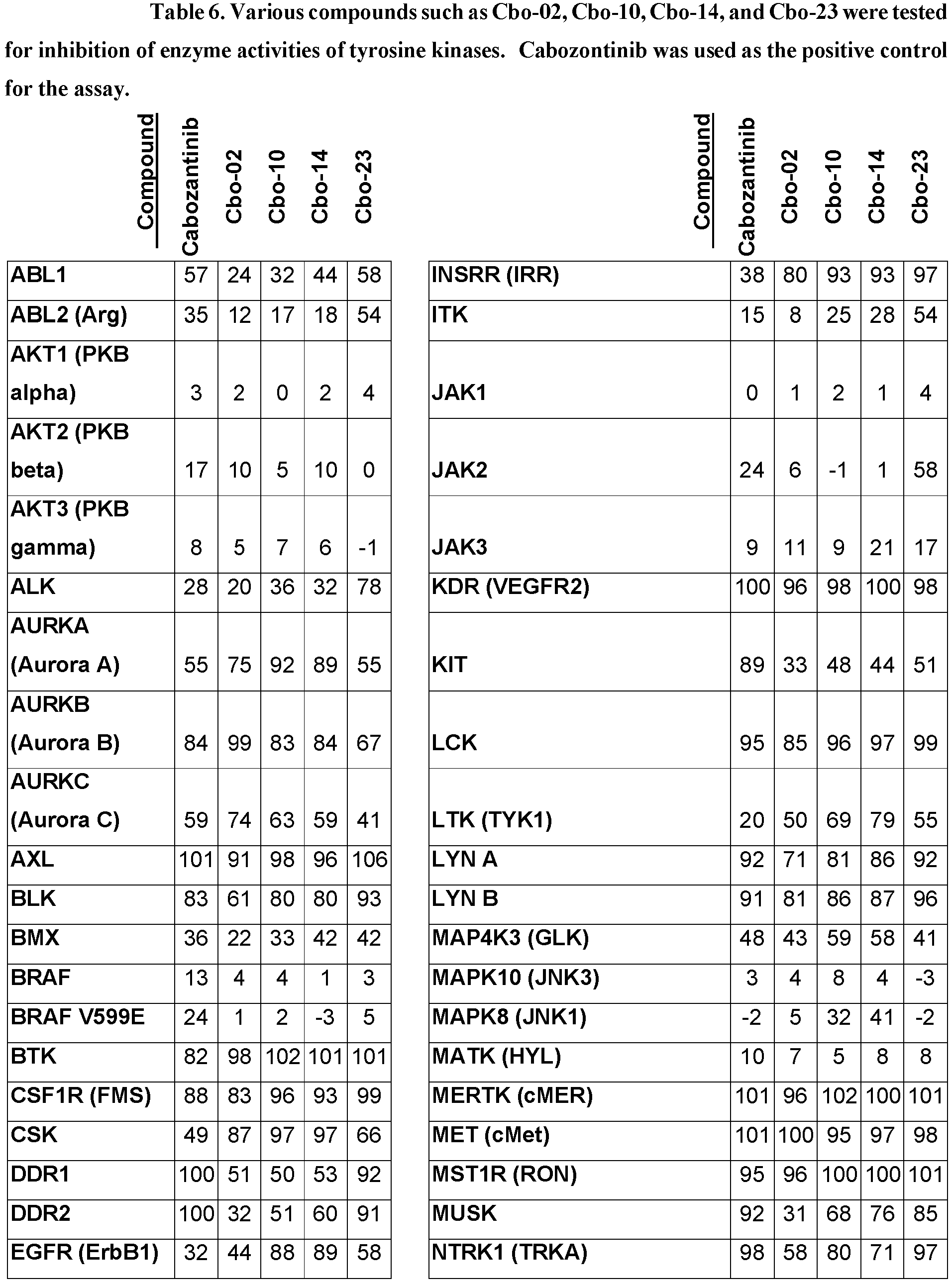

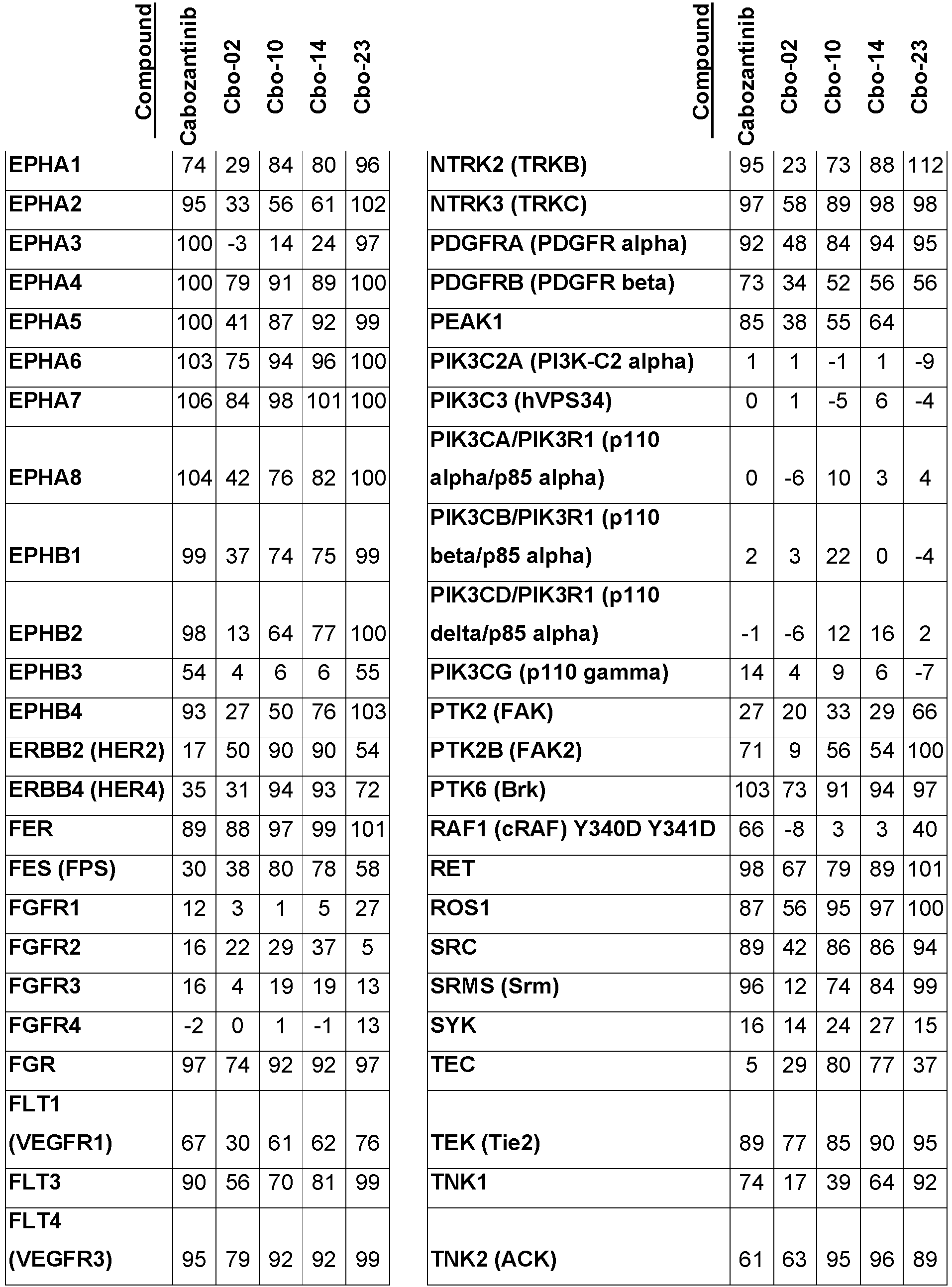

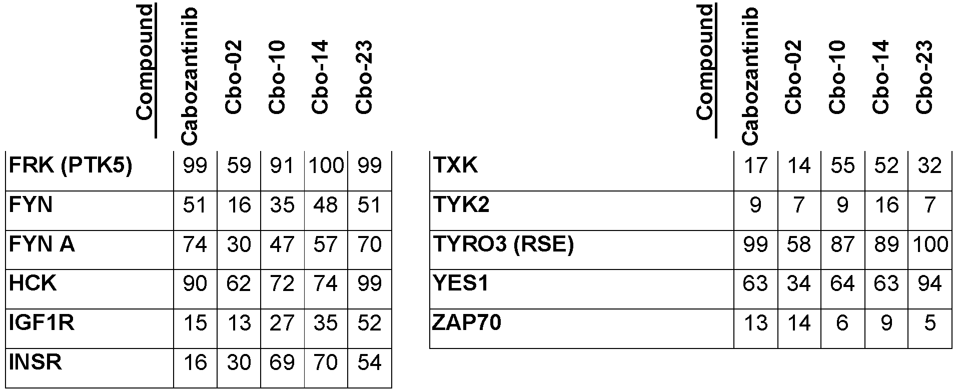

*The inhibition activities of compounds were determined in biochemical assays using single concentration (1.0 µM) measurements. The results were provided as percent inhibition values. A compound displaying a percent inhibition value of greater 80% was defined as a hit. 99 different tyrosine kinases were used.*

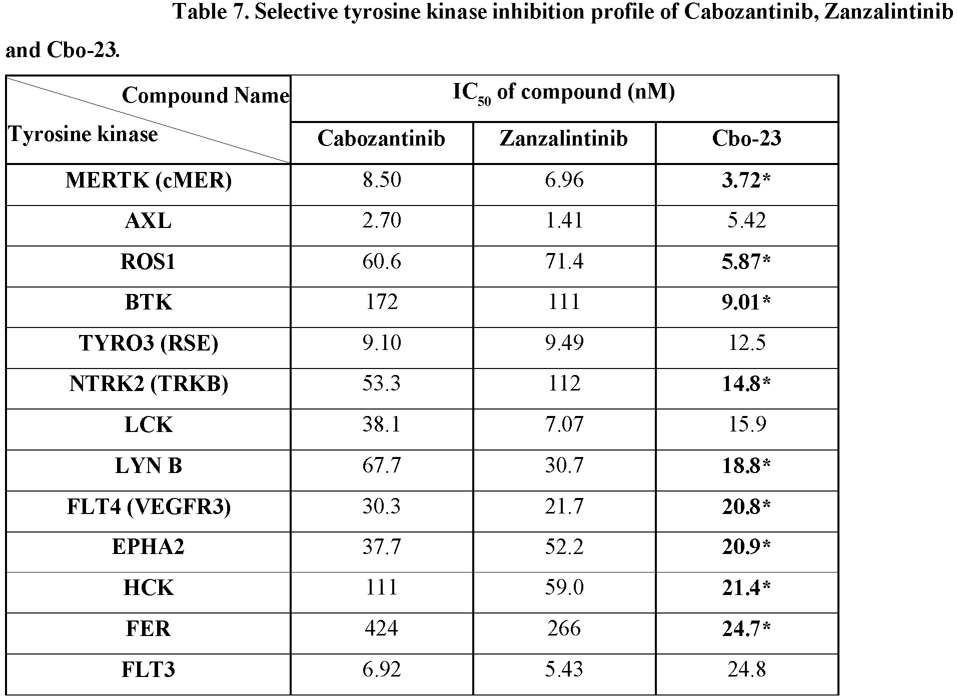

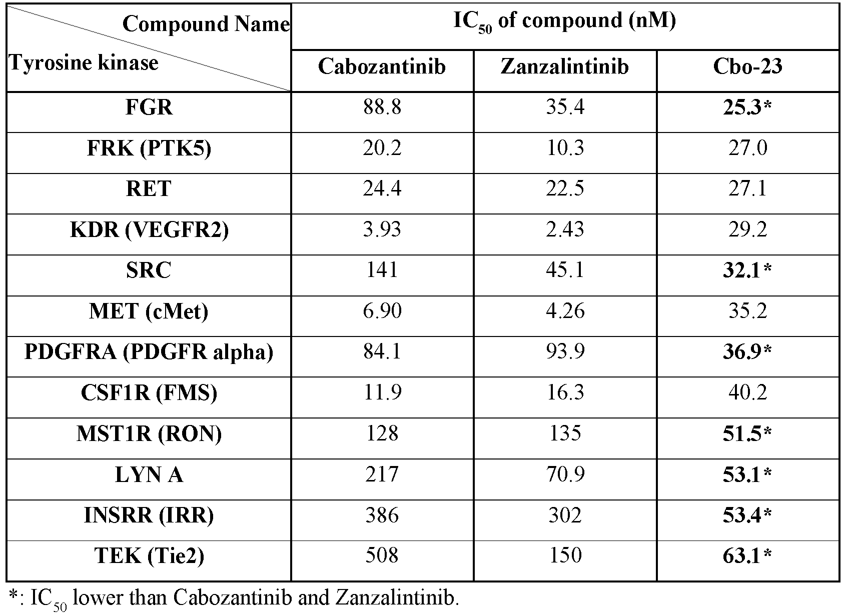

**Table 8. Rechallenge test to evaluate the induction of immune memory after treatment with Cbo-20 combined with GNTbm-38-K30.**

| CT26 rechallenge | | | | | | | |
|---|---|---|---|---|---|---|---|
| Status | Groups | Day 56 | | Day 67 | | | |
| | | mm³ | Fold change | mm³ | Fold change | Score | Immunity Percentage (%) |
| | GNTbm-38-K30 (40 mg/kg) + Cabozantinib (30 mg/kg) | | | | | | 100% (5/5) |
| CR | 152 | 102.59 | 1 | 0 | 0 | - | |
| CR | 153 | 138.34 | 1 | 0 | 0 | - | |
| CR | 154 | 94.60 | 1 | 0 | 0 | - | |
| CR | 155 | 133.67 | 1 | 174.89 | 1.31 | - | |
| CR | 156 | 94.39 | 1 | 0 | 0 | - | |
| | | | | | | | |
| | GNTbm-38-K30 (40 mg/kg)+ Cbo-20 (30 mg/kg) | | | | | | 71% (5/7) |

### CT26 rechallenge

| Status | Groups | Day 56 | | Day 67 | | | |
|---|---|---|---|---|---|---|---|
| | | mm³ | Fold change | mm³ | Fold change | Score | Immunity Percentage (%) |
| CR | 141 | 139.93 | 1 | 0 | 0 | - | |
| CR | 142 | 135.37 | 1 | 0 | 0 | - | |
| CR | 143 | 8020 | 1 | 378.52 | 4.72 | + | |
| CR | 144 | 155.35 | 1 | 0 | 0 | - | |
| PR | 145 | 151.68 | 1 | 403.51 | 2.66 | + | |
| CR | 147 | 150.70 | 1 | 47.84 | 0.32 | - | |
| CR | 148 | 115.57 | 1 | 0 | 0 | - | |

### LLC1 rechallenge

| Status | Groups | Day 56 | | Day 67 | | | |
|---|---|---|---|---|---|---|---|
| | | mm³ | Fold change | mm³ | Fold change | Score | Immunity Percentage (%) |
| | GNTbm-38-K30 (40 mg/kg) + Cabozantinib (30 mg/kg) | | | | | | 20% (1/5) |
| CR | 152 | 235.95 | 1 | 530.18 | 2.25 | + | |
| CR | 153 | 119.19 | 1 | 103.10 | 0.86 | - | |
| CR | 154 | 125.21 | 1 | 948.64 | 7.58 | + | |
| CR | 155 | 147.35 | 1 | 264.89 | 1.80 | + | |
| CR | 156 | 178.24 | 1 | 964.91 | 5.41 | + | |
| | | | | | | | |
| | GNTbm-38-K30 (40 mg/kg)+ Cbo-20 (30 mg/kg) | | | | | | 0% (0/7) |
| CR | 141 | 242.87 | 1 | 1417.21 | 5.84 | + | |
| CR | 142 | 145.45 | 1 | 1984.11 | 13.64 | + | |
| CR | 143 | 187.64 | 1 | 742.53 | 3.96 | + | |
| CR | 144 | 171.96 | 1 | 1969.98 | 11.46 | + | |
| PR | 145 | 258.97 | 1 | 1023.38 | 3.95 | + | |
| CR | 147 | 180.22 | 1 | 1623.33 | 9.01 | + | |
| CR | 148 | 141.51 | 1 | 792.24 | 5.60 | + | |

| | | | | | | | |
|---|---|---|---|---|---|---|---|
| *If both of the following criteria are met, the response will be considered as relapse: first, the tumor size over 1.5 folds when compared to that of baseline; second, the tumor volume at day 67 was over 150 mm³.* | | | | | | | |

**Table 9. Rechallenge test to evaluate the induction of immune memory after treatment with Cbo-23 combined with GNTbm-38-K30.**

| CT26 rechallenge | | | | | | | |
|---|---|---|---|---|---|---|---|
| Status | Groups | Day 45 | | Day 56 | | | |
| | | mm³ | Fold change | mm³ | Fold change | Score | Immunity Percentage (%) |
| | GNTbm-38-K30 (20 mg/kg) + Regorafenib (30 mg/kg) | | | | | | |
| CR | 117 | 47.64 | 1 | 0 | 0 | - | 100% (5/5) |
| CR | 119 | 68.65 | 1 | 0 | 0 | - | |
| CR | 103 | 70.93 | 1 | 0 | 0 | - | |
| CR | 104 | 70.88 | 1 | 0 | 0 | - | |
| CR | 105 | 74.19 | 1 | 0 | 0 | - | |

| | Cbo-23 (30 mg/kg) | | | | | | |
|---|---|---|---|---|---|---|---|
| CR | 132 | 119.17 | 1 | 309.20 | 2.6 | + | 50% (1/2) |
| CR | 135 | 60.82 | 1 | 0 | 0 | - | |

| | GNTbm-38-K30 (20 mg/kg) + Cbo-23 (30 mg/kg) | | | | | | |
|---|---|---|---|---|---|---|---|
| CR | 146 | 91.29 | 1 | 0 | 0 | - | 100% (8/8) |
| CR | 147 | 53.28 | 1 | 0 | 0 | - | |
| CR | 148 | 82.24 | 1 | 0 | 0 | - | |
| CR | 149 | 109.61 | 1 | 0 | 0 | - | |
| CR | 142 | 73.84 | 1 | 106.27 | 1.4 | - | |
| CR | 143 | 126.93 | 1 | 0 | 0 | - | |
| CR | 144 | 108.54 | 1 | 0 | 0 | - | |
| CR | 145 | 134.49 | 1 | 0 | 0 | - | |

### LLC1 rechallenge

| Status | Groups | Day 45 | | Day 56 | | | |
|---|---|---|---|---|---|---|---|
| | | mm³ | Fold change | mm³ | Fold change | Score | Immunity Percentage (%) |
| | GNTbm-38-K30 (20 mg/kg) + Regorafenib (30 mg/kg) | | | | | | |
| CR | 117 | 98.55 | 1 | 778.37 | 7.9 | + | 0% (0/5) |
| CR | 119 | 112.55 | 1 | 167.34 | 1.5 | + | |
| CR | 103 | 179.77 | 1 | 909.83 | 5.1 | + | |
| CR | 104 | 168.35 | 1 | 392.61 | 2.3 | + | |
| CR | 105 | 196.30 | 1 | 408.60 | 2.1 | + | |

| | Cbo-23 (30 mg/kg) | | | | | | |
|---|---|---|---|---|---|---|---|
| CR | 132 | 163.76 | 1 | 403.54 | 2.5 | + | 50% (1/2) |
| CR | 135 | 173.53 | 1 | 169.80 | 1.0 | +/- | |

| | GNTbm-38-K30 (20 mg/kg) + Cbo-23 (30 mg/kg) | | | | | | |
|---|---|---|---|---|---|---|---|
| CR | 146 | 152.51 | 1 | 371.38 | 2.4 | + | 0% (0/8) |
| CR | 147 | 156.74 | 1 | 482.16 | 3.1 | + | |
| CR | 148 | 161.72 | 1 | 682.69 | 4.2 | + | |
| CR | 149 | 144.85 | 1 | 342.12 | 2.4 | + | |
| CR | 142 | 184.19 | 1 | 667.92 | 3.6 | + | |
| CR | 143 | 145.65 | 1 | 397.40 | 2.7 | + | |
| CR | 144 | 151.45 | 1 | 768.40 | 5.1 | + | |
| CR | 145 | 127.77 | 1 | 351.73 | 2.8 | + | |

| | | | | | | | |
|---|---|---|---|---|---|---|---|
| *If both of the following criteria are met, the response will be considered as relapse: first, the tumor size was over 1.5folds when compared to that of baseline; second, the tumor volume at day 56 was over 150 mm³.* | | | | | | | |

**Synthesis of various kinase inhibitors with novel structures and potent immune regulation activities.**

Immunocompetent mouse animal testing platforms were used for the screening of compounds to evaluate their immunomodulatory activity. These structurally similar compounds were tested for the anti-cancer activity in in vivo tests through monotherapy or combination therapy, in which the anti-cancer activity was demonstrated to be achieved by selectively attacking tumors and generating immune memory through the mechanism of cancer immunotherapy. In the present disclosure, inventors focused on the mechanism of these orally available and strongly immunomodulatory multi-kinase inhibitors, specifically in combination with specific HDAC inhibitors, which can significantly inhibit tumor growth. The inventors proved that this anti-cancer mechanism is correlated to cancer immunotherapy mechanism. All novel compounds were confirmed for their chemical structures by NMR and mass spectrometry, followed by a series of cell growth and enzyme activity inhibition assays, and animal tests. In this disclosure, three well-known multi-kinase inhibitors (Cabozantinib, Zanzalintinib, and Regorafenib) are specifically used as positive controls. At the same time, two unique HDAC inhibitors (Chidamide/Tucidinostat and GNTbm-38) were also used to combine the new compounds to confirm the unique anti-cancer immunotherapy activity. Synthesis pathways, conditions, and spectroscopic data for all new compounds can be found in the Materials and Methods section.

**The newly synthesized compounds have been shown to inhibit growth of a variety of cancer cell lines.**

Four human cancer cell lines (MDA-MB-453, MDA-MB-231, SW48, and A549) and one human normal cell line (M10) and murine colorectal cancer cell lines (CT-26) were tested for growth inhibition after treatment with these compounds as shown in **Table 2.** Many novel compounds showed superior growth inhibition activity to these cancer cell lines when compared to the two positive controls (Cabozantinib and Zanzalintinib), such as Cbo-02, Cbo-05, Cbo-07, Cbo-08, Cbo-10, Cbo-14, Cbo-20 and Cbo-23. Furthermore, Cbo-02, Cbo-10 and Cbo-23 were tested on the NCI-60 human cancer cell lines for the analysis of growth inhibition, as shown in **Table 3, Table 4 and Table 5,** The GI₅₀ of these compounds were analyzed. The compounds with potent in vitro anti-cancer activity were further screened for inhibitory activity against tyrosine kinases and analyzed by in vivo animal test for anti-cancer activity. Cabozantinib was used as a positive control for these analyses. Cbo-02, Cbo-10, Cbo-14, and Cbo-23 were tested for tyrosine kinases enzyme inhibitory activity to determine whether there is a difference in the profile of tyrosine kinases inhibition, as shown in **Table 6.**

***In vitro* tyrosine kinase inhibition profile of Cbo-02, Cbo-10, Cbo-14, and Cbo-23.**

To determine the selective inhibition activity of a given compound against various tyrosine kinases, the SelectScreen Kinase Profiling Services from ThermoFisher was used. Multiple potential compounds such as Cbo-02, Cbo-10, Cbo-14, and Cbo-23 further entered the tyrosine kinases enzyme platform for inhibition activity assays. Data were determined using the SelectScreen Biochemical Kinase Profiling Service and performed at Thermo Fisher Life Technologies. The inhibition activities of compounds were determined in biochemical assays using single concentration (1.0 µM) measurements. The results were provided as percent inhibition values. A compound displaying a percent inhibition value of greater 80% was defined as a hit. 99 different tyrosine kinases were used, and the lists of kinases for these respective panels are provided in **Table 6.** Among the compounds, the most noteworthy is Cbo-23, which has been shown to have a similar inhibitory tyrosine kinase profile to Cabozantinib, suggesting that the inhibition may primarily correlate with the inhibition of tumor development, angiogenesis inhibition, inhibition of tumor invasion and tumor metastasis. The difference in the profile may suggest that Cbo-23 has immune regulation activity within the tumor microenvironment, as shown in **Table 6.** It is interesting that Cbo-23 significantly inhibits more than 20 kinase targets, such as: AXL, BTK, CSF1R, FGR, FLT3, FLT4, KDR(VEGFR2), c-MER, c-MET, RON, PDGFR-A, RET, ROS-1, SRC, TEK(Tie-2), TYRO3, FER, FRK, HCK, INSRR, LCK, TYN-A/-B, MST1R, NTRK-2, EPHAs, EPHBs and YES 1. As shown in **Table 7**, the inhibition of enzyme activity of Cbo-23 in 16 tyrosine kinases was significantly stronger than that of Cabozantinib and Zanzalintinib such as MERTK (c-MER), ROS1, BTK, NTRK2 (TRKB), LYNB, FLT4 (VEGFR3), EPHA2, HCK, FER, FGR, SRC, PDGFRA, MST1R (RON), LYNA, INSRR (IRR), and TEK (Tie-2). There are several important tyrosine kinase targets, and Cbo-23, although not significantly superior to Cabozantinib and Zanzalintinib, also has very strong inhibitory activity on kinases such as AXL, TYRO3 (RSE), LCK, FLT3, FRK (PTK5), RET, KDR (VEGFR2), and MET (c-MET). From the above results, it is apparent that Cbo-23 is a very promising multi-kinase inhibitor with immune regulation activity.

**Therapeutic response of Cbo-02 combined with Chidamide-K30 in CT26 tumor-bearing mice.**

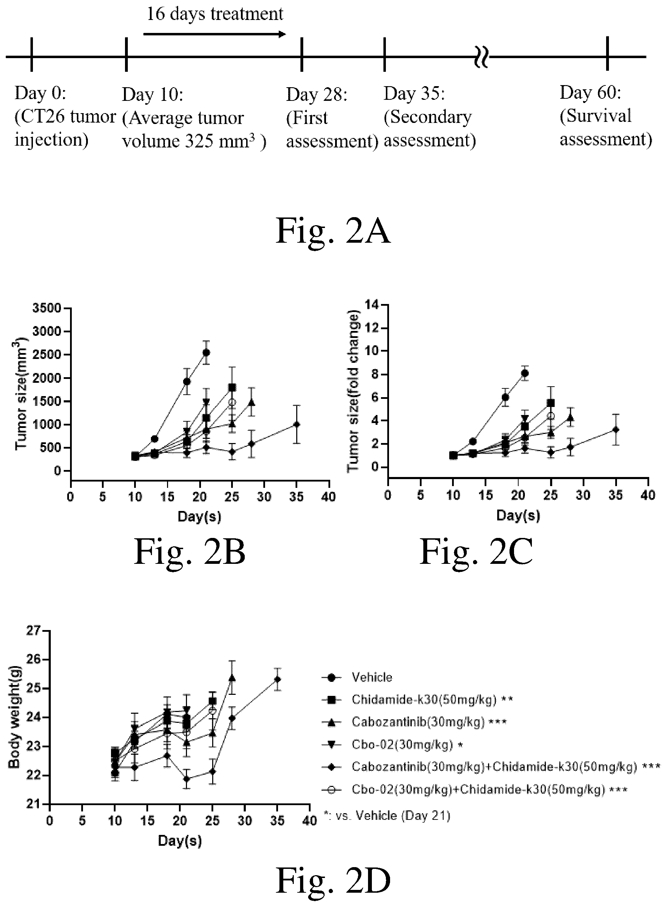

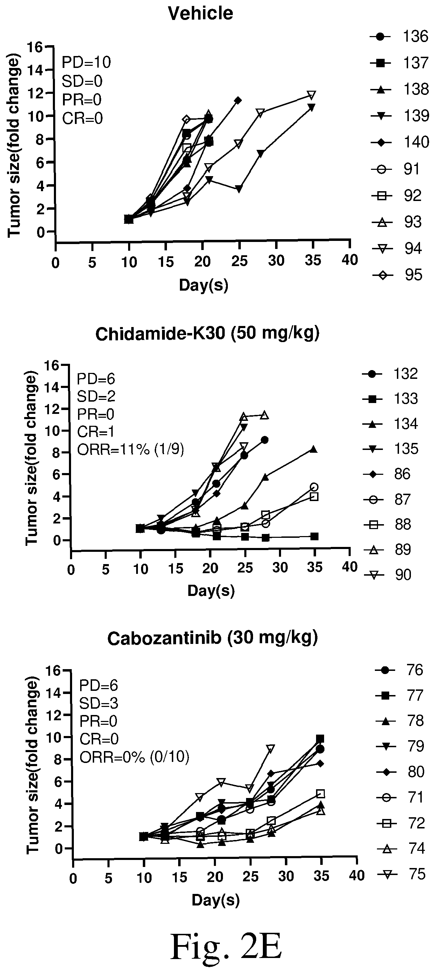

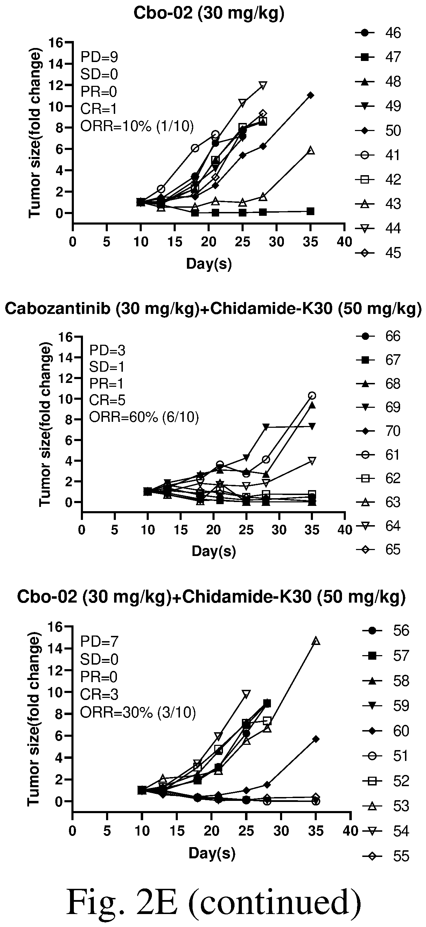

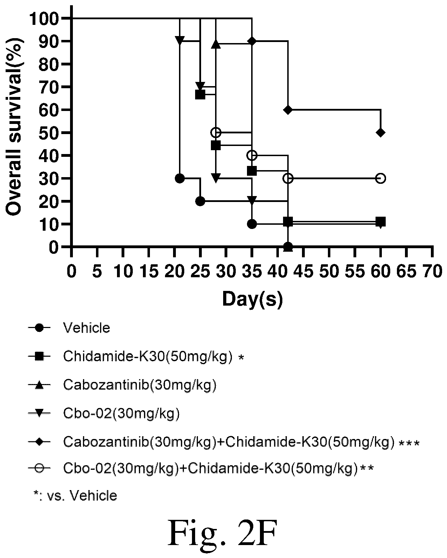

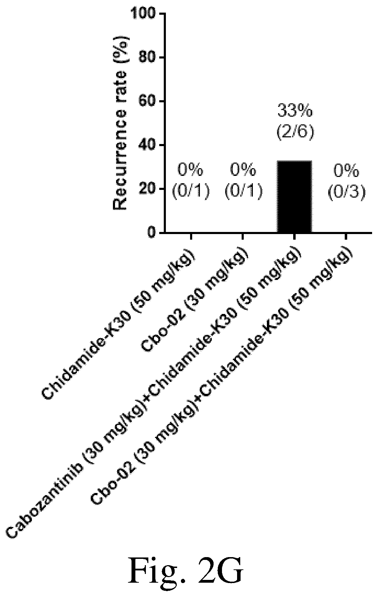

After confirmation of multiple-kinase-inhibiting activity, the compounds were evaluated for the in vivo anti-cancer activity by using immunocompetent cancer-burdened mice, in which CT-26 cold tumor cancer cell line was chosen as a stringent test for the evaluation of tumor growth inhibition. As shown in **Figure 2A**, the timing of compound administration, along with efficacy and survival evaluation, was carefully designed. For the evaluation, both monotherapy and combination therapy were tested for the inhibition of tumor growth, as shown in **Figure 2B** and **Figure 2C**, respectively. It appears that the anti-cancer activity of the combination of Cabozantinib plus Chidamide-K30 is significantly better than that of the combination of Cbo-02 and Chidamide-K30, and superior to the treatment of any monotherapy. Besides the anti-cancer effect of these monotherapies, the inventors are particularly interested in the regulatory mechanism of multi-kinase inhibitors when combined with special HDAC inhibitors, suggesting significantly superior immune-regulating activity within the tumor microenvironment, which can greatly inhibit tumor growth and obtain high ORR. When in combination with Chidamide-K30 (HDAC inhibitor), the results indicate that the efficacy of treatment with Cabozantinib (30 mg/kg, as positive control) + Chidamide-K30 (50 mg/kg) (ORR=60%) > Cbo-02 (30 mg/kg) + Chidamide-K30 (50 mg/kg) (ORR=30%) > Chidamide-K30 (50 mg/kg) (ORR=11%) > Cbo-02 (30 mg/kg) (ORR=10%) > Cabozantinib (30 mg/kg) (ORR=0%), as shown in **Figure 2E****.** The results clearly demonstrated that Cbo-02 combined with Chidamide-K30 has an additive anti-cancer effect; however, Cabozantinib combined with Chidamide-K30 seems to have a synergistic anti-cancer effect. As shown in **Figure 2F**, the survival rate data also demonstrated that the anti-cancer activity of Cabozantinib combined with Chidamide-K30 was significantly better than that of Cbo-02 combined with Chidamide-K30. Although the combination of Cabozantinib plus Chidamide-K30 has excellent anti-cancer effects, after stopping treatment, tumor recurrence was found to be higher, as shown in **Figure 2G****.** Body weight showed little change after treatment with the compound or drug was stopped, and no significant difference occurred, as shown in **Figure 2D****.**

**Therapeutic response of Cbo-20 combined with GNTbm-38-K30 in CT26 tumor-bearing mice.**

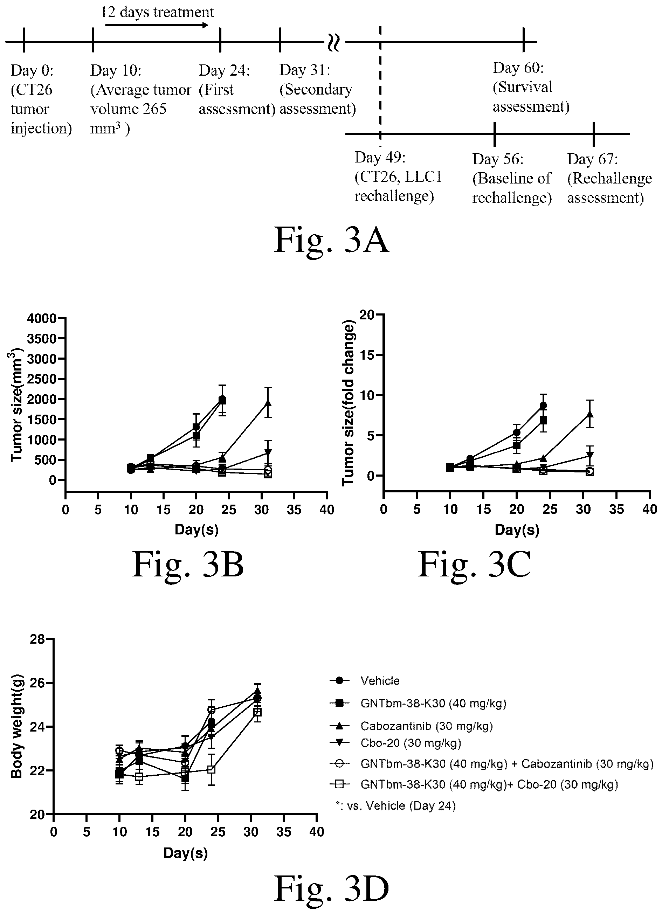

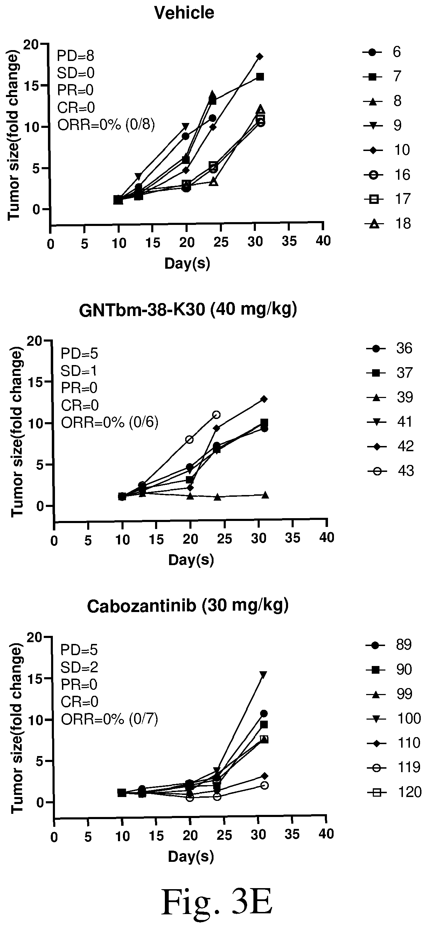

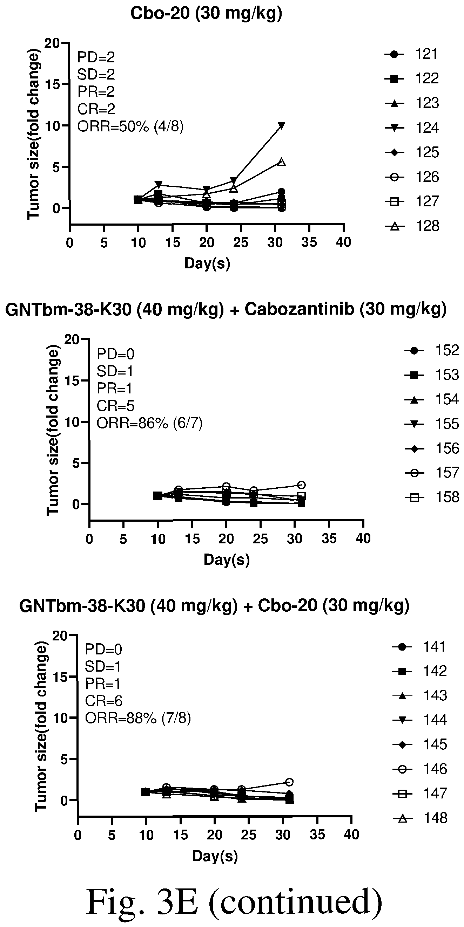

The design of the in vivo test is presented in **Figure 3A****.** All compounds or drugs were administered orally for 12 days, followed by an evaluation of tumor inhibition growth 10 days after stopping treatment, during which tumor size measurements were performed twice a week as shown in **Figures 3B** and **3C****.** Monotherapy with Cbo-20 is superior to Cabozantinib's monotherapy in anti-cancer activity. However, in terms of combination therapy, Cbo-20 combined with GNTbm-38 is as good as Cabozantinib combined with GNTbm-38, and both showed very superior anti-cancer activity, as shown in **Figures 3B** and **3C****.** The individual tumor sizes fold change and ORR, as shown in **Figure 3E****,** indicated that GNTbm-38-K30 (40 mg/kg) group achieved 0 CR, 0 PR, 1 SD, and 5 PD, with the ORR 0%; Cabozantinib (30 mg/kg) group achieved 0 CR, 0 PR, 2 SD, and 5 PD, with the ORR 0%; Cbo-20 (30 mg/kg) group achieved 2 CR, 2 PR, 2 SD, and 2 PD, with the ORR 50%; Cabozantinib (30 mg/kg) + GNTbm-38-K30 (40 mg/kg) group achieved 5 CR, 1 PR, 1 SD, and 0 PD, with the ORR 86%; Cbo-20 (30 mg/kg) + GNTbm-38-K30 (40 mg/kg) group achieved 6 CR, 1 PR, 1 SD, and 0 PD, with the ORR 88%. These results demonstrated that Cbo-20 combined with GNTbm-38 group is as good as Cabozantinib combined with GNTbm-38 group, and both showed a very superior ORR. The results indicate that treatment with Cbo-20 (30 mg/kg) + GNTbm-38-K30 (40 mg/kg) (ORR=88%) > Cabozantinib (30 mg/kg) + GNTbm-38-K30 (40 mg/kg) (ORR=86%) > Cbo-20 (30 mg/kg) (ORR=50%) > Cabozantinib (30 mg/kg) (ORR=0%) = GNTbm-38-K30 (40 mg/kg) group (ORR=0%).

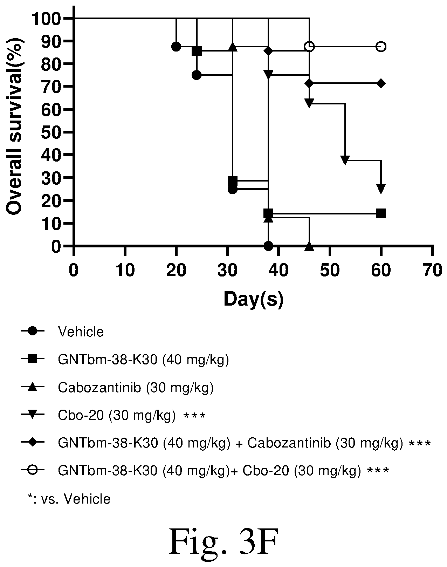

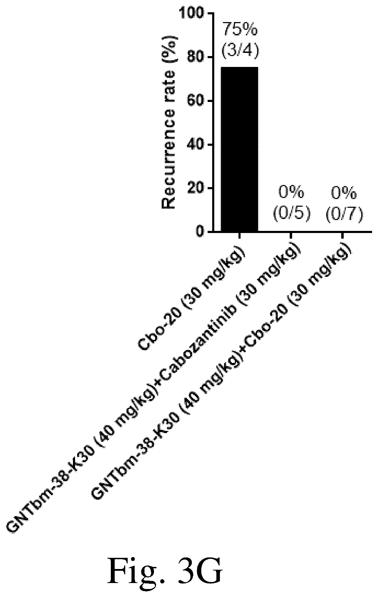

Analysis of survival rate showed that the combination of Cbo-20 combined with GNTbm-38 was slightly superior to the combination of Cabozantinib combined with GNTbm-38, as shown in **Figure 3F**, indicating Cbo-20 (30 mg/kg) + GNTbm-38-K30 (40 mg/kg) (Survival Rate= 88%) > Cabozantinib (30 mg/kg) + GNTbm-38-K30 (40 mg/kg) (Survival Rate= 71%) > Cbo-20 (30 mg/kg) (Survival Rate= 25%) > GNTbm-38-K30 (40 mg/kg) group (Survival Rate= 16%) > Cabozantinib (30 mg/kg) group (Survival Rate= 0%). The recurrence rate was evaluated as shown in **Figure 3G****.** Only in the Cbo-20 (30 mg/kg) group, 3 mice with tumor recurrence (recurrence rate=75% (3/4)) was observed, while the other two combination groups did not show any recurrence on day 60. This also shows that these two combinations are superior in the prevention of cancer recurrence. After all treatments alone or in combination, the body weight of mice did not change much, and no significant changes occurred, as shown in **Figure 3D****.** To further assess whether immune memory is induced, a rechallenge test was used for the assessment. As shown in **Table 8**, the combination of Cbo-20 and GNTbm-38 significantly inhibited tumor growth and significantly induced immune memory. In the rechallenge test, two tumors were implanted with two different cancer cell lines separately on the back of mice that had CR and PR responses after treatment. These two cancer cell lines were CT-26, which the immune system had been induced to recognize during treatment, and LLC-1 cell line, which the immune system had not been induced to recognize during treatment. Therefore, CT-26 tumor did not grow, and LLC-1 tumor grew significantly.

**Therapeutic response of Cbo-22 combined with GNTbm-38-K30 in CT26 tumor-bearing mice.**

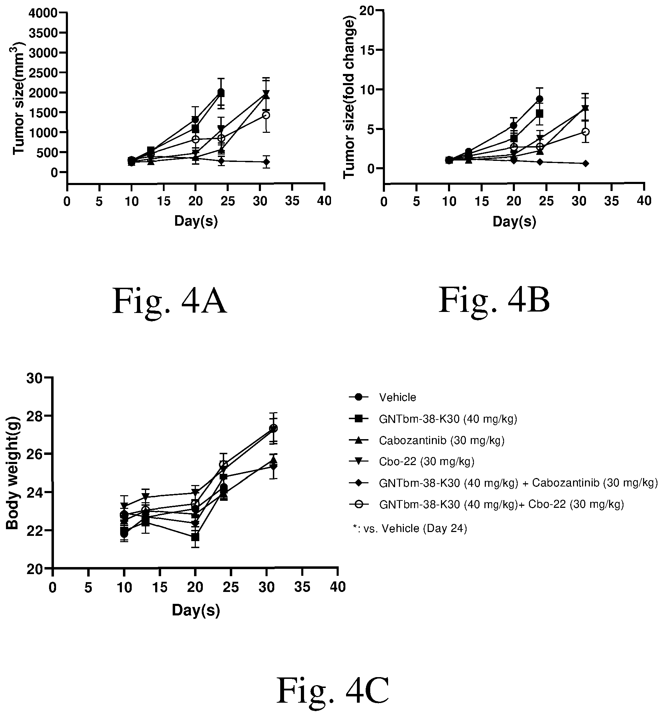

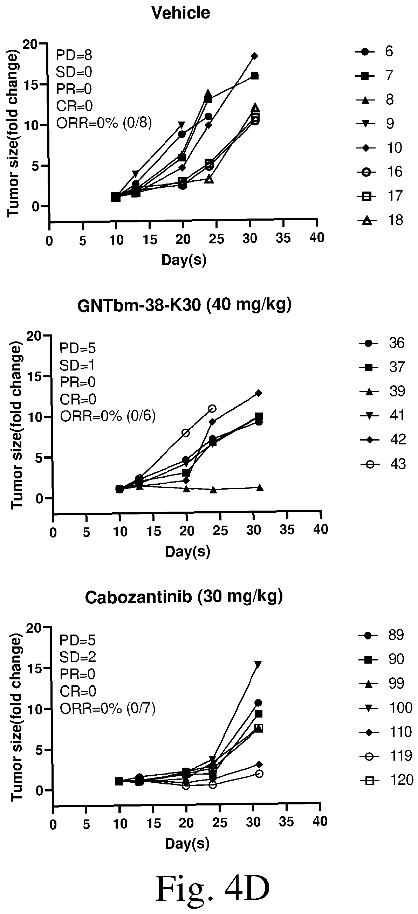

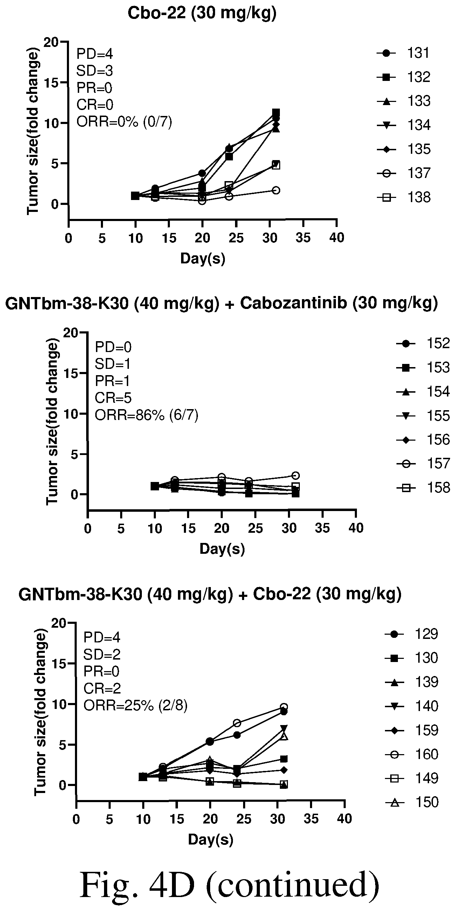

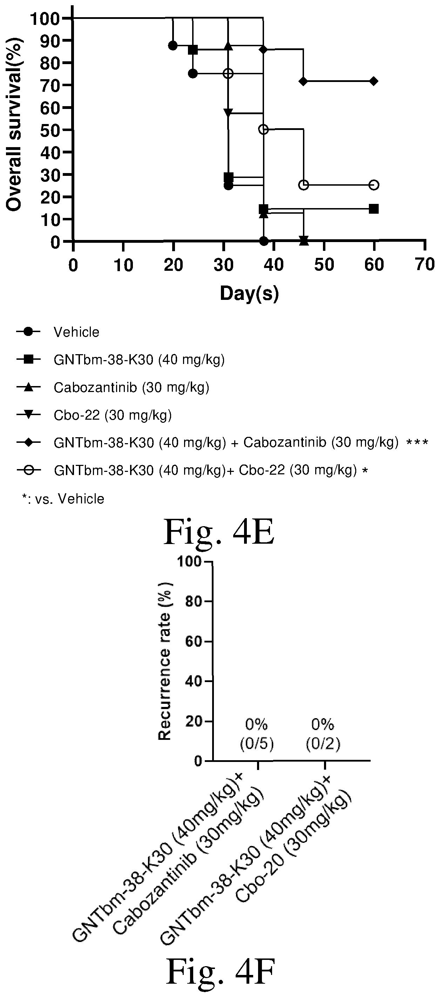

Monotherapy or combination therapy with GNTbm-38 was used to test the tumor inhibition activity of Cbo-22 in CT-26 bearing mice, as shown in **Figures 4A** and **4B****.** The anti-cancer activity of Cabozantinib combined with GNTbm-38 was significantly better than that of Cbo-22 combined with GNTbm-38. This combination was significantly superior to the other treatments, as shown in **Figures 4A** and **4B****.** The results indicate that treatment with Cabozantinib (30 mg/kg) + GNTbm-38-K30 (40 mg/kg) (ORR=86%) > Cbo-22 (30 mg/kg) + GNTbm-38-K30 (40 mg/kg) (ORR=25%) > Cbo-22 (30 mg/kg) (ORR=0%) = Cabozantinib (30 mg/kg), and that the synergistic anti-cancer effect of Cabozantinib combined with GNTbm-38 was significantly better than that of the combination of Cbo-22 and GNTbm-38 regimen, as shown in **Figure 4D****.** The overall survival rate is shown in **Figure 4E**, in which Cabozantinib (30 mg/kg) + GNTbm-38-K30 (40 mg/kg) group (Survival Rate= 71%) > Cbo-22 (30 mg/kg) + GNTbm-38-K30 (40 mg/kg) group (Survival Rate= 25%) > GNTbm-38-K30 (40 mg/kg) group (Survival Rate= 16%) > Cabozantinib (30 mg/kg) group (Survival Rate= 0%) > Cbo-22 (30 mg/kg) group (Survival Rate= 0%). The recurrence rate was evaluated as shown in **Figure 4F****.** The recurrence rate results demonstrated that there was no relapse in these two combination groups. The treatments did not cause a significant difference in body weight, as shown in **Figure 4C****.**

**Therapeutic response of Cbo-23 combined with GNTbm-38-K30 in CT26 tumor-bearing mice.**

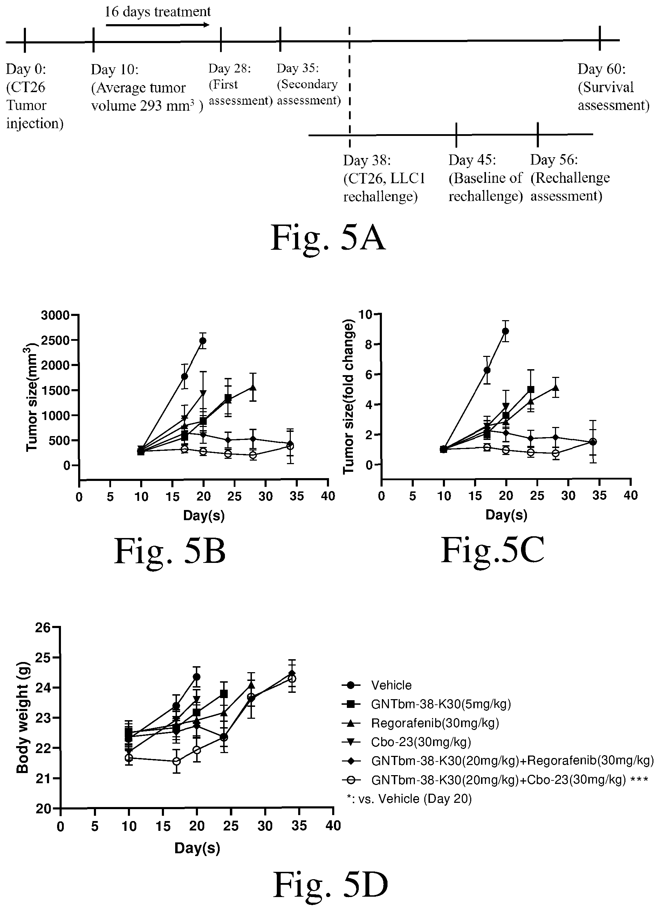

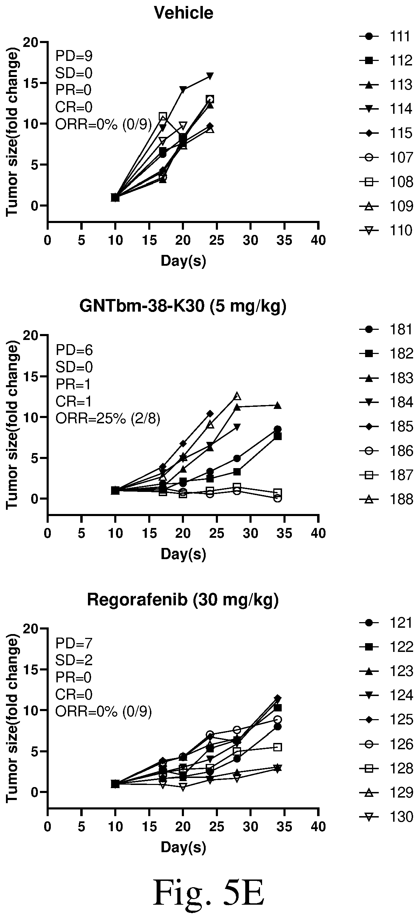

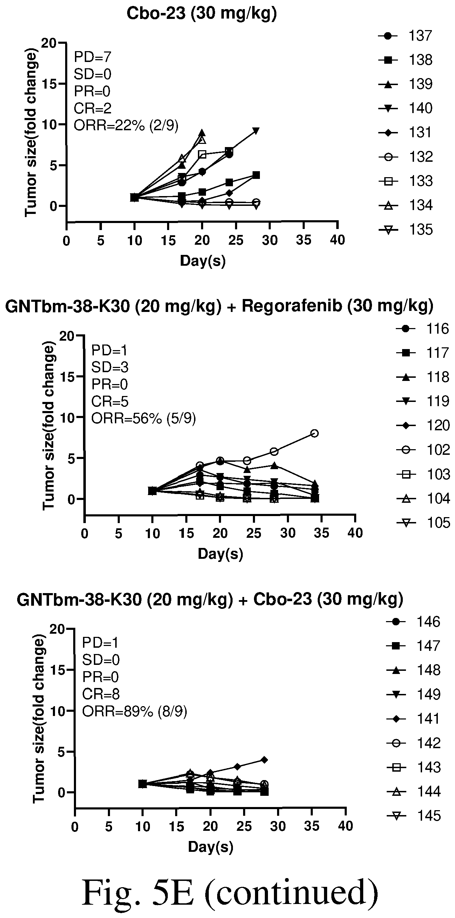

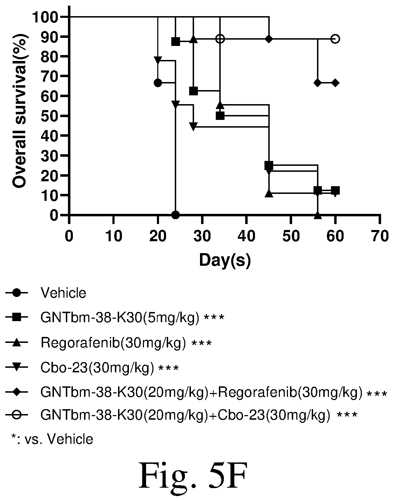

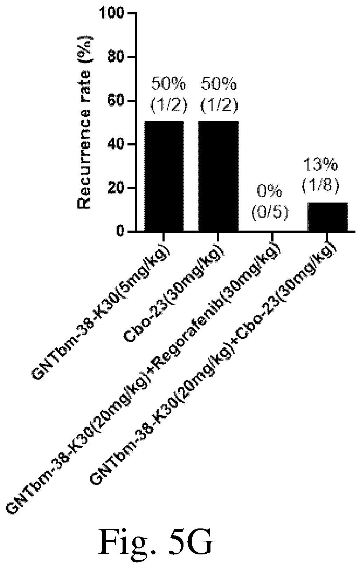

The animal test scheme is shown in **Figure 5A****.** It was recognized that, in addition to Cabozantinib, the multi-kinase inhibitor Regorafenib also showed a similar immunomodulatory activity compared to Cabozantinib. Therefore, in this animal experiment, Regorafinib was particularly used as a positive control. As shown in **Figures 5B** and **5C****,** the Cbo-23 combined with GNTbm-38 is superior to the Regorafenib combined with GNTbm-38 in inhibiting tumor growth. After all treatments, monotherapy or in combination, the body weight of mice did not change significantly, as shown in **Figure 5D****.** The results indicate that treatment with Cbo-23 (30 mg/kg) + GNTbm-38-K30 (20 mg/kg) (ORR=89%) > Regorafenib (30 mg/kg) + GNTbm-38-K30 (20 mg/kg) (ORR=56%) > GNTbm-38-K30 (5 mg/kg) (ORR=25%) > Cbo-23 (30 mg/kg) (ORR=22%) > Regorafenib (30 mg/kg) (ORR=0%), as shown in **Figure 5E****.** The individual tumor sizes fold change and ORR, as shown in **Figure 5E****,** indicated that GNTbm-38-K30 (5 mg/kg) group achieved 1 CR, 1 PR, 0 SD, and 6 PD, with the ORR 25%; Regorafenib (30 mg/kg) group achieved 0 CR, 0 PR, 2 SD, and 7 PD, with the ORR 0%; Cbo-23 (30 mg/kg) group achieved 2 CR, 0 PR, 0 SD, and 7 PD, with the ORR 22%; Regorafenib (30 mg/kg) + GNTbm-38-K30 (20 mg/kg) group achieved 5 CR, 0 PR, 3 SD, and 1 PD, with the ORR 56%; Cbo-23 (30 mg/kg) + GNTbm-38-K30 (20 mg/kg) group achieved 8 CR, 0 PR, 0 SD, and 1 PD, with the ORR 89%. From these results, it is clear that Cbo-23 combined with GNTbm-38 has excellent anti-cancer benefits, and Cbo-23 is a very promising multi-kinase inhibitor with immune regulation activity. The overall survival rate is shown in **Figure 5F**, in which Cbo-23 (30 mg/kg) + GNTbm-38-K30 (20 mg/kg) group (Survival Rate= 89%) > Regorafenib (30 mg/kg) + GNTbm-38-K30 (20 mg/kg) group (Survival Rate= 67%) > GNTbm-38-K30 (5 mg/kg) group (Survival Rate= 13%) > Cbo-23 (30 mg/kg) group (Survival Rate= 11%) > Regorafenib (30 mg/kg) group (Survival Rate= 0%). The recurrence rate was evaluated as shown in **Figure 5G****.** The recurrence rate results demonstrated that GNTbm-38-K30 (5 mg/kg) group had 1 mouse with recurrence (recurrence rate=50% (1/2), Cbo-23 (30 mg/kg) had 1 mouse with recurrence (recurrence rate=50% (1/2), Cbo-23 (30 mg/kg) + GNTbm-38-K30 (20 mg/kg) had 1 mouse with recurrence (recurrence rate=13% (1/8), and Regorafenib (30 mg/kg) + GNTbm-38-K30 had no mice with recurrence on day 60. To further confirm whether immune memory is induced, the rechallenge test was performed as shown in **Table 9.** The inventors found it intriguing that Cbo-23 combined with GNTbm-3 8 showed an induction of immune memory, as evidenced by LLC 1 tumor growth alongside the inhibition of CT-26 tumor growth. This indeed proved that Cbo-23 in combination with GNTbm-38 is a potent regimen that can activate the immune system, selectively attacking tumors, and generate immune memory as shown in **Table 9.**

**Therapeutic response of Cbo-23 as compared with Cabozantinib, monotherapy or in combination, in CT26 tumor-bearing mice.**

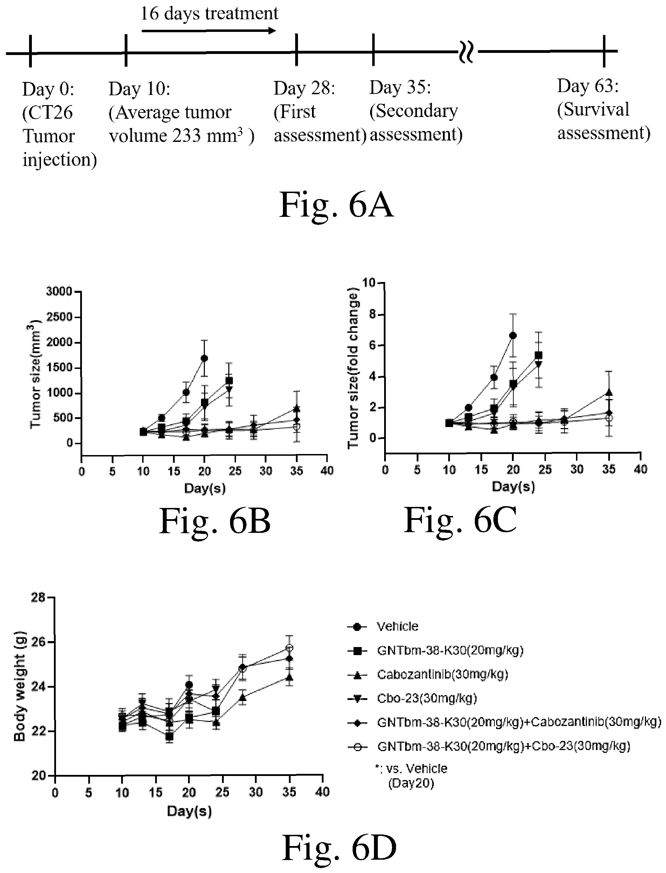

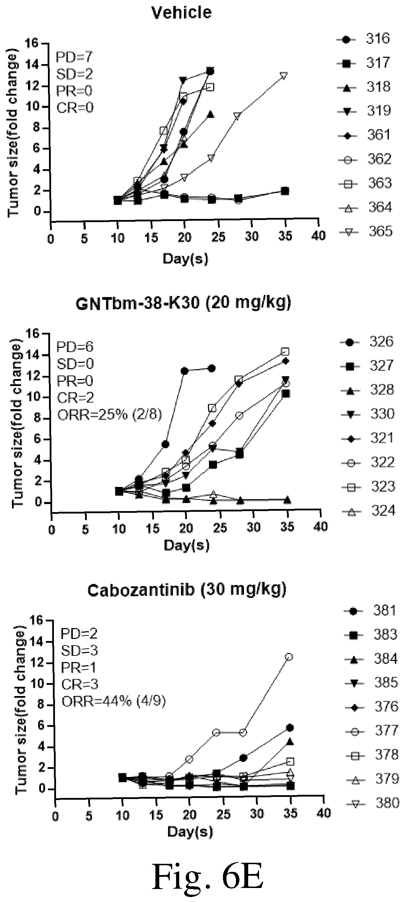

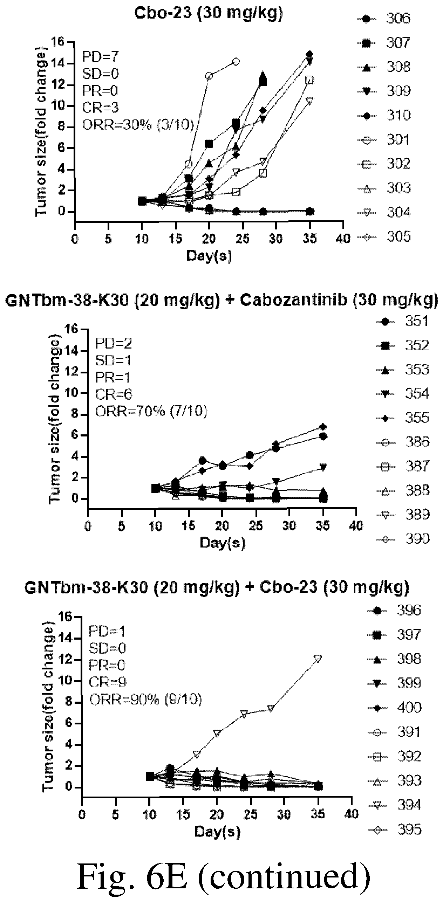

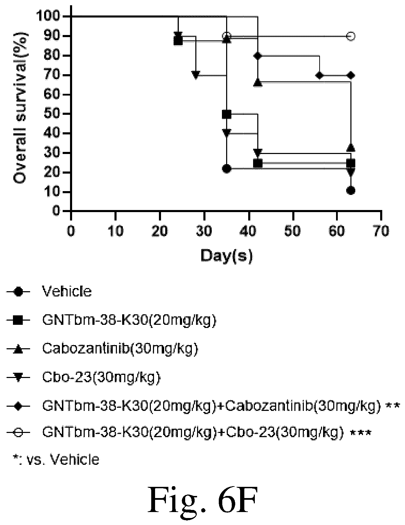

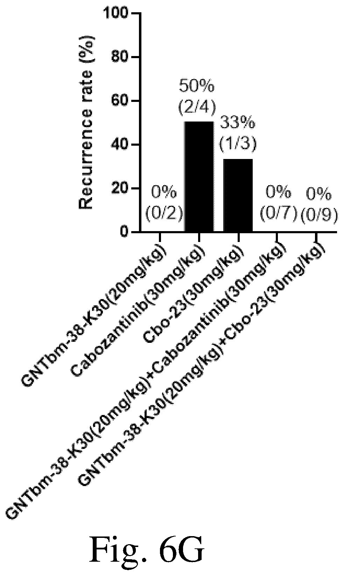

The multi-kinase inhibitor Cabozantinib is known for showing immunomodulatory activity. Therefore, an animal experiment was designed to compare the immunomodulatory activity between Cbo-23 and Cabozantinib. The animal test scheme is shown in **Figure 6A****.** As shown in **Figures 6B** and **6C****,** the activity of Cbo-23 combined with GNTbm-38 is superior to the activity of Cabozantinib combined with GNTbm-38 in inhibiting tumor growth. After all treatments, monotherapy or in combination, the body weight of mice did not change significantly, as shown in **Figure 6D****.** The results indicate that treatment with Cbo-23 (30 mg/kg) + GNTbm-38-K30 (20 mg/kg) (ORR=90%) > Cabozantinib (30 mg/kg) + GNTbm-38-K30 (20 mg/kg) (ORR=70%) > Cabozantinib (30 mg/kg) (ORR=44%) > Cbo-23 (30 mg/kg) (ORR=30%) > GNTbm-38-K30 (20 mg/kg) (ORR=25%), as shown in **Figure 6E****.** The individual tumor sizes fold change and ORR, as shown in **Figure 6E****,** indicated that GNTbm-38-K30 (20 mg/kg) group achieved 2 CR, 0 PR, 0 SD, and 6 PD, with the ORR 25%; Cabozantinib (30 mg/kg) group achieved 3 CR, 1 PR, 3 SD, and 2PD, with the ORR 44%; Cbo-23 (30 mg/kg) group achieved 3 CR, 0 PR, 0 SD, and 7 PD, with the ORR 30%; Cabozantinib (30 mg/kg) + GNTbm-38-K30 (20 mg/kg) group achieved 6 CR, 1 PR, 1 SD, and 2 PD, with the ORR 70%; Cbo-23 (30 mg/kg) + GNTbm-38-K30 (20 mg/kg) group achieved 9 CR, 0 PR, 0 SD, and 1 PD, with the ORR 90%. From these results, it is clear that Cbo-23 combined with GNTbm-38 has excellent anti-cancer benefits, and Cbo-23 is a very promising multi-kinase inhibitor with immune regulation activity. The overall survival rate is shown in **Figure 6F**, in which Cbo-23 (30 mg/kg) + GNTbm-38-K30 (20 mg/kg) group (Survival Rate= 90%) > Cabozantinib (30 mg/kg) + GNTbm-38-K30 (20 mg/kg) group (Survival Rate= 70%) > Cabozantinib (30 mg/kg) (Survival Rate= 33%) >GNTbm-38-K30 (20 mg/kg) group (Survival Rate= 25%) > Cbo-23 (30 mg/kg) group (Survival Rate= 20%). The recurrence rate was evaluated as shown in **Figure 6G****.**

**Therapeutic response of Cbo-23-K30 combined with GNTbm-38-2HCL in CT26 tumor-bearing mice.**

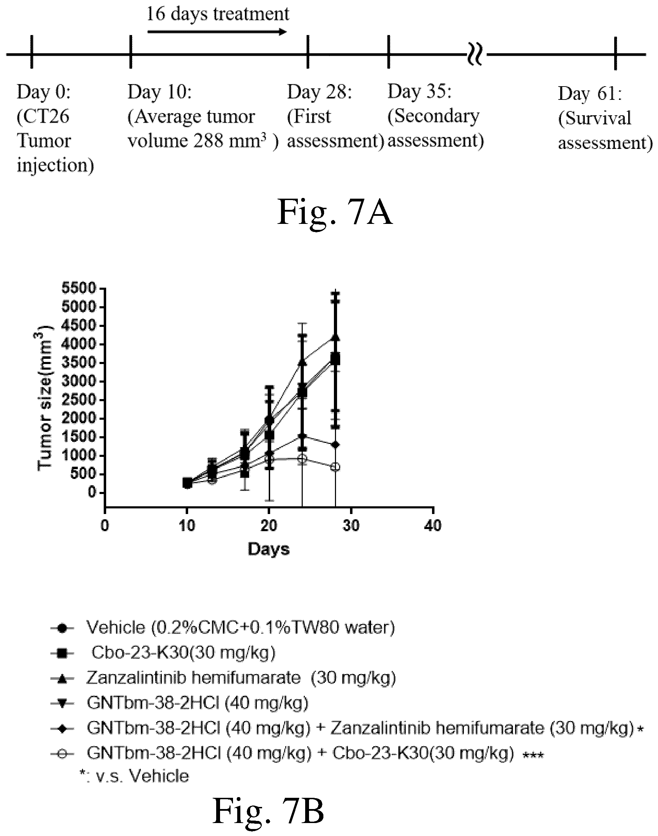

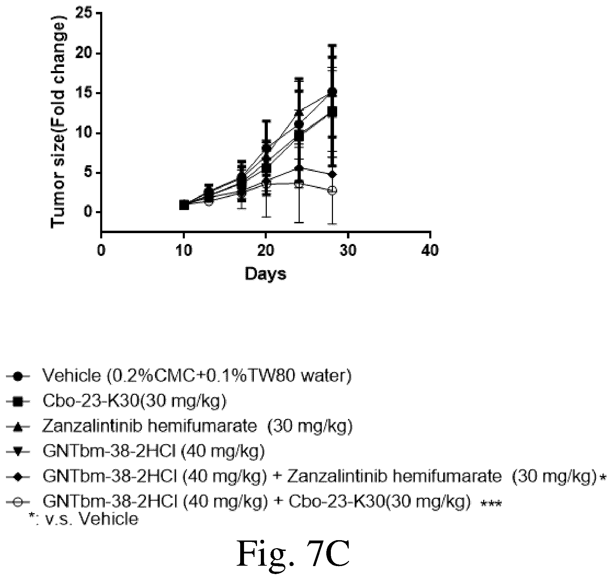

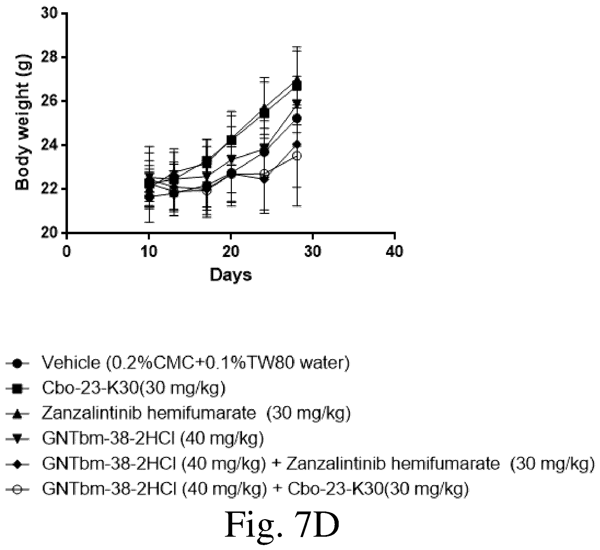

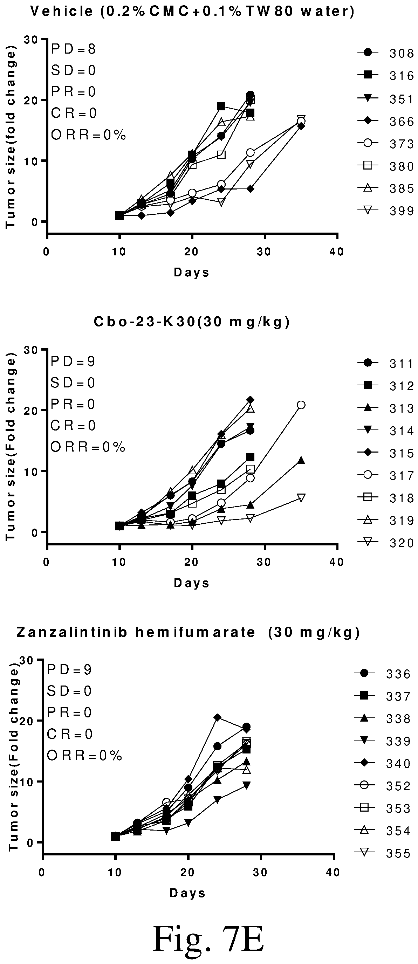

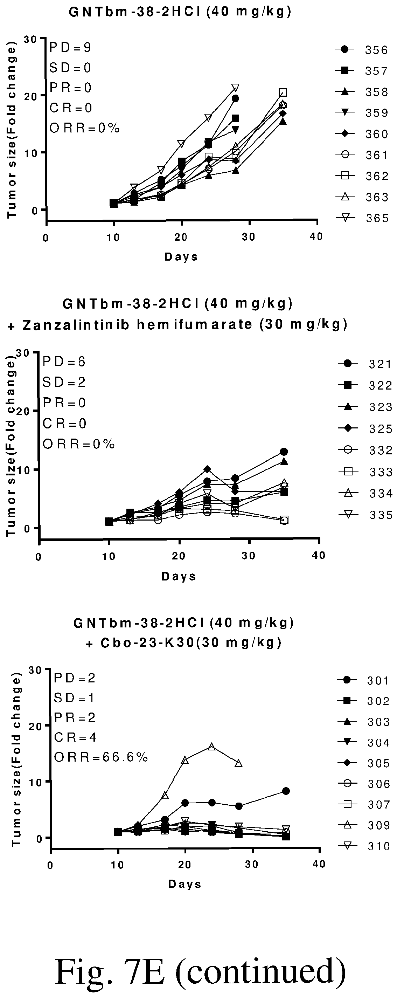

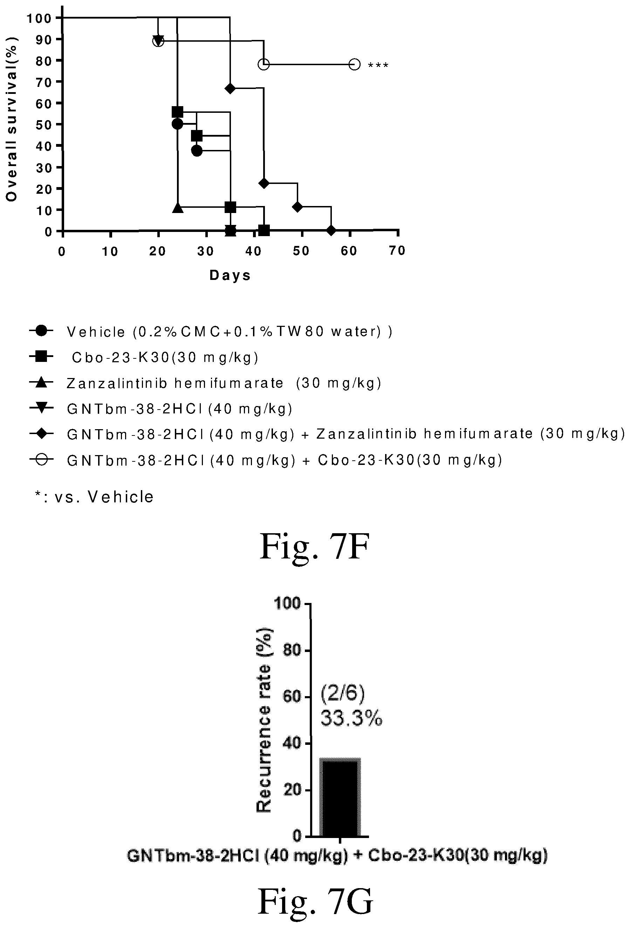

Zanzalintinib, a new generation multi-kinase inhibitor, is also known for showing immunomodulatory activity. In order to increase the solubility and bioavailability of Cbo-23, based on the salt form of Zanzalintinib (Zanzalintinib hemifumarate), Cbo-23 was formulated into a fumarate salt form and also an amorphous solid dispersion (ASD) in PVP K-30. As shown in **Figure 7A****,** the initial tumor size was 263.9 mm³ before treatment. Therefore, in this animal experiment, Zanzalintinib hemifumarate was specifically used as a positive control. As shown in **Figures 7B** and **7C****,** the tumor inhibition rate at day 28 in the single-agent zanzalitinib hemifumarate (30 mg/kg) and Cbo-23-K30 (30 mg/kg) group was 2% and 22%, respectively. The activity of Cbo-23-K30 (30 mg/kg) combined with GNTbm-38-2HCL (40 mg/kg) is superior to the activity of Zanzalintinib hemifumarate (30 mg/kg) combined with GNTbm-38-2HCL (40 mg/kg) in inhibiting tumor growth, and the tumor inhibition rate at day 28 was 87% and 76%, respectively. After all treatments, monotherapy or in combination, the body weight of mice did not change significantly, as shown in **Figure 7D****.** The results indicate that treatment with Cbo-23- K30 (30 mg/kg) + GNTbm-38-2HCL (40 mg/kg) (ORR=66.6%) > Zanzalintinib hemifumarate (30 mg/kg) + GNTbm-38-2HCL (40 mg/kg) (ORR=0%) as shown in **Figure 7E****.** The individual tumor sizes fold change and ORR, as shown in **Figure 7E****,** indicated that GNTbm-38--2HCL (40 mg/kg) group achieved 0 CR, 0 PR, 0 SD, and 9 PD, with the ORR 0%; Zanzalintinib hemifumarate (30 mg/kg) group achieved 0 CR, 0 PR, 0 SD, and 9 PD, with the ORR 0 %; Cbo-23- K30 (30mg/kg) group achieved 0 CR, 0 PR, 0 SD, and 9 PD, with the ORR 0%; Zanzalintinib hemifumarate (30 mg/kg) + GNTbm-38-2HCL (40 mg/kg) group achieved 0 CR, 0 PR, 2 SD, and 6 PD, with the ORR 0%; Cbo-23-K30 (30 mg/kg) + GNTbm-38-2HCL (40 mg/kg) group achieved 4 CR, 2 PR, 1 SD, and 2 PD, with the ORR 66.6 %. From these results, it is obvious that the combination of Cbo-23-K30 and GNTbm-38-2HCL has excellent anti-cancer effects through synergistic mechanisms. Cbo-23 is a very promising multi-kinase inhibitor with immunomodulatory activity. The overall survival rate is shown in **Figure 7F**, in which Cbo-23-K30 (30 mg/kg) + GNTbm-38-2HCL (40 mg/kg) group (Survival Rate= 77.7%) > Zanzalintinib hemifumarate (30 mg/kg) + GNTbm-38-2HCL (40 mg/kg) group (Survival Rate= 0%). In the analysis of OS on day 61, it can be observed that there are still 3 CRs in the Cbo-23-K30 combination group, which shows that compared with the zanzalitinib hemifumarate combination group, it has significant tumor immune activation ability. The recurrence rate was evaluated as shown in **Figure 7G****.**

A person of ordinary skill in the art of the subject disclosure should understand that variations and modifications may be made to the teaching and the disclosure of the subject disclosure without departing from the spirit and scope of the subject application. Based on the contents above, the subject application intends to cover any variations and modification thereof with the proviso that the variations or modifications fall within the scope as defined in the appended claims or their equivalents.

## Claims

1. A compound of formula (I): 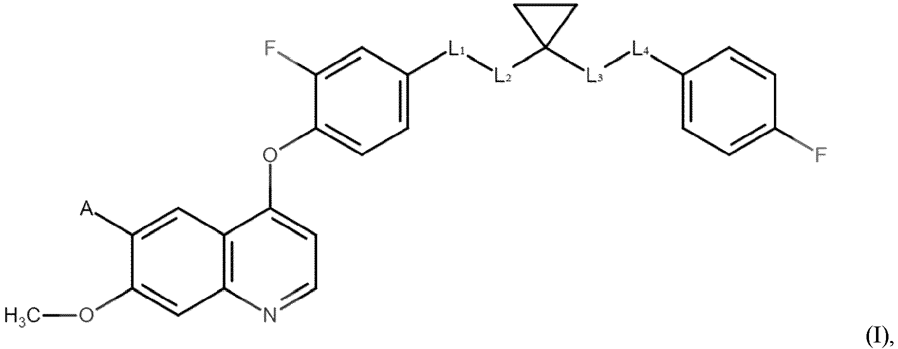 wherein:
A is -OD or 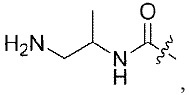 wherein the wavy line presents the linkage position;
D is H, C₁₋₃alkyl or Ar;
Ar is 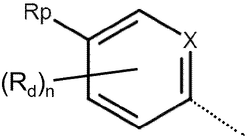 wherein the dashed line presents the linkage position;
X is N, CH or CR_{d}; n is an integer ranging from 0 to 3;
each R_{d}, when present, is independently selected from the group consisting of methyl, NH₂, OH, SH, methoxy, dimethylamino, triflouromethoxy, cyanide, nitro, nitroso, sulfonate, sulfonamide, C₁-C₆ alkyl, C₁-C₆ alkyloxy, carboxamide, F, Cl and Br;
Rₚ is selected from the group consisting of H, methyl, NH₂, OH, SH, methoxy, dimethylamino, triflouromethoxy, cyanide, nitro, nitroso, sulfonate, sulfonamide, C₁-C₆ alkyl, C₁-C₆ alkyloxy, carboxamide, F, Cl and Br;
L₁, L₂ is (i) -NH-C(=O)- or (ii) -(C=O)-NH-; and
L₃, L₄ is (i) -NH-C(=O)- or (ii) -(C=O)-NH-;
or a pharmaceutically acceptable salt, hydrate, stereoisomer, solvate or prodrug thereof.

2. The compound of claim 1, which has the formula (II): 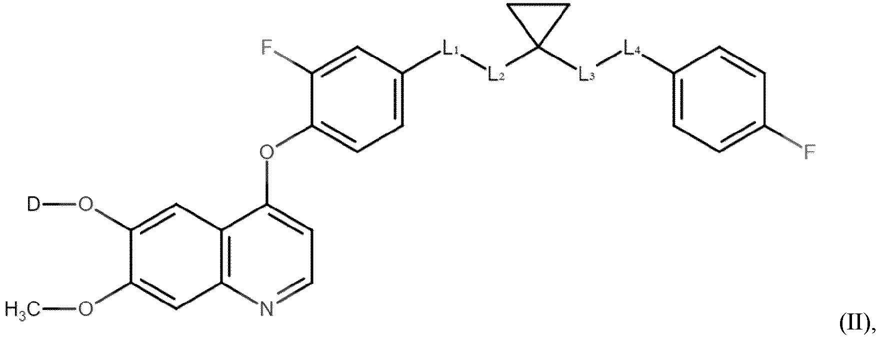 wherein D, L₁, L₂, L₃ and L₄, have the same meaning as described in formula (I); or a pharmaceutically acceptable salt, hydrate, stereoisomer, solvate or prodrug thereof.

3. The compound of claim 1, which has the formula (III): 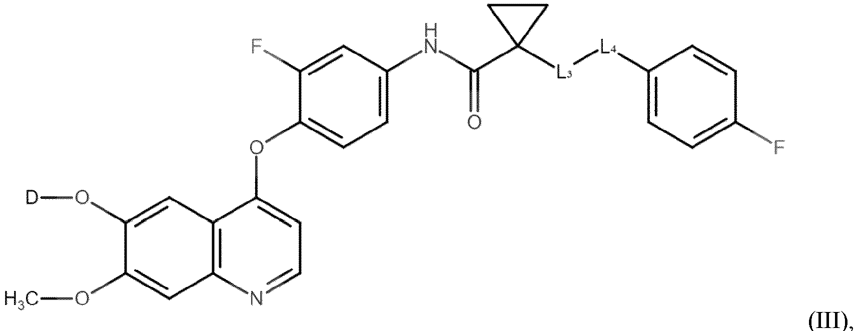 wherein D, L₃ and L₄, have the same meaning as described in formula (I); or a pharmaceutically acceptable salt, hydrate, stereoisomer, solvate or prodrug thereof.

4. The compound of claim 1, which has the formula (IV): 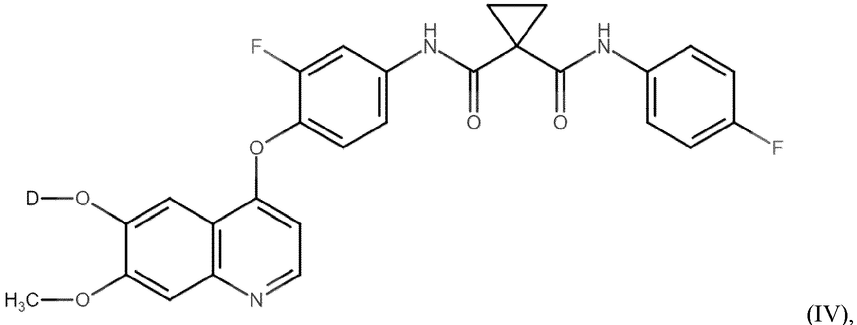 wherein D has the same meaning as described in formula (I); or a pharmaceutically acceptable salt, hydrate, stereoisomer, solvate or prodrug thereof.

5. The compound of any one of claims 1 to 4, wherein Ar is 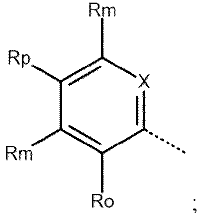
X is N, CH or CRₒ;
Rₚ is selected from the group consisting of H, NH₂ and nitro;
each Rₘ is independently selected from the group consisting of H, methyl, NH₂, nitro, nitroso, sulfonate, sulfonamide, C₁-C₆ alkyl, C₁-C₆ alkyloxy, carboxamide, F, Br and Cl;
each Rₒ is independently selected from the group consisting of H, methyl, NH₂, nitro, nitroso, sulfonate, sulfonamide, C₁-C₆ alkyl, C₁-C₆ alkyloxy, carboxamide, F, Br and Cl; or a pharmaceutically acceptable salt, hydrate, stereoisomer, solvate or prodrug thereof,
preferably wherein Ar is 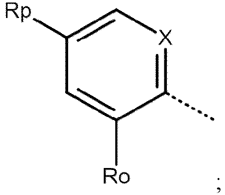
X is N, CH or CRₒ;
Rₚ is selected from the group consisting of H, NH₂ and nitro;
each Rₒ is independently selected from the group consisting of H, methyl, NH₂, nitro, nitroso, sulfonate, sulfonamide, C₁-C₆ alkyl, C₁-C₆ alkyloxy, carboxamide, F, Br and Cl; or a pharmaceutically acceptable salt, hydrate, stereoisomer, solvate or prodrug thereof.

6. The compound of any one of claims 1 to 4, wherein Ar is selected from the group consisting of 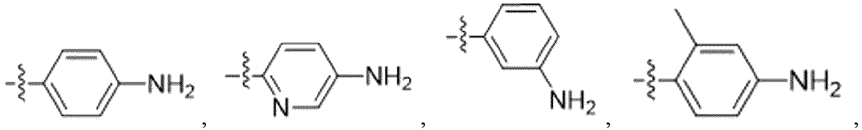 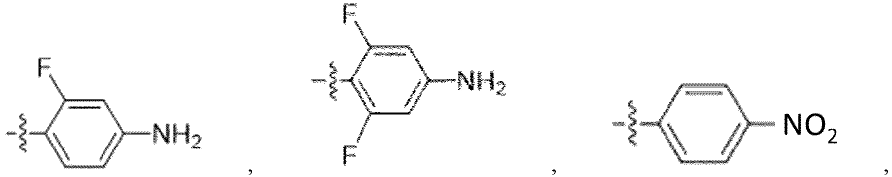 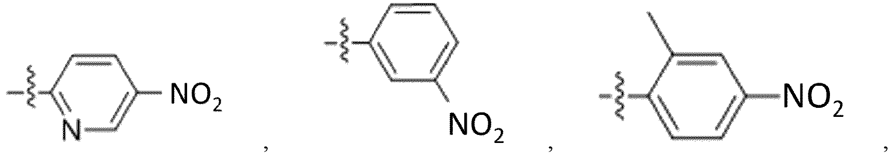 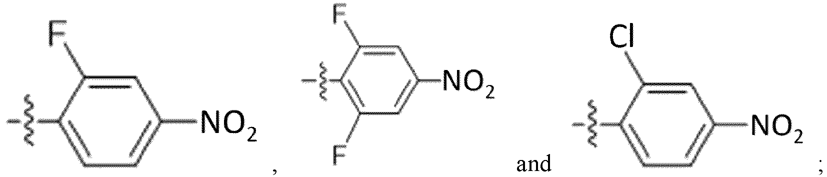 or a pharmaceutically acceptable salt, hydrate, stereoisomer, solvate or prodrug thereof.

7. The compound of claim 1, which is selected from the group consisting of: 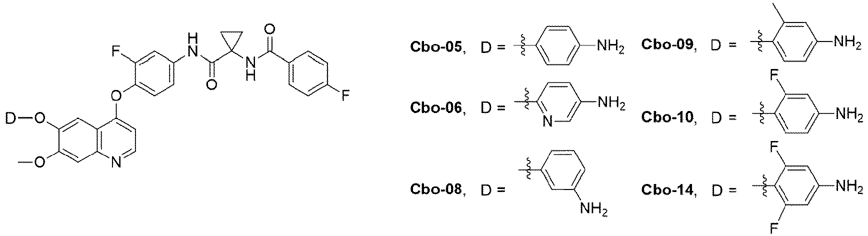 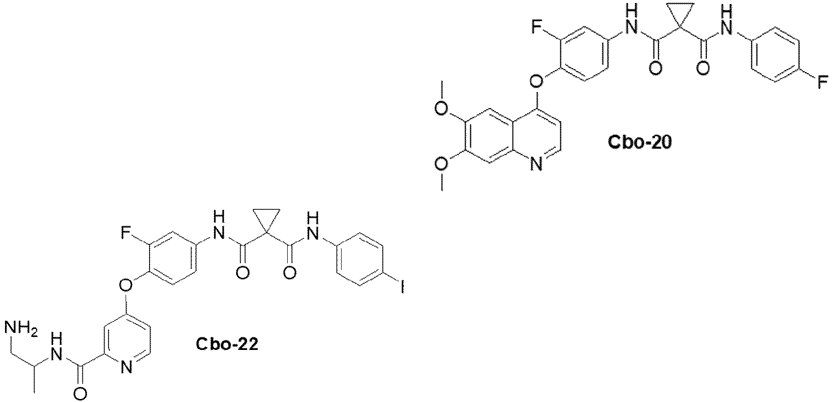 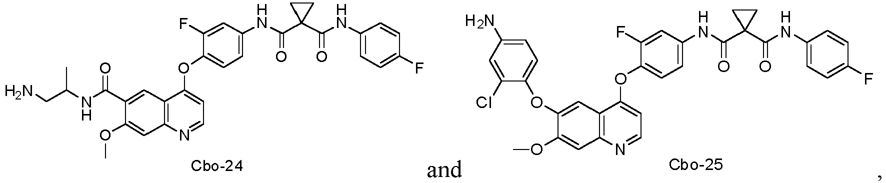 or a pharmaceutically acceptable salt, hydrate, stereoisomer, solvate or prodrug thereof.

8. A pharmaceutical composition or pharmaceutical combination comprising the compound of any of claims 1 to 7 or a pharmaceutically acceptable salt, hydrate, stereoisomer, solvate or prodrug thereof, and a pharmaceutical acceptable carrier.

9. The pharmaceutical composition or pharmaceutical combination of claim 8, which further comprises one or more second agents,
preferably wherein the second agent is an immune checkpoint inhibitor, an anti-cancer agent or a combination thereof.

10. A compound according to any one of claims 1 to 7 or a pharmaceutically acceptable salt, hydrate, stereoisomer, solvate or prodrug thereof or a pharmaceutical composition or pharmaceutical combination according to claim 8 or 9 for use in a method for suppressing angiogenesis, metastasis or oncogenesis in a subject in need thereof, the method comprising administering an effective amount of a compound according to any one of claims 1 to 7 or a pharmaceutically acceptable salt, hydrate, stereoisomer, solvate or prodrug thereof or a pharmaceutical composition or pharmaceutical combination according to claim 8 or 9.

11. A compound according to any one of claims 1 to 7 or a pharmaceutically acceptable salt, hydrate, stereoisomer, solvate or prodrug thereof or a pharmaceutical composition or pharmaceutical combination according to claim 8 or 9 for use in a method for modulating immune regulation activities in a subject in need thereof, the method comprising administering an effective amount of a compound according to any one of claims 1 to 8 or a pharmaceutically acceptable salt, hydrate, stereoisomer, solvate or prodrug thereof or a pharmaceutical composition or pharmaceutical combination according to any one of claims 9 to 11 to the subject.

12. A compound according to any one of claims 1 to 7 or a pharmaceutically acceptable salt, hydrate, stereoisomer, solvate or prodrug thereof or a pharmaceutical composition or pharmaceutical combination according to claim 8 or 9 for use in a method for treating or preventing the disease associated with tyrosine kinases (TKs) in a subject in need thereof, the method comprising administering an effective amount of a compound according to any one of claims 1 to 8 or a pharmaceutically acceptable salt, hydrate, stereoisomer, solvate or prodrug thereof or a pharmaceutical composition or pharmaceutical combination according to any one of claims 9 to 11 to the subject.

13. A compound according to any one of claims 1 to 7 or a pharmaceutically acceptable salt, hydrate, stereoisomer, solvate or prodrug thereof or a pharmaceutical composition or pharmaceutical combination according to claim 8 or 9 for use in a method for treating tumors or cancers in a subject in need thereof, the method comprising administering an effective amount of a compound according to any one of claims 1 to 7 or a pharmaceutically acceptable salt, hydrate, stereoisomer, solvate or prodrug thereof or a pharmaceutical composition or pharmaceutical combination according to claim 8 or 9 to the subject.

14. The compound or a pharmaceutically acceptable salt, hydrate, stereoisomer, solvate or prodrug thereof or a pharmaceutical composition or pharmaceutical combination for use according to claim 15, wherein the method involves cancer immunotherapy by regulating the tumor microenvironment, reducing tumor hypoxia, reducing lactic acid accumulation, activating CTL, inhibiting the number and activity of immunosuppressive cells, obtaining superior anti-cancer benefits and/or inducing lasting immune memory.

15. A compound according to any one of claims 1 to 7 or a pharmaceutically acceptable salt, hydrate, stereoisomer, solvate or prodrug thereof or a pharmaceutical composition or pharmaceutical combination according to claim 8 or 9 for use in a method for treating tumors or cancers with enhanced Objective Response Rate (ORR) as compared to treatment of tumors or cancers based on a pharmaceutical composition or pharmaceutical combination comprising conventional tyrosine kinases (TKs) inhibitors, the method comprising administering an effective amount of a compound according to any one of claims 1 to 7 or a pharmaceutically acceptable salt, hydrate, stereoisomer, solvate or prodrug thereof or a pharmaceutical composition or pharmaceutical combination according to claim 8 or 9 to the subject.
